# EUROPEAN PATENT APPLICATION

(11) **EP 4 692 169 A1**
(43) Date of publication of application: **11.02.2026**
(21) Application number: 24800259.4
(22) Date of filing: 03.05.2024
(51) Int. Cl.: C08G 75/30, C08F 290/06, H01M 10/42, H01M 10/052, H01M 10/0567, C08G 75/00, C07C 381/00, H01M 10/056

(54) **COMPOUND, COMPOSITE, AND SOLID ELECTROLYTE AND ALL-SOLID-STATE BATTERY COMPRISING SAME**

(30) Priority: 03.05.2023 KR 20230057976; 03.05.2023 KR 20230057984; 20.07.2023 KR 20230094880
(71) Applicant: LG Chem, Ltd., Seoul 07336 (KR)
(72) Inventor: KIM, Ki Cheol, Daejeon 34122 (KR); KIM, Su Jeong, Daejeon 34122 (KR); YOON, Jeong Ae, Daejeon 34122 (KR); OH, Mi Yeon, Daejeon 34122 (KR); KIM, Jae Yoon, Daejeon 34122 (KR)
(74) Representative: Hoffmann Eitle
(86) International application number: PCT/KR2024/005994
(87) International publication number: WO 2024/228578

(57) **Abstract**

The present invention aims to improve upon conventional low lithium ion conductivity and resolve the performance and safety issues of an all-solid-state battery, and relates to a compound comprising a repeating unit represented by chemical formula 1 described in the present specification or a compound which is a lithium salt thereof, a composite comprising the compound and a lithium compound, and a solid electrolyte and an all-solid-state battery comprising same.

## Description

### TECHNICAL FIELD

### Cross-reference to Related Applications

This application claims the benefit of priority from Korean Patent Application Nos. 10-2023-0057976, filed on May 3, 2023, 10-2023-0057984, filed on May 3, 2023, and 10-2023-0094880, filed on July 20, 2023, in the Korean Intellectual Property Office, the disclosures of which are incorporated herein in its entirety by reference.

### Technical Field

The present invention relates to a compound, a composite, a solid electrolyte including the same, and an all-solid-state battery for improving the ion conductivity of the all-solid-state battery, and for suppressing the growth of dendritic crystals of lithium generated on the surface of a lithium metal negative electrode.

### BACKGROUND ART

A lithium secondary battery with a high energy density, which is currently mainly used in laptops and smart phones, is composed of a positive electrode made of a lithium oxide, a carbon-based negative electrode, a separator, and a liquid or solid electrolyte. However, a lithium secondary battery composed of a flammable liquid electrolyte has stability problems such as leakage, ignition, and explosion, and has a disadvantage in that a battery design becomes complicated in order to prevent the problems.

Therefore, in order to solve the problems of such a liquid electrolyte, research has been conducted on a solid electrolyte having non-flammable or flame-retardant properties, and the solid electrolyte may be classified into a sulfide-based electrolyte, an oxide-based electrolyte, and a polymer electrolyte.

The sulfide-based electrolyte has a high ion conductivity and has advantages of having low interface resistance and being stable in a wide voltage range, but there is a case in which a trace amount of impurities such as moisture mixed inside a battery reacts with the sulfide-based electrolyte, thereby generating hydrogen sulfide inside the battery, and there is a risk of hydrogen sulfide also being generated during charging and discharging of the battery, so that the sulfide-based electrolyte has a problem of being poor in terms of stability.

In addition, the oxide-based electrolyte has high chemical stability, but has a lower ion conductivity than the sulfide-based electrolyte, and requires a high-temperature sintering process, and has a disadvantage of making it difficult to manufacture a large-area battery.

Therefore, the polymer electrolyte has properties of low cost, flexibility, process convenience, and the like, thereby having an advantage as a solid electrolyte material, so that research on the polymer electrolyte has been actively conducted.

The polymer electrolyte is largely classified into a gel type and a solid type. The gel-type polymer electrolyte is an electrolyte which exhibits conductivity by impregnating a liquid electrolyte having a high boiling point in a polymer film and fixing the same together with a lithium salt, and contains a large amount of liquid electrolyte, and thus, has ion conductivity similar to that of a pure liquid electrolyte, but there is still a problem of in terms of electrochemical stability.

On the other hand, the solid polymer electrolyte does not include a liquid electrolyte, and thus, may solve a stability problem associated with leakage, and also, has an advantage of having high chemical and electrochemical stability. However, since the ion conductivity at room temperature of the solid polymer electrolyte is lower than that of the liquid electrolyte, research on addressing the issue has been significantly conducted.

Currently, a material that is most widely used for a solid polymer electrolyte is polyethylene oxide (PEO), which has the ability to conduct lithium ions despite being a solid. However, in the case of a linear PEO polymer electrolyte, there are problems in that the fluidity of a chain is limited due to high crystallinity, a large amount of lithium ions are not dissociated due to a low dielectric constant (5.0), and it is difficult to apply the linear PEO polymer electrolyte to a lithium secondary battery due to very low ion conductivity at room temperature.

(Patent Document 001) JP 1985-069140 A.

### DISCLOSURE OF THE INVENTION

### TECHNICAL PROBLEM

An object of the present invention is to provide a compound, a solid electrolyte including the same, and an all-solid-state battery for improving the ion conductivity of the solid electrolyte and for improving the performance and stability of the all-solid-state battery.

In addition, another object of the present invention is to provide a compound, a solid electrolyte including the same, and an all-solid-state battery for suppressing the growth of lithium dendritic crystals, i.e., dendrites, generated on the surface of a lithium metal negative electrode.

### TECHNICAL SOLUTION

The present invention provides a compound, a composite, a solid electrolyte including the same, and an all-solid-state battery.
(1) The present invention provides a compound including a repeating unit represented by Formula 1 below, or a compound being a lithium salt thereof.

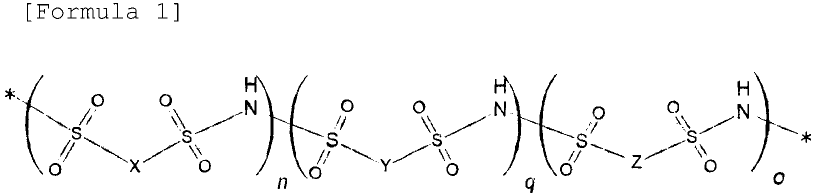

In Formula 1 above, the X, Y, and Z are each independently one or more selected from the group consisting of Formulas a-1, b, and c-1 below, the n is an integer of 1 to 100, the q is an integer of 1 to 100, the o is an integer of 0 to 100, and the * is a connection site between the repeating units, or an end site, and 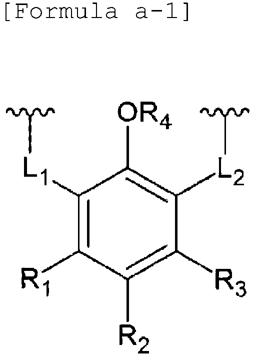 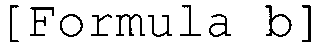 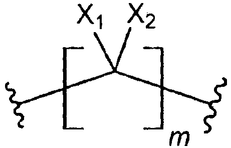 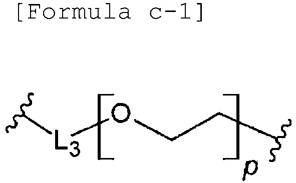 in Formulas a-1, b, and c-1 above, the R₁, R₂, and R₃ are each independently a hydrogen element, a halogen element, -CN, -NO₂, a substituted or unsubstituted C₁-C₁₀ alkyl group, a substituted or unsubstituted C₁-C₁₀ alkoxy group, a substituted or unsubstituted C₂-C₁₀ alkenyl group, a substituted or unsubstituted C₂-C₁₀ alkynyl group, -C(=O)R₅, -P(=O) (OR₅)₂, - P(OR₅)(OR₆), -OP(=O)(OR₅)(OR₆), -S(=O)R₅, or -S(=O)₂R₅, the R₄ is a hydrogen element, or a substituted or unsubstituted C₁-C₁₀ alkyl group, the R₅ and R₆ are each independently a hydrogen element, a substituted or unsubstituted C₁-C₁₀ alkyl group, or a substituted or unsubstituted C₂-C₁₀ alkenyl group, the L₁, L₂, and L₃ are each independently a direct linkage, or a substituted or unsubstituted C₁-C₁₀ alkylene group, the X₁ and X₂ are each independently a hydrogen element, a halogen element, -CN, -NO₂, a substituted or unsubstituted C₁-C₁₀ alkyl group, or a substituted or unsubstituted C₆-C₁₂ aryl group, the m is an integer of 1 to 40, and the p is an integer of 1 to 200.
(2) In (1) above, the present invention provides a compound, wherein at least one of the X, Y, and Z includes Formula a-1 above.
(3) In (1) or (2) above, the present invention provides a compound, wherein R₂ and R₄ are each independently a C₁-C₅ alkyl group.
(4) In any one of (1) to (3) above, the present invention provides a compound, wherein L₁, L₂, and L₃ above are each independently a C₁-C₅ alkylene group.
(5) In any one of (1) to (4) above, the present invention provides a compound, wherein the X₁ and X₂ are each independently a hydrogen element, or a halogen element.
(6) In any one of (1) to (5) above, the present invention provides a compound, wherein the n is an integer of 1 to 20, the q is an integer of 1 to 20, the o is an integer of 1 to 20, the m is an integer of 1 to 10, and the p is an integer of 1 to 50.
(7) In any one of (1) to (6) above, the present invention provides a compound, wherein the compound including a repeating unit represented by Formula 2 above is represented by Formula 1-a or Formula 1-b below.

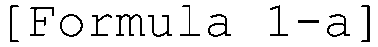

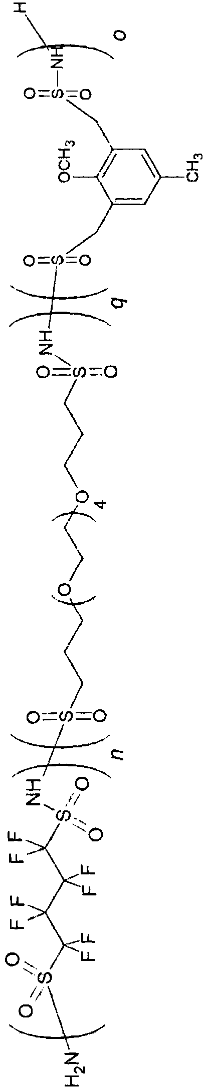

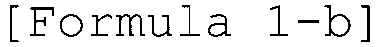

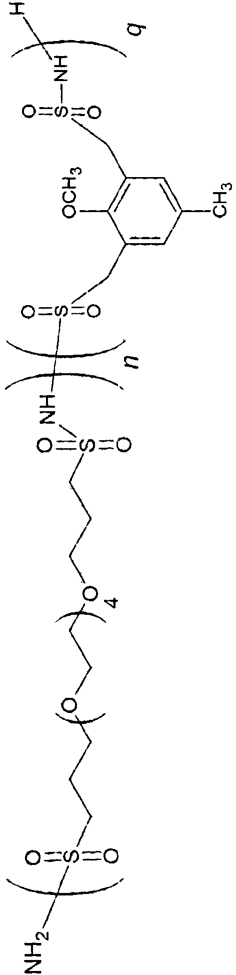

In Formulas 1-a and 1-b above, the n is an integer of 1 to 20, the q is an integer of 1 to 20, and the o is an integer of 1 to 20.

(8) In any one of (1) to (7) above, the present invention provides a compound, wherein the compound includes one or more functional groups selected from the group consisting of Formulas d to g below at at least one end site among the end sites.

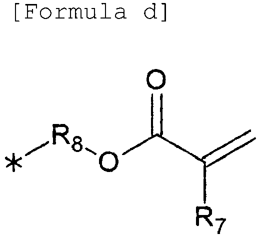

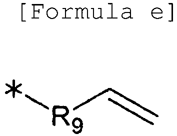

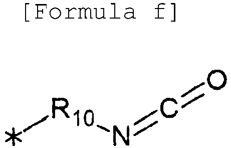

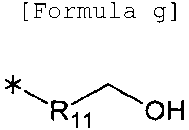

In Formulas d to g above, the R₇ is a hydrogen element, or a substituted or unsubstituted C₁-C₁₀ alkyl group, the R₈ to R₁₁ are each independently a direct linkage, a substituted or unsubstituted C₁-C₁₀ alkylene group, or -R₁₂-O-R₁₃-, the R₁₂ and R₁₃ are each independently a direct linkage, or a substituted or unsubstituted C₁-C₁₀ alkylene group, and the * is a bonding position.

(9) In (8) above, the present invention provides a compound, wherein the R₈ to R₁₁ are each independently a direct linkage, a C₁-C₆ alkylene group substituted or unsubstituted with a fluorine element, or -R₁₂-O-R₁₃-, and the R₁₂ and R₁₃ are each independently a direct linkage, or a C₁-C₆ alkylene group substituted or unsubstituted with a fluorine element.

(10) In (8) or (9) above, the present invention provides a compound, wherein the compound is represented by Formula 1-c below.

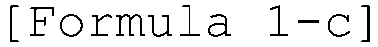

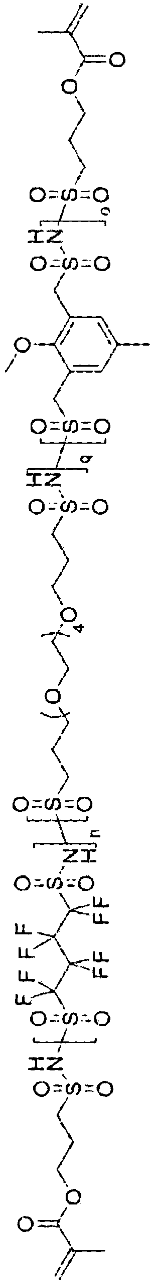

In Formula 1-c above, the n is an integer of 1 to 20, the q is an integer of 1 to 20, and the o is an integer of 1 to 20.

(11) In any one of (1) to (10) above, the present invention provides a compound, wherein the lithium salt includes a repeating unit represented by Formula 2 below.

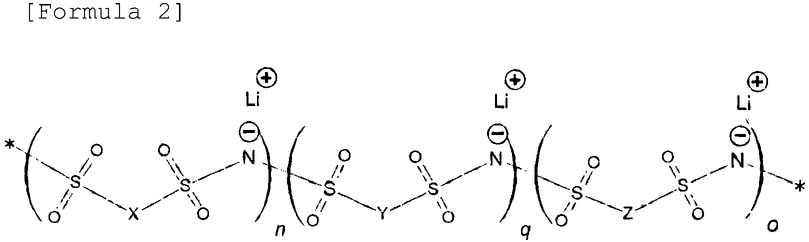

In Formula 2 above, the X, Y, and Z are each independently one or more selected from the group consisting of Formulas a-1, b, and c-1 below, the n is an integer of 1 to 100, the q is an integer of 1 to 100, the o is an integer of 0 to 100, and the * is a connection site between the repeating units, or an end site, and 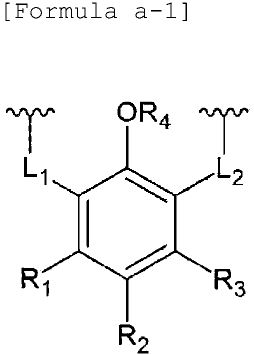 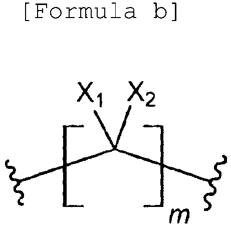 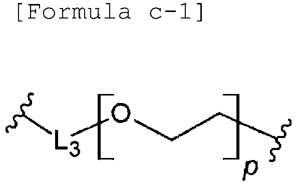 in Formulas a-1, b, and c-1 above, the R₁, R₂, and R₃ are each independently a hydrogen element, a halogen element, -CN, -NO₂, a substituted or unsubstituted C₁-C₁₀ alkyl group, a substituted or unsubstituted C₁-C₁₀ alkoxy group, a substituted or unsubstituted C₂-C₁₀ alkenyl group, a substituted or unsubstituted C₂-C₁₀ alkynyl group, -C(=O)R₅, -P(=O)(OR₅)₂, - P(OR₅)(OR₆), -OP(=O)(OR₅)(OR₆), -S(=O)R₅, or -S(=O)₂R₅, the R₄ is a hydrogen element, or a substituted or unsubstituted C₁-C₁₀ alkyl group, the R₅ and R₆ are each independently a hydrogen element, a substituted or unsubstituted C₁-C₁₀ alkyl group, or a substituted or unsubstituted C₂-C₁₀ alkenyl group, the L₁, L₂, and L₃ are each independently a direct linkage, or a substituted or unsubstituted C₁-C₁₀ alkylene group, the X₁ and X₂ are each independently a hydrogen element, a halogen element, -CN, -NO₂, a substituted or unsubstituted C₁-C₁₀ alkyl group, or a substituted or unsubstituted C₆-C₁₂ aryl group, the m is an integer of 1 to 40, and the p is an integer of 1 to 200.

(12) In (11) above, the present invention provides a compound, wherein the compound including a repeating unit represented by Formula 2 above is represented by Formula 2-a or Formula 2-b below.

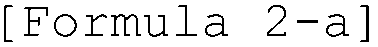

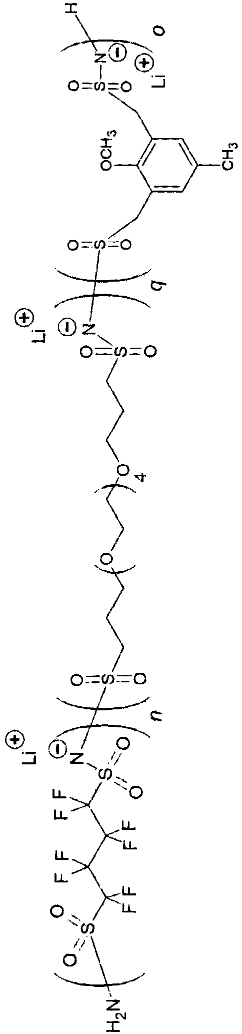

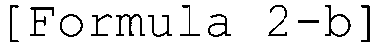

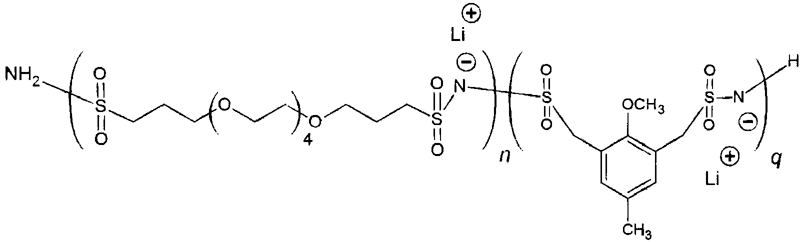

In Formulas 2-a and 2-b above, the n is an integer of 1 to 20, the q is an integer of 1 to 20, and the o is an integer of 1 to 20.

(13) In (11) or (12) above, the present invention provides a compound, wherein the compound includes one or more functional groups selected from the group consisting of Formulas d to g below at at least one end site among the end sites.

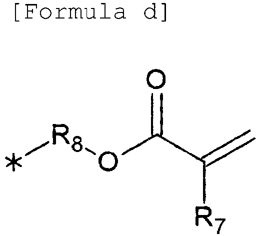

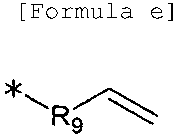

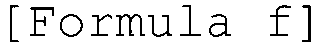

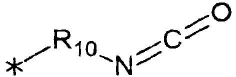

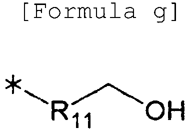

In Formulas d to g above, the R₇ is a hydrogen element, or a substituted or unsubstituted C₁-C₁₀ alkyl group,
the R₈ to R₁₁ are each independently a direct linkage, a substituted or unsubstituted C₁-C₁₀ alkylene group, or -R₁₂-O-R₁₃-, the R₁₂ and R₁₃ are each independently a direct linkage, or a substituted or unsubstituted C₁-C₁₀ alkylene group, and the * is a bonding position.

(14) In (13) above, the present invention provides a compound, wherein the compound including a repeating unit represented by Formula 2 above is represented by Formula 2-c below.

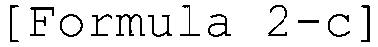

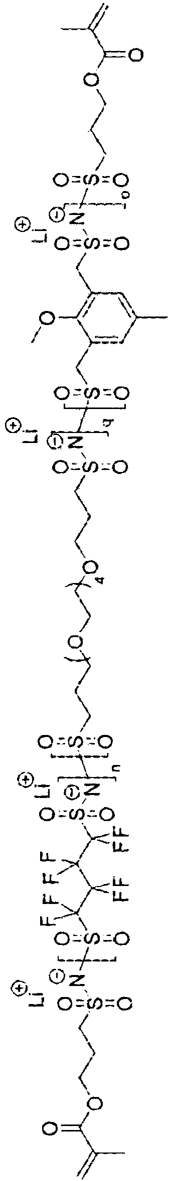

In Formula 2-c above, the n is an integer of 1 to 20, the q is an integer of 1 to 20, and the o is an integer of 1 to 20.
(15) The present invention provides a composite including the compound according to at least one of (1) to (14) above, and a lithium compound.
(16) In (15) above, the present invention provides a compound, wherein the lithium compound is one or more selected from the group consisting of LiCl, LiBr, LiI, LiBF₄, LiClO₄, LiB₁₀Cl₁₀, LiAlCl₄, LiAlO₄, LiPF₆, LiCF₃SO₃, LiCH₃CO₂, LiCF₃CO₂, LiAsF₆, LiSbF₆, LiCH₃SO₃, lithium bis(fluorosulfonyl)imide (LiFSI) (LiN(SO₂F)₂), lithium bisperfluoroethanesulfonimide (LiBETI) (LiN(SO₂CF₂CF₃)₂), and lithium (bis) trifluoromethanesulfonimide (LiTFSI) (LiN(SO₂CF₃)₂).
(17) In (15) or (16) above, the present invention provides a composite, wherein the composite is represented by one or more selected from the group consisting of Formulas 3-a, 4-a, and 4-b below.

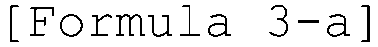

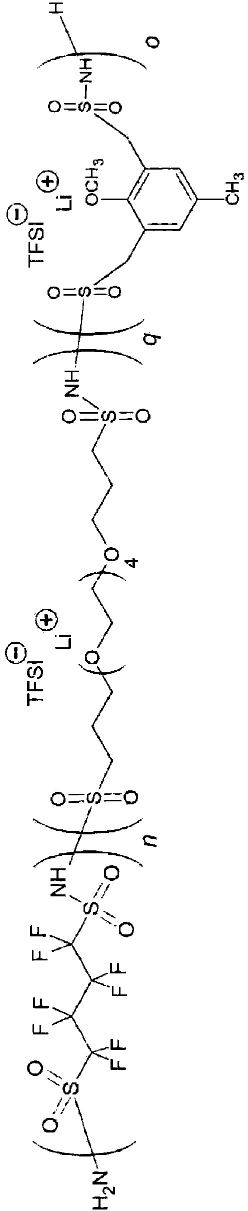

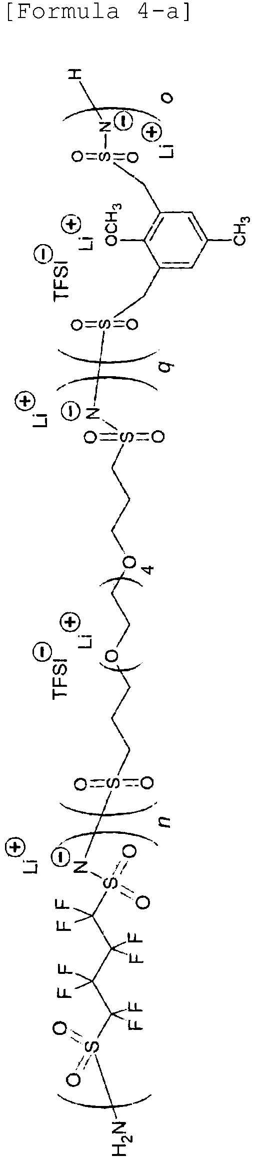

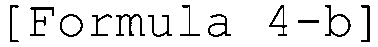

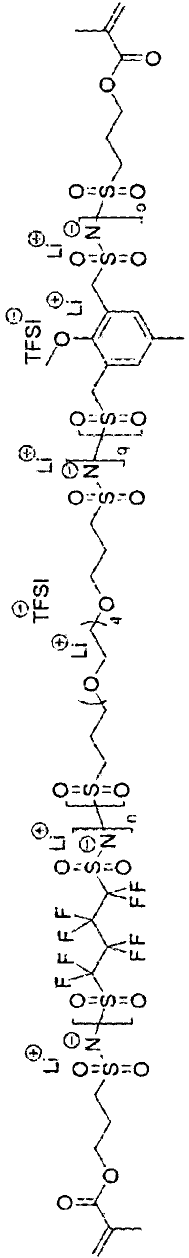

In Formulas 3-a, 4-a, and 4-b above, the n is an integer of 1 to 20, the q is an integer of 1 to 20, and the o is an integer of 1 to 20.
(18) The present invention provides a solid electrolyte including one or more selected from the group consisting of the compounds according to (1) to (14) above and the composite according to (15) to (17) above.
(19) In (18) above, the present invention provides a solid electrolyte further including a crosslinkable compound.
(20) The present invention provides an all-solid-state battery including the solid electrolyte according to (19) above.
(21) The present invention provides a compound represented by Formula A below.

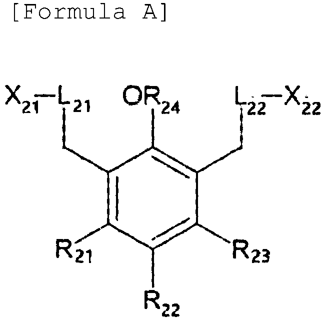

In Formula A above, the R₂₁, R₂₂. and R₂₃ are each independently a hydrogen element, a halogen element, a substituted or unsubstituted C₁-C₁₀ alkyl group, a substituted or unsubstituted C₁-C₁₀ alkoxy group, a substituted or unsubstituted C₂-C₁₀ alkenyl group, or a substituted or unsubstituted C₂-C₁₀ alkynyl group, the R₂₄ is a hydrogen element, or a substituted or unsubstituted C₁-C₁₀ alkyl group, the L₂₁ and L₂₂ are each independently a substituted or unsubstituted C₁-C₁₀ alkylene group, -P(=O)(OR₂₅)-, - (C=O) -, - (S=O)-, or -S(=O)₂-, the X₂₁ and X₂₂ are each independently a halogen element, -NH₂, phosphate(-OP(=O)(OR₂₅)₂), nitrate (-ON(=O)(OR₂₅)), tosylate(-OTs), sulfonate(-OS(=O)₂R₂₅), or carboxylate(-OC(=O)CH₃), and the R₂₅ is a substituted or unsubstituted C₁-C₁₀ alkyl group, a substituted or unsubstituted C₂-C₁₀ alkenyl group, or a substituted or unsubstituted C₂-C₁₀ alkynyl group.
(22) In (21) above, the present invention provides a compound, wherein R₂₂, R₂₄, and R₂₅ are each independently a C₁-C₅ alkyl group.
(23) In (21) or (22) above, the present invention provides a compound, wherein the L₂₁ and L₂₂ are -S(=O)₂-.
(24) In any one of (21) to (23) above, the present invention provides a compound, wherein the X₂₁ and X₂₂ are each independently a hydrogen element or -NH₂.
(25) In any one of (21) to (24) above, the present invention provides a compound, wherein the compound represented by Formula A above is one or more selected from the group consisting of Formulas A-a, A-b, and A-c below.

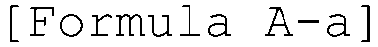

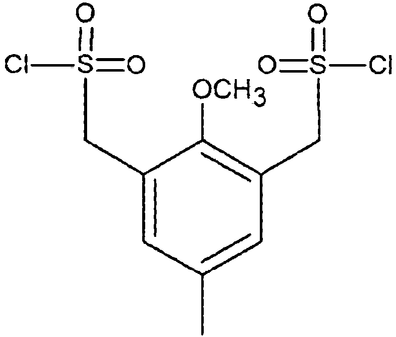

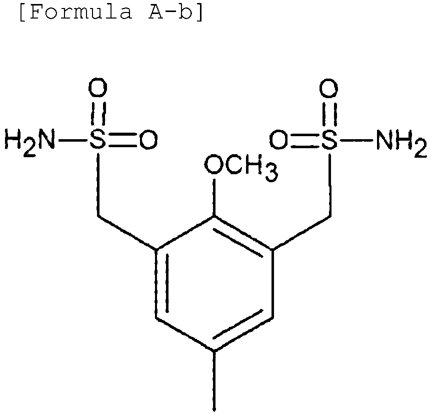

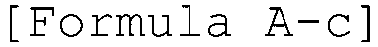

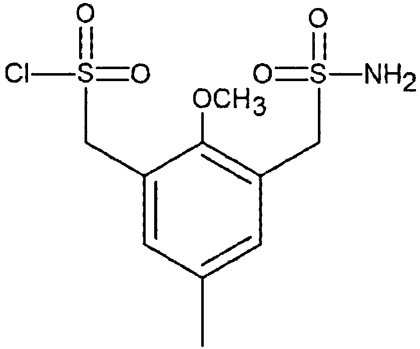

(26) The present invention provides a composite including the compound according to any one of (21) to (25) above, and a lithium compound.
(27) In (26) above, the present invention provides a composite, wherein the composite is represented by Formula B below.

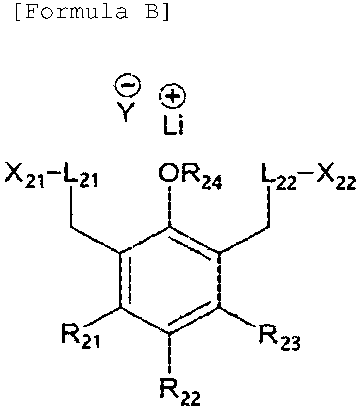

In Formula B above, the R₂₁, R₂₂, and R₂₃ are each independently a hydrogen element, a halogen element, a substituted or unsubstituted C₁-C₁₀ alkyl group, a substituted or unsubstituted C₁-C₁₀ alkoxy group, a substituted or unsubstituted C₂-C₁₀ alkenyl group, or a substituted or unsubstituted C₂-C₁₀ alkynyl group, the R₂₄ is a hydrogen element, or a substituted or unsubstituted C₁-C₁₀ alkyl group, the L₂₁ and L₂₂ are each independently a substituted or unsubstituted C₁-C₁₀ alkylene group, -P(=O)(OR₂₅)-, - (C=O) -, - (S=O)-, or -S(=O)₂-, the X₂₁ and X₂₂ are each independently a halogen element, -NH₂, phosphate(-OP(=O)(OR₂₅)₂), nitrate (-ON(=O)(OR₂₅)), tosylate(-OTs), sulfonate(-OS(=O)₂R₂₅), or carboxylate(-OC(=O)CH₃), the R₂₅ is a substituted or unsubstituted C₁-C₁₀ alkyl group, a substituted or unsubstituted C₂-C₁₀ alkenyl group, or a substituted or unsubstituted C₂-C₁₀ alkynyl group, and the Y⁻ is one or more selected from the group consisting of a halogen anion, BF₄⁻, ClO₄⁻, AlCl₄⁻, AlO₄⁻, PF₆⁻, CF₃SO₃⁻, CH₃CO₂⁻, CF₃CO₂⁻, AsF₆⁻, SbF₆⁻, CH₃SO₃⁻, bis (fluorosulfonyl) imide (FSI⁻) (N(SO₂F)₂⁻), bisperfluoroethanesulfonimide (BETI) (N(SO₂CF₂CF₃)₂⁻), and (bis) trifluoromethanesulfonimide (TFSI) (N(SO₂CF₃)₂⁻).

(28) In (26) or (27) above, the present invention provides a composite, wherein the composite is one or more selected from the group consisting of Formulas B-a, B-b, and B-c below. 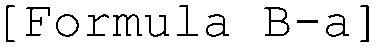 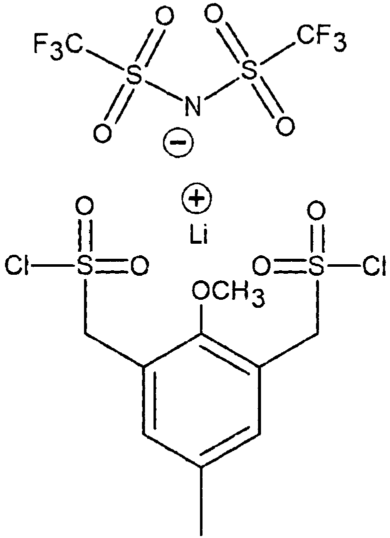 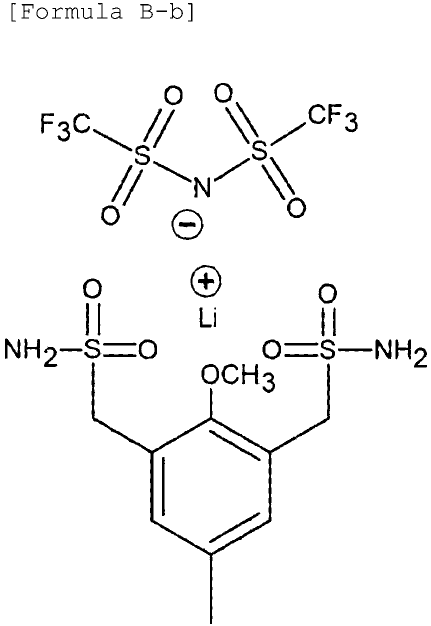 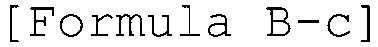 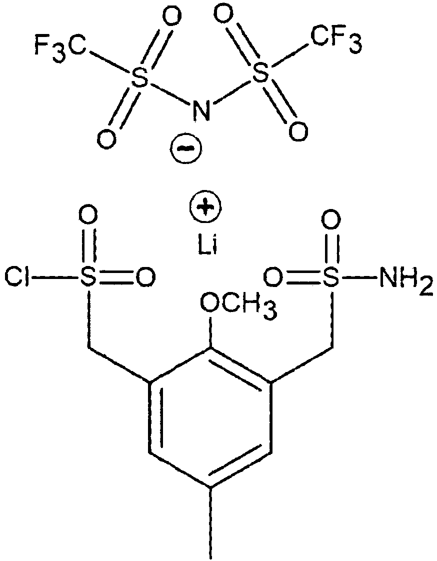
(29) The present invention provides a polymer including a repeating unit derived from the compound according to any one of (21) to (25) above, a repeating unit derived from the composite according to any one of (26) to (28) above, or a combination thereof.
(30) In (29) above, the present invention provides a polymer, wherein the repeating unit derived from the compound according to any one of (21) to (25) above is represented by Formula W-a or W-b below.

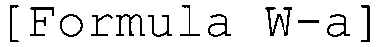

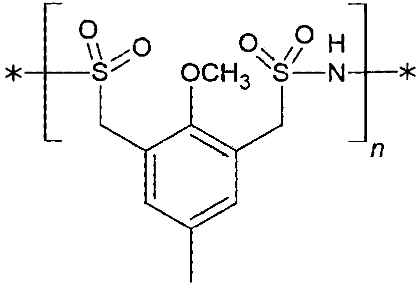

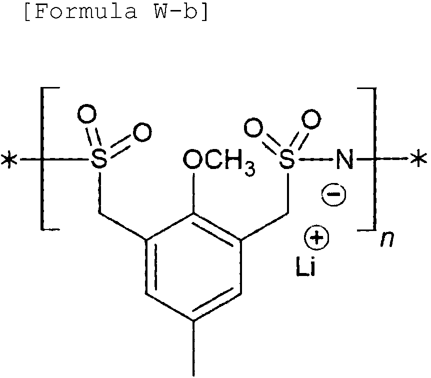

In Formulas W-a and W-b above, the n is an integer of 1 to 300, and the * is a connection site between the repeating units.

(31) In (29) above, the present invention provides a polymer, wherein the repeating unit derived from the composite according to any one of (26) to (28) above is represented by Formula X-a or X-b below.

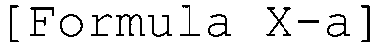

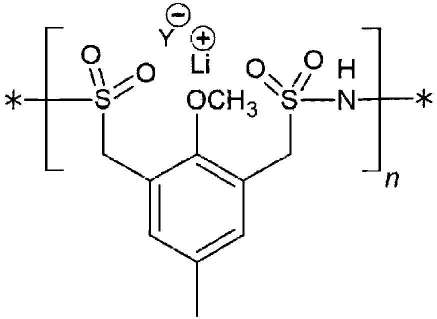

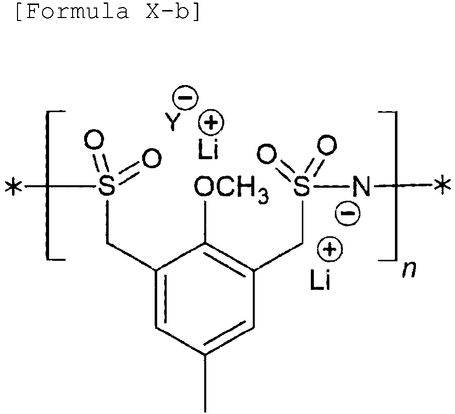

In Formulas X-a and X-b above, the Y⁻ is one or more selected from the group consisting of a halogen anion, BF₄⁻, ClO₄⁻, AlCl₄⁻, AlO₄⁻, PF₆⁻, CF₃SO₃⁻, CH₃CO₂⁻, CF₃CO₂⁻, AsF₆⁻, SbF₆⁻, CH₃SO₃⁻, bis(fluorosulfonyl)imide (FSI⁻) (N(SO₂F)₂⁻), bisperfluoroethanesulfonimide (BETI) (N(SO₂CF₂CF₃)₂⁻), and (bis) trifluoromethanesulfonimide (TFSI) (N(SO₂CF₃)₂⁻), the n is an integer of 1 to 300, and the * is a connection site between the repeating units.

(32) In (29) above, the present invention provides a polymer, wherein the combination of the repeating unit derived from the compound according to any one of (21) to (25) above and the repeating unit derived from the composite according to any one of (26) to (28) above is represented by Formula Y or Z below.

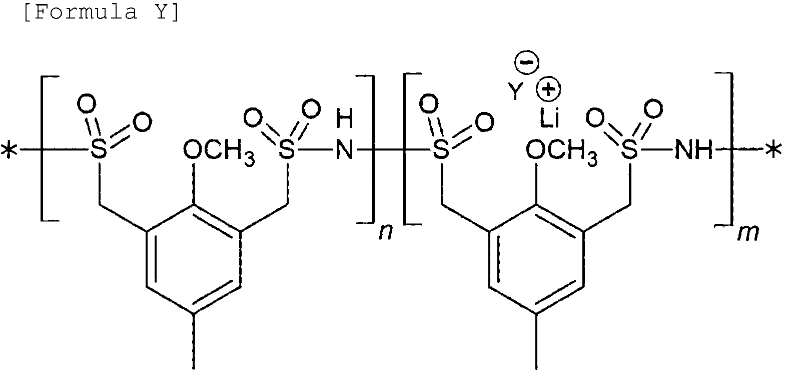

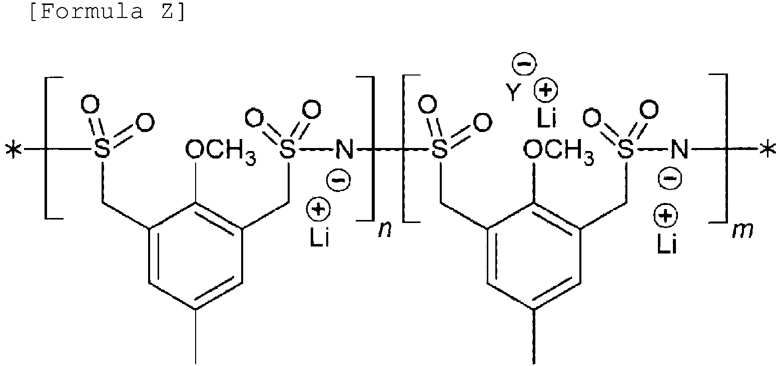

In Formulas Y and Z above, the Y⁻ is one or more selected from the group consisting of a halogen anion, BF₄⁻, ClO₄⁻, AlCl₄⁻, AlO₄⁻, PF₆⁻, CF₃SO₃⁻, CH₃CO₂⁻, CF₃CO₂⁻, AsF₆⁻, SbF₆⁻, CH₃SO₃⁻, bis (fluorosulfonyl) imide (FSI⁻) (N(SO₂F)₂⁻), bisperfluoroethanesulfonimide (BETI) (N(SO₂CF₂CF₃)₂⁻), and (bis) trifluoromethanesulfonimide (TFSI) (N(SO₂CF₃)₂⁻), the n and m are each independently an integer of 1 to 300, and the * is a connection site between the repeating units.

(33) The present invention provides a solid electrolyte including the polymer according to any one of (29) to (32) above.

### ADVANTAGEOUS EFFECTS

According to the present invention, if a compound having a structure having affinity with lithium, which is capable of chelating to lithium ions and a compound which is a lithium salt thereof are applied to an all-solid-state battery, there may be an effect of improving the ion conductivity and ion transference number.

According to the present invention, if a compound having a high lithium ion transference number and a compound which is a lithium salt thereof are applied to an electrolyte, there may be an effect of suppressing the growth of lithium dendritic crystals generated on the surface of a negative electrode.

### MDOE FOR CARRYING OUT THE INVENTION

Hereinafter, the present invention will be described in more detail to facilitate understanding of the present invention. At this time, it will be understood that words or terms used in the specification and claims shall not be interpreted as having the meaning defined in commonly used dictionaries. It will be further understood that the words or terms should be interpreted as having a meaning that is consistent with their meaning in the context of the relevant art and the technical idea of the invention, based on the principle that an inventor may properly define the meaning of the words or terms to best explain the invention.

The terms used herein are only used to describe exemplary embodiments, and are not intended to limit the present invention. Singular expressions include plural expressions unless the context clearly indicates otherwise.

In the present specification, it should be understood that the terms , "include," "comprise," or "have" are intended to specify the presence of stated features, numbers, steps, elements, or combinations thereof, but do not preclude the presence or addition of one or more other features, numbers, steps, elements, or combinations thereof.

The term 'all-solid-state battery' used in the present specification refers to a battery in which all components of the battery are solid, which is distinguished from a liquid electrolyte secondary battery using a liquid electrolyte such as an electrolyte solution and a gel polymer secondary battery using a polymer electrolyte instead of a separator and also using a liquid electrolyte.

In the present specification, the term "substituted or unsubstituted" means being substituted with one or more substituents selected from deuterium, a halogen group, a hydroxy group, an amino group, a thiol group, a nitro group, a nitrile group, a silyl group, and a straight or branched C₁-C₆ alkoxy group, or not having any substituent.

The term "alkyl group" used in the present specification may refer to, unless otherwise stated, a straight or branched acyclic group having 1 to 20 carbon atoms, 1 to 16 carbon atoms, 1 to 12 carbon atoms, 1 to 8 carbon atoms, or 1 to 6 carbon atoms, a cyclic group having 3 to 20 carbon atoms, 3 to 16 carbon atoms, 3 to 12 carbon atoms, 3 to 8 carbon atoms, or 3 to 6 carbon atoms, or a saturated hydrocarbon group to which these are combined.

The term "alkenyl group" used in the present specification may refer to, unless otherwise stated, a straight or branched acyclic group having 2 to 20 carbon atoms, 2 to 16 carbon atoms, 2 to 12 carbon atoms, 2 to 8 carbon atoms, or 2 to 6 carbon atoms having one or more double bonds, a cyclic group having 3 to 20 carbon atoms, 3 to 16 carbon atoms, 3 to 12 carbon atoms, 3 to 8 carbon atoms, or 3 to 6 carbon atoms having one or more double bonds, or an unsaturated hydrocarbon group to which these are combined.

The term "alkynyl group" used in the present specification may refer to, unless otherwise stated, a straight or branched acyclic group having 2 to 20 carbon atoms, 2 to 16 carbon atoms, 2 to 12 carbon atoms, 2 to 8 carbon atoms, or 2 to 6 carbon atoms having one or more triple bonds, a cyclic group having 3 to 20 carbon atoms, 3 to 16 carbon atoms, 3 to 12 carbon atoms, 3 to 8 carbon atoms, or 3 to 6 carbon atoms having one or more triple bonds, or an unsaturated hydrocarbon group to which these are combined.

In the present specification, "*" refers to a bonding position, which is a connection site between repeating units, or a position at which a functional group is bonded to an end, and in the case of the end, it may refer to one or more selected from the group consisting of a hydrogen atom, -CN, -NH₂, a halogen element, a substituted or unsubstituted C₁-C₁₀ alkyl group, a substituted or unsubstituted C₁-C₁₀ alkoxy group, a substituted or unsubstituted C₂-C₁₀ alkenyl group, or a substituted or unsubstituted C₂-C₁₀ alkynyl group.

### Compound

The present invention provides a compound including a repeating unit represented by Formula 1 below, or a compound being a lithium salt thereof.

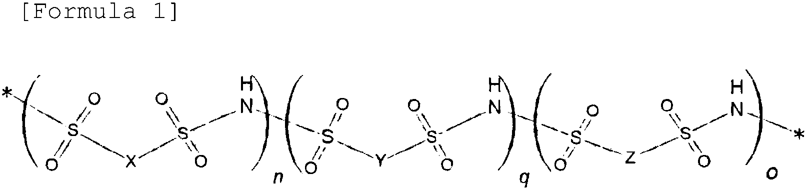

In Formula 1 above, the X, Y, and Z are each independently one or more selected from the group consisting of Formulas a-1, b, and c-1 below, the n is an integer of 1 to 100, the q is an integer of 1 to 100, the o is an integer of 0 to 100, and the * is a connection site between the repeating units, or an end site, and 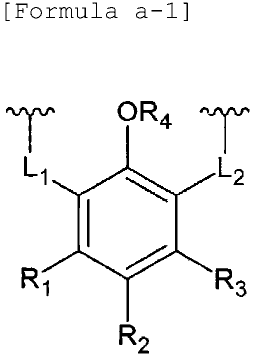 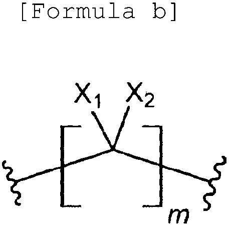 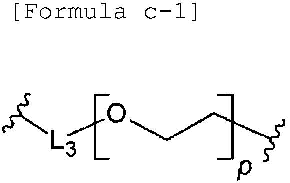 in Formulas a-1, b, and c-1 above, the R₁, R₂, and R₃ are each independently a hydrogen element, a halogen element, -CN, -NO₂, a substituted or unsubstituted C₁-C₁₀ alkyl group, a substituted or unsubstituted C₁-C₁₀ alkoxy group, a substituted or unsubstituted C₂-C₁₀ alkenyl group, a substituted or unsubstituted C₂-C₁₀ alkynyl group, -C(=O)R₅, -P(=O) (OR₅)₂, - P(OR₅)(OR₆), -OP(=O)(OR₅)(OR₆), -S(=O)R₅, or -S(=O)₂R₅, the R₄ is a hydrogen element, or a substituted or unsubstituted C₁-C₁₀ alkyl group, the R₅ and R₆ are each independently a hydrogen element, a substituted or unsubstituted C₁-C₁₀ alkyl group, or a substituted or unsubstituted C₂-C₁₀ alkenyl group, the L₁, L₂, and L₃ are each independently a direct linkage, or a substituted or unsubstituted C₁-C₁₀ alkylene group, the X₁ and X₂ are each independently a hydrogen element, a halogen element, -CN, -NO₂, a substituted or unsubstituted C₁-C₁₀ alkyl group, or a substituted or unsubstituted C₆-C₁₂ aryl group, the m is an integer of 1 to 40, and the p is an integer of 1 to 200.

A typical liquid electrolyte is flammable, and thus, has safety problems such as ignition. A solid electrolyte has no leakage problem of a liquid, and thus, has excellent process stability, and may be processed into a thin film and a film form, and thus, has excellent process convenience, but as described above, the solid electrolyte has a disadvantage of having a significantly lower ion conductivity than the typical liquid electrolyte, and therefore, the present inventors have developed a compound having a structure having affinity with lithium according to the present invention and a solid electrolyte including the compound.

A compound including a repeating unit represented by Formula 1 above and a lithium salt thereof are compounds having a structure having affinity with lithium, which is capable of chelating to lithium ions. The compound including a repeating unit represented by Formula 1 above and the lithium salt thereof contain sulfonimide or an anion of sulfonimide in which proton bonded to a nitrogen element in the sulfonimide is detached, and thus, may have high affinity with lithium ions when used as a material of a solid electrolyte. Meanwhile, in the process of producing a solid electrolyte containing the compound including a repeating unit represented by Formula 1 above, the proton bonded to the nitrogen element in the sulfonimide may be detached. In addition, the anion of sulfonimide has a relatively low electron density compared to an anion of other organic groups such as carboxylate, and thus, has weak electrostatic attraction with lithium ions, so that it is possible to increase the transference number of lithium ions in a solid electrolyte. Particularly, the above lithium salt may have an effect of improving a lithium ion transference number compared to a material hydrogenated from a typical ethylene oxide-based material or a material mixed separately with a lithium salt, e.g., a PEO mixture mixed with a lithium salt.

In addition, the compound including a repeating unit represented by Formula 1 above and the lithium salt thereof contain one or more selected from the group consisting of Formulas a-1, b, and c-1, so that an electron of the anion of sulfonimide may be delocalized to further improve the ion conductivity of a solid electrolyte. In addition, the compound according to the present invention also serves as a typical lithium salt in a solid electrolyte, and thus, may replace the typical lithium salt, so that it is possible to reduce manufacturing costs of an all-solid-state battery.

In addition, a lithium metal negative electrode, which is a negative electrode material in the spotlight in the next-generation battery including a typical solid-state battery, has the chronic problems such as shortened lifespan and side reactions due to the formation of lithium dendrites, i.e., lithium dendritic crystals, and a solid electrolyte including the compound of the present invention has a high lithium ion transference number, so that it is possible to suppress the dendrite phenomenon, thereby improving the lifespan of a battery. In addition, in a fast charging environment, the formation of lithium dendrites is accelerated, so that it has been difficult to introduce fast charging in a typical lithium metal battery composition, but in the case of an all-solid-state battery containing the compound of the present invention, the dendrite phenomenon is suppressed, making fast charging possible. The compound of the present invention may be a compound including a repeating unit represented by Formula 1 and a compound which is a lithium salt thereof, and the lithium salt may be a compound including a repeating unit represented by Formula 2 to be described later. Particularly, results of an experiment to be described later show that the above lithium salt has a relatively high level of lithium transference number, so that it is possible to more effectively suppress the formation of dendrites.

In addition, according to an embodiment of the present invention, the X, Y, and Z may each independently be one or more selected from the group consisting of Formulas a-1, b, and c-1 below, and at least one of the X, Y, and Z may include Formula a-1. The compound including a repeating unit represented by Formula 1 above and the lithium salt thereof are ionic conductive polymers, and include a structure including an aromatic ring in which an alkoxy group is substituted at one carbon position of a benzene ring, and an alkyl group substituted by a sulfonyl group is positioned on both sides of an ortho position, and thus, may have a structure having affinity with lithium, which is capable of chelating to lithium ions. The present invention provides a compound having lithium ion conductivity, which is capable of increasing the mobility of lithium ions through the above-described structure having affinity with lithium, and when the compound is used as a material of a solid electrolyte, there may be an effect of improving ion conductivity.

In addition, according to an embodiment of the present invention, the R₂ and R₄ may be C₁-C₅ alkyl groups.

In addition, according to an embodiment of the present invention, the L₁, L₂, and L₃ may be C₁-C₅ alkylene groups.

In addition, according to an embodiment of the present invention, the X₁ and X₂ may each independently be a hydrogen element, or a halogen element, wherein the halogen element may be one or more selected from the group consisting of F, Cl, Br, and I. The compound including a repeating unit represented by Formula 1 above and the lithium salt thereof may include an alkylene group in which a halogen element has been substituted, and preferably, the halogen element may be fluorine. If the halogen element is fluorine, the fluorine, which has high electronegativity, may delocalize an anion of neighboring sulfonimide, thereby lowering the electron density of the sulfonimide, and as a result, the electrostatic attraction between lithium ions and a solid electrolyte may be alleviated. Accordingly, the above compound may move dissociated lithium ions in an electrolyte with higher efficiency, and thus, may have higher ion conductivity than a typical alkylene oxide-based polymer, and when used in an all-solid-state battery, may have electrochemical stability even at high voltages.

In addition, according to an embodiment of the present invention, in Formula 1, and Formulas a-1, b, and c-1 above, it may be that the n is an integer of 1 to 20, the q is an integer of 1 to 20, the o is an integer of 1 to 20, the m is an integer of 1 to 10, and the p is an integer of 1 to 50.

In addition, according to an embodiment of the present invention, the compound including a repeating unit represented by Formula 1 above may be represented by Formula 1-a or 1-b below.

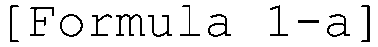

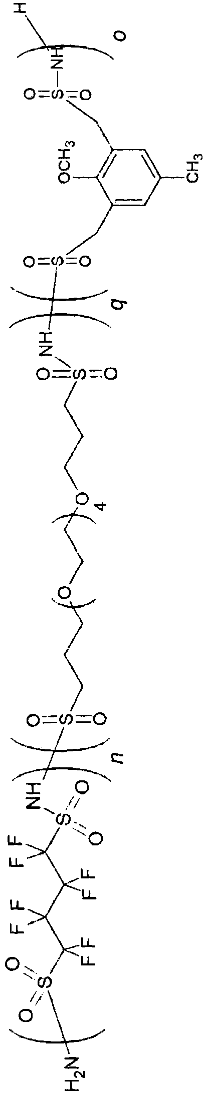

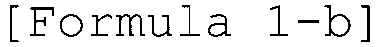

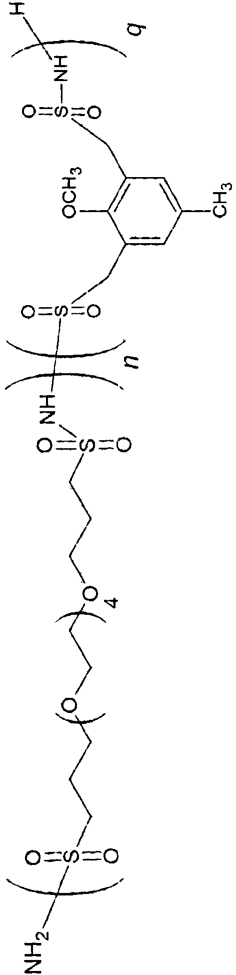

In Formulas 1-a and 1-b above, the n is an integer of 1 to 20, the q is an integer of 1 to 20, and the o is an integer of 1 to 20.

If the compound including a repeating unit represented by Formula 1 above is represented by Formula 1-a or 1-b above, the compound may have higher lithium ion conductivity than a polymer material of a solid electrolyte, which has been typically used.

According to an embodiment of the present invention, the compound may include one or more functional groups selected from the group consisting of Formulas d to g below at at least one end site among the end sites.

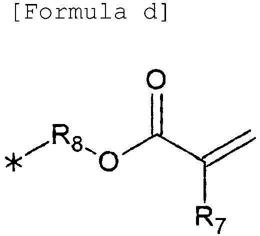

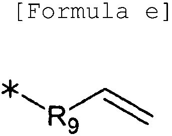

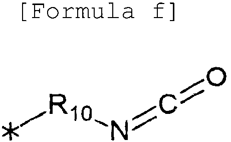

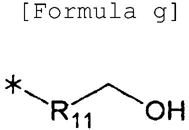

In Formulas d to g above, the R₇ is a hydrogen element, or a substituted or unsubstituted C₁-C₁₀ alkyl group, the R₈ to R₁₁ are each independently a direct linkage, a substituted or unsubstituted C₁-C₁₀ alkylene group, or -R₁₂-O-R₁₃-, the R₁₂ and R₁₃ are each independently a direct linkage, or a substituted or unsubstituted C₁-C₁₀ alkylene group, and the * is a bonding position.

The compound contains one or two or more functional groups selected from the group consisting of Formulas d to g at at least one end of both ends of the repeating unit, and thus, may facilitate the formation of a network between the repeating units in the compound, and may significantly increase the weight average molecular weight of the polymer through the formation of the network, and accordingly, if the compound and the lithium salt thereof are applied to a solid electrolyte, it is possible to improve the mechanical strength of the solid electrolyte.

In addition, according to an embodiment of the present invention, in the Formulas d to g above, the R₇ is a C₁-C₆ alkyl group, the R₈ to R₁₁ are each independently a direct linkage, a C₁-C₆ alkylene group substituted or unsubstituted with a fluorine element, or -R₁₂-O-R₁₃-, and the R₁₂ and R₁₃ are each independently a direct linkage, or a C₁-C₆ alkylene group substituted or unsubstituted with a fluorine element. Specifically, a functional group of Formulas d to g may be one or more selected from the group consisting of acrylate, vinyl group, isocyanate, and alcohol structures.

Meanwhile, in a process of polymerizing the compound included in the solid electrolyte of the present invention, in addition to the compound including a repeating unit represented by Formula 1 above and one or more functional groups selected from the group consisting of Formulas d to g below at at least one end of both ends of the above repeating unit, and the lithium salt thereof, a crosslinkable compound may be further included. The crosslinkable compound may be one or more selected from the group consisting of polyfunctional acrylate, vinyl group, isocyanate, and alcohol structures.

For example, if a functional group of Formula d or Formula e having an acrylate or vinyl group structure is positioned at one end of the repeating unit, a crosslinkable compound containing pentaerythritol tetraacrylate (PETA) may further be added to form a network between the repeating units. At this time, the acrylate, the vinyl group, and the PETA contain a double bond structure, so that radical polymerization may be formed between the functional group of Formula d or Formula e above and the double bond of the crosslinkable compound.

In addition, for example, if a functional group of Formula g above having an alcohol structure (-OH) is positioned at an end of the repeating unit, a crosslinkable compound containing hexamethylene diisocyanate (1,6-diisocyanatohexane) may be added to form a network between the repeating units. In addition, if a functional group of Formula f above having an isocyanate structure (-NCO) is positioned at an end of the repeating unit, a crosslinkable compound containing glycerol or propane-1,2,3-tricarboxylic acid may be added to form a network between the repeating units. At this time, the -OH group and the -NCO group may form a bond of a -NHCOO- structure through an additive polymerization reaction.

In addition, according to an embodiment of the present invention, the compound may be represented by Formula 1-c below.

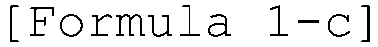

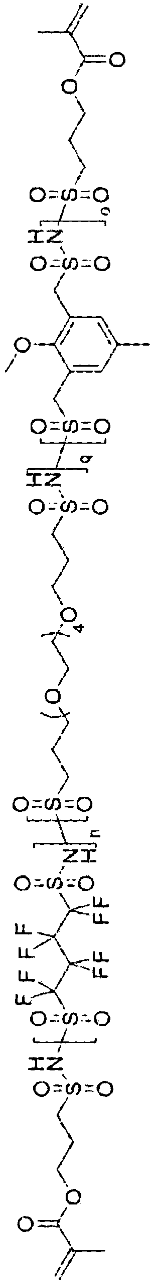

In Formula 1-c above, the n is an integer of 1 to 20, the q is an integer of 1 to 20, and the o is an integer of 1 to 20.

If the compound, specifically the compound including a repeating unit represented by Formula 1, is represented by Formula 1-c above, the compound may have higher lithium ion conductivity than a polymer material of a solid electrolyte, which has been typically used.

According to an embodiment of the present invention, a lithium salt of the compound including a repeating unit represented by Formula 1 above may be a compound including a repeating unit represented by Formula 2 below.

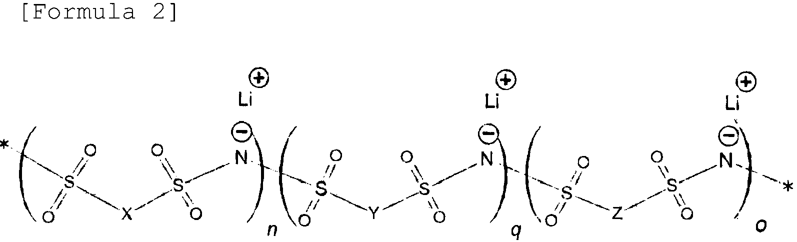

In Formula 2 above, the X, Y, and Z are each independently one or more selected from the group consisting of Formulas a-1, b, and c-1 below, the n is an integer of 1 to 100, the q is an integer of 1 to 100, the o is an integer of 0 to 100, and the * is a connection site between the repeating units, and 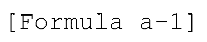 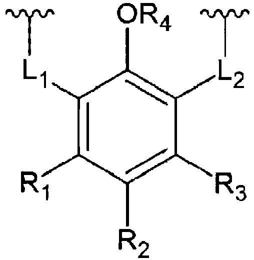 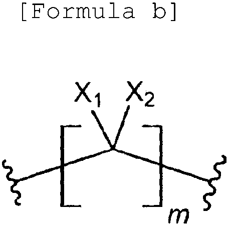 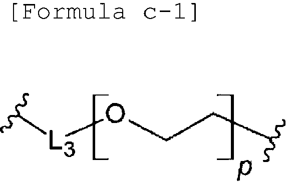 in Formulas a-1, b, and c-1 above, the R₁, R₂, and R₃ are each independently a hydrogen element, a halogen element, -CN, -NO₂, a substituted or unsubstituted C₁-C₁₀ alkyl group, a substituted or unsubstituted C₁-C₁₀ alkoxy group, a substituted or unsubstituted C₂-C₁₀ alkenyl group, a substituted or unsubstituted C₂-C₁₀ alkynyl group, -C(=O)R₅, -P(=O) (OR₅)₂, - P(OR₅)(OR₆), -OP(=O)(OR₅)(OR₆), -S(=O)R₅, or -S(=O)₂R₅, the R₄ is a hydrogen element, or a substituted or unsubstituted C₁-C₁₀ alkyl group, the R₅ and R₆ are each independently a hydrogen element, a substituted or unsubstituted C₁-C₁₀ alkyl group, or a substituted or unsubstituted C₂-C₁₀ alkenyl group, the L₁, L₂, and L₃ are each independently a direct linkage, or a substituted or unsubstituted C₁-C₁₀ alkylene group, the X₁ and X₂ are each independently a hydrogen element, a halogen element, -CN, -NO₂, a substituted or unsubstituted C₁-C₁₀ alkyl group, or a substituted or unsubstituted C₆-C₁₂ aryl group, the m is an integer of 1 to 40, and the p is an integer of 1 to 200.

The compound including a repeating unit represented by Formula 2 above is the compound including a repeating unit represented by Formula 1 above that has been lithiated, and may have an effect of improving a lithium ion transference number.

In addition, according to an embodiment of the present invention, the compound including a repeating unit represented by Formula 2 above may be a compound represented by Formula 2-a or 2-b below.

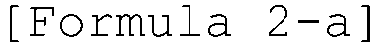

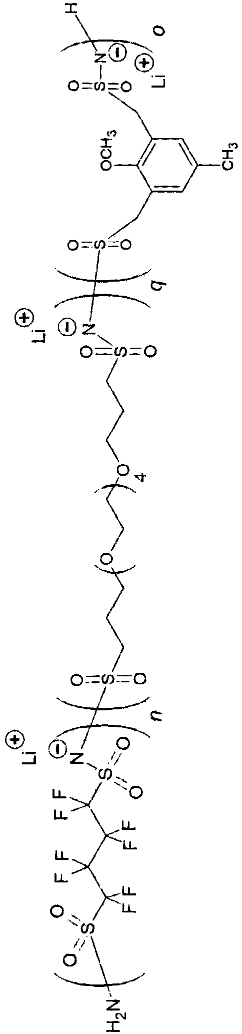

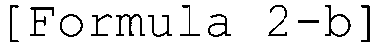

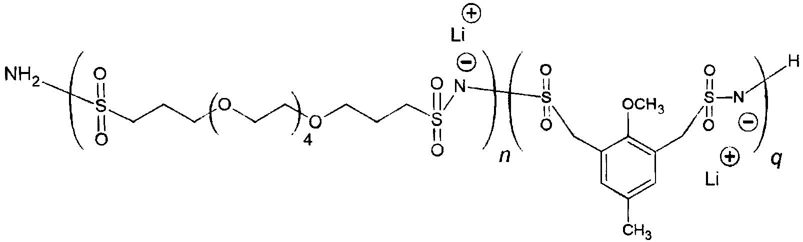

In Formula 2-a and 2-b above, the n is an integer of 1 to 20, the q is an integer of 1 to 20, and the o is an integer of 1 to 20.

If the compound including a repeating unit represented by Formula 2 above is represented by Formula 2-a or 2-b above, the compound may have higher lithium ion conductivity than a polymer material of a solid electrolyte, which has been typically used.

According to an embodiment of the present invention, the lithium salt compound may include one or more functional groups selected from the group consisting of Formulas d to g below at at least one end site among the end sites.

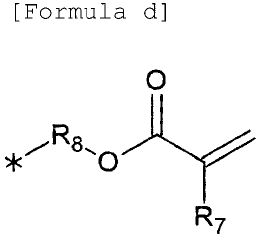

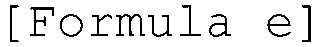

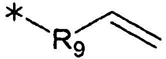

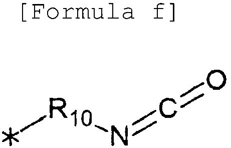

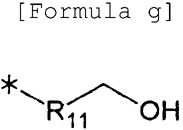

In Formulas d to g above, the R₇ is a hydrogen element, or a substituted or unsubstituted C₁-C₁₀ alkyl group, the R₈ to R₁₁ are each independently a direct linkage, a substituted or unsubstituted C₁-C₁₀ alkylene group, or -R₁₂-O-R₁₃-, the R₁₂ and R₁₃ are each independently a direct linkage, or a substituted or unsubstituted C₁-C₁₀ alkylene group, and the * is a bonding position.

In addition, according to an embodiment of the present invention, the compound including a repeating unit represented by Formula 2 above may be represented by Formula 2-c below.

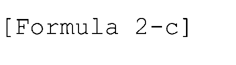

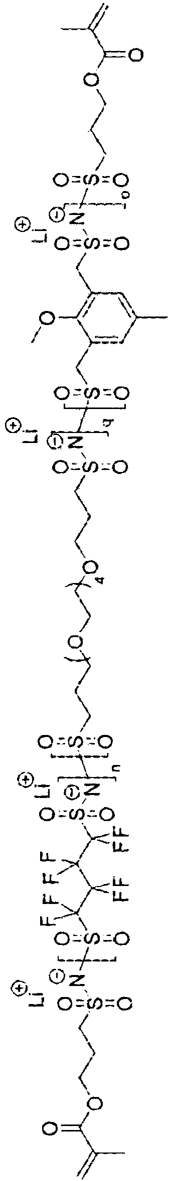

In Formula 2-c above, the n is an integer of 1 to 20, the q is an integer of 1 to 20, and the o is an integer of 1 to 20.

If the compound, specifically the compound including a repeating unit represented by Formula 2, is represented by Formula 2-c above, the compound may have higher lithium ion conductivity than a polymer material of a solid electrolyte, which has been typically used.

### Composite

The present invention provides a composite including the compound according to the present invention, and a lithium compound.

Specifically, the present invention provides a composite including one or more compounds selected from the group consisting of the compound of Formula 1 above and a lithium salt thereof, and a lithium compound.

The composite may be a composite including a repeating unit represented by Formula 3 below or a repeating unit represented by Formula 4 below.

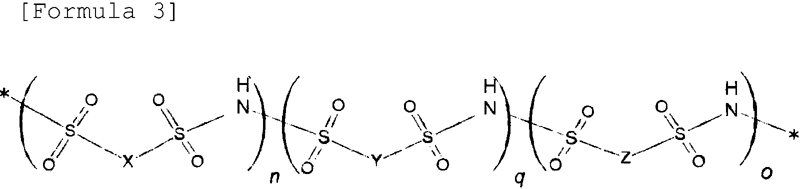

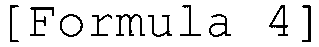

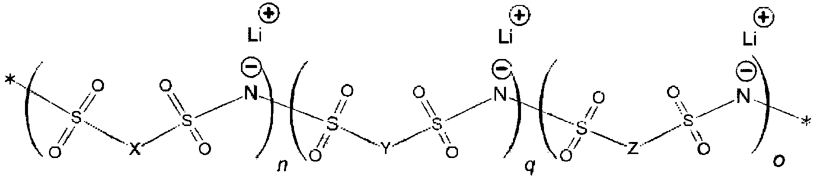

In Formulas 3 and 4 above, the X, Y, and Z are each independently one or more selected from the group consisting of Formulas a-2, b, c-1 and c-2 below, wherein at least one of the X, Y and Z necessarily includes Formula a-2 or c-2, the n is an integer of 1 to 100, the q is an integer of 1 to 100, the o is an integer of 0 to 100, and the * is a connection site between the repeating units, and

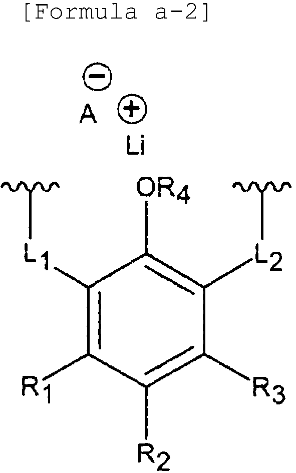

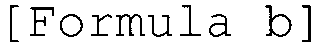

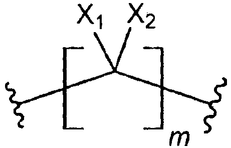

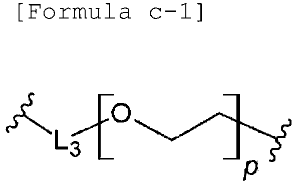

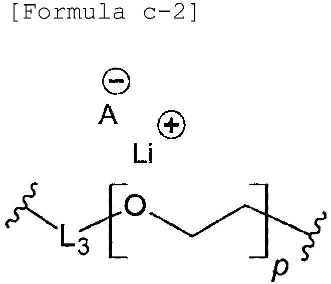

in Formulas a-2, b, c-1, and c-2 above, the R₁, R₂, and R₃ are each independently a hydrogen element, a halogen element, -CN, -NO₂, a substituted or unsubstituted C₁-C₁₀ alkyl group, a substituted or unsubstituted C₁-C₁₀ alkoxy group, a substituted or unsubstituted C₂-C₁₀ alkenyl group, a substituted or unsubstituted C₂-C₁₀ alkynyl group, -C(=O)R₅, -P(=O)(OR₅)₂, - P(OR₅)(OR₆), -OP(=O)(OR₅)(OR₆), -S(=O)R₅, or -S(=O)₂R₅, the R₄ is a hydrogen element, or a substituted or unsubstituted C₁-C₁₀ alkyl group, the R₅ and R₆ are each independently a hydrogen element, a substituted or unsubstituted C₁-C₁₀ alkyl group, or a substituted or unsubstituted C₂-C₁₀ alkenyl group, the L₁, L₂, and L₃ are each independently a direct linkage, or a substituted or unsubstituted C₁-C₁₀ alkylene group, the X₁ and X₂ are each independently a hydrogen element, a halogen element, -CN, -NO₂, a substituted or unsubstituted C₁-C₁₀ alkyl group, or a substituted or unsubstituted C₆-C₁₂ aryl group, the A⁻ is one or more selected from the group consisting of a halogen anion, BF₄⁻, ClO₄⁻, AlCl₄⁻, AlO₄⁻, PF₆⁻, CF₃SO₃⁻, CH₃CO₂⁻, CF₃CO₂⁻, AsF₆⁻, SbF₆⁻, CH₃SO₃⁻, bis (fluorosulfonyl) imide (FSI⁻) (N(SO₂F)₂⁻), bisperfluoroethanesulfonimide (BETI) (N(SO₂CF₂CF₃)₂⁻), and (bis)trifluoromethanesulfonimide (TFSI) (N(SO₂CF₃)₂⁻), the m is an integer of 1 to 40, and the p is an integer of 1 to 200.

According to an embodiment of the present invention, the composite including a repeating unit represented by Formula 3 above or a repeating unit represented by Formula 4 above may include one or more of the compound including a repeating unit represented by Formula 1 above or the lithium salt thereof, and a lithium compound, and at least one of the compound including a repeating unit represented by Formula 1 above or the lithium salt thereof and the lithium compound are bonded by electrostatic attraction. By further including the lithium compound, the composite of the present invention may increase the transference number of lithium ions, thereby improving the ion conductivity, and may achieve an effect of reducing diffusion resistance of the lithium ions, thereby implementing an effect of improving cycle capacity properties of a lithium secondary battery.

According to an embodiment of the present invention, the X, Y, and Z are each independently one or two or more selected from the group consisting of Formulas a-2, b, c-1, c-2 below, wherein at least one of the X, Y and Z may necessarily include Formula a-2 or c-2. The L₁, L₂, and L₃ may be C₁-C₅ alkylene groups, and the X₁ to X₄ may each independently be a hydrogen element, or a halogen element, wherein the halogen element may be one or more selected from the group consisting of F, Cl, Br, and I. In addition, it may be that the n is an integer of 1 to 20, the q is an integer of 1 to 20, the o is an integer of 1 to 20, the m is an integer of 1 to 10, and the p is an integer of 1 to 50. An effect according to the definition of the substituent described above is the same as the effect of the compound including a repeating unit represented by Formula 1 and the lithium salt thereof described above.

The lithium compound may be expressed as Li⁺ Y⁻. An anion Y⁻ of the lithium compound is one or more selected from the group consisting of a halogen anion, BF₄⁻, ClO₄⁻, AlCl₄⁻, AlO₄⁻, PF₆⁻, CF₃SO₃⁻, CH₃CO₂⁻, CF₃CO₂⁻, AsF₆⁻, SbF₆⁻, CH₃SO₃⁻, bis(fluorosulfonyl)imide (FSI⁻)(N(SO₂F)₂⁻), bisperfluoroethanesulfonimide (BETI) (N(SO₂CF₂CF₃)₂⁻), and (bis)trifluoromethanesulfonimide (TFSI) (N(SO₂CF₃)₂⁻), and specifically, the anion Y⁻ of the lithium compound may be a halogen anion.

According to an embodiment of the present invention, the R₂ and R₄ may be C₁-C₅ alkyl groups. In the structure of Formula 3 or Formula 4 above, if R₄ is substituted by a C₁-C₅ alkyl group, so that the above composite contains an alkoxy group, the above lithium compound may be positioned in close proximity to the alkoxy group by electrostatic attraction, and specifically the lithium compound may be positioned in the form of Li⁺Y⁻.

In addition, the lithium compound included in the above composite may be included in an amount of 1 wt% to 40 wt% in the composite. The composite includes the lithium compound in a content of the above-described range, and thus, may increase a lithium ion transference number, and may reduce battery resistance, so that an effect of improving cycle capacity properties may be implemented.

According to an embodiment of the present invention, the composite may include one or more functional groups selected from the group consisting of Formulas d to g below at at least one end site among the end sites.

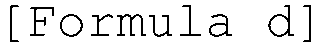

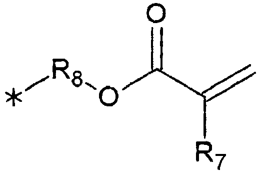

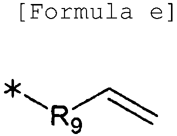

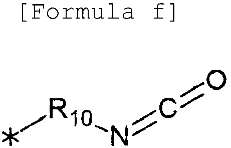

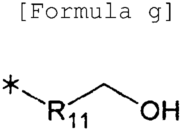

In Formulas d to g above, the R₇ is a hydrogen element, or a substituted or unsubstituted C₁-C₁₀ alkyl group,
the R₈ to R₁₁ are each independently a direct linkage, a substituted or unsubstituted C₁-C₁₀ alkylene group, or -R₁₂-O-R₁₃-, the R₁₂ and R₁₃ are each independently a direct linkage, or a substituted or unsubstituted C₁-C₁₀ alkylene group, and the * is a bonding position.

According to an embodiment of the present invention, the composite may be a composite represented by one or more selected from the group consisting of Formulas 3-a, 4-a, and 4-b below.

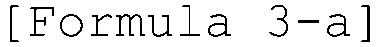

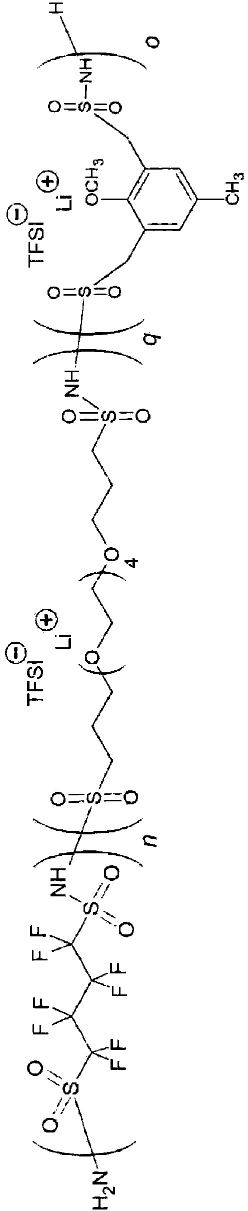

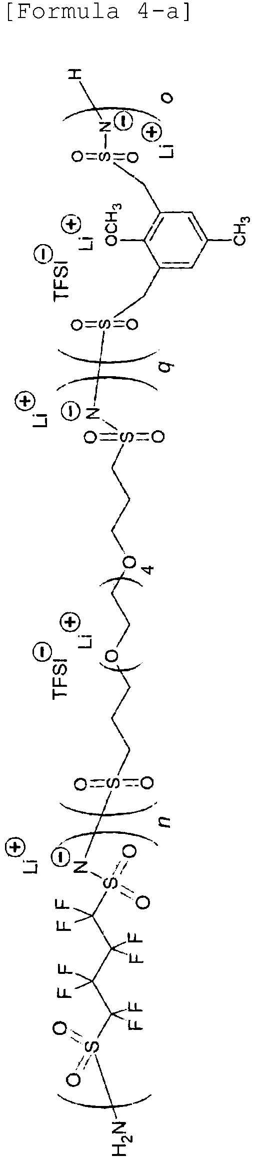

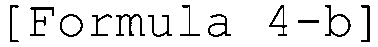

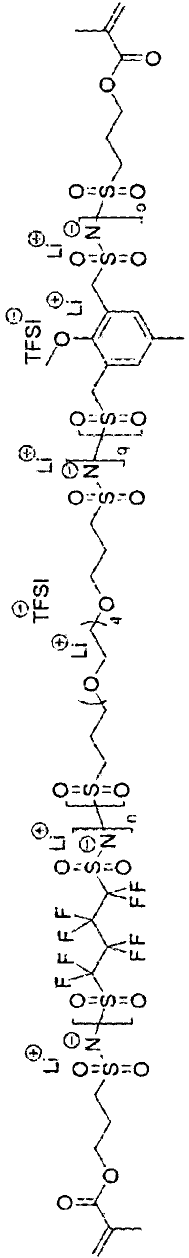

In Formulas 3-a, 4-a, and 4-b above, the n is an integer of 1 to 20, the q is an integer of 1 to 20, and the o is an integer of 1 to 20.

If the composite including a repeating unit represented by Formula 3 above or a repeating unit represented by Formula 4 above is the composite represented by one or more selected from the group consisting of Formulas 3-a, 4-a, and 4-b above, the composite may have higher lithium ion conductivity than a polymer material of a solid electrolyte, which has been typically used.

### Compound (monomer)

The present invention provides a compound represented by Formula A below.

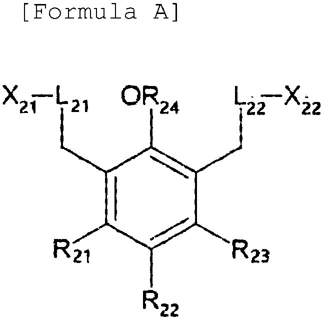

In Formula A above, the R₂₁, R₂₂. and R₂₃ are each independently a hydrogen element, a halogen element, a substituted or unsubstituted C₁-C₁₀ alkyl group, a substituted or unsubstituted C₁-C₁₀ alkoxy group, a substituted or unsubstituted C₂-C₁₀ alkenyl group, or a substituted or unsubstituted C₂-C₁₀ alkynyl group, the R₂₄ is a hydrogen element, or a substituted or unsubstituted C₁-C₁₀ alkyl group, the L₂₁ and L₂₂ are each independently a substituted or unsubstituted C₁-C₁₀ alkylene group, -P(=O)(OR₂₅)-, - (C=O) -, - (S=O)-, or -S(=O)₂-, the X₂₁ and X₂₂ are each independently a halogen element, -NH₂, phosphate (-OP(=O)(OR₂₅)₂), nitrate (-ON(=O)(OR₂₅)), tosylate(-OTs), sulfonate(-OS(=O)₂R₂₅), or carboxylate(-OC(=O)CH₃), and the R₂₅ is a substituted or unsubstituted C₁-C₁₀ alkyl group, a substituted or unsubstituted C₂-C₁₀ alkenyl group, or a substituted or unsubstituted C₂-C₁₀ alkynyl group.

The compound represented by Formula A above may be a monomer. The compound represented by Formula A may be an aromatic ring material in which an alkoxy group is substituted at one carbon position of a benzene ring, and an alkyl group substituted by a sulfonyl group is positioned on both sides of an ortho position. The above compound, like a crown ether-based compound to which the molecular recognition concept of supermolecular chemistry is applied, may have high selectivity for Li⁺ of a target material on the basis of a structure-specific interaction based on structural properties capable of chelating to lithium ions. The present invention provides a compound having lithium ion conductivity, which is capable of increasing the mobility (lithium ion transference number) of lithium ions through the above-described structure having affinity with lithium, and when the compound is used as a material of a solid electrolyte, there may be an effect of improving ion conductivity.

According to an embodiment of the present invention, in Formula A above, the R₂₂, R₂₄, and R₂₅ may be C₁-C₅ alkyl groups.

According to an embodiment of the present invention, in Formula A above, the X₂₁ and X₂₂ may each independently be a hydrogen element or -NH₂. The compound represented by Formula A above may form an ion conductive polymer as a monomer, and when the compound represented by Formula A above contains a halogen element, for example, at least one of F, Cl, I, and Br, or NH₂, a condensation reaction may occur between the halogen element and NH₂ to form the ion conductive polymer.

According to an embodiment of the present invention, in Formula A above, the L₂₁ and L₂₂ may be -S(=O)₂-. Specifically, if the compound represented by Formula A above contains a sulfonyl group in the L₂₁ and L₂₂, and the X₂₁ and X₂₂ contain -NH₂, the compound represented by Formula A above contains sulfonimide or an anion of sulfonimide in which proton bonded to a nitrogen element in the sulfonimide is detached, and thus, may have high affinity with lithium ions when used as a material of a solid electrolyte. Meanwhile, in the process of producing a solid electrolyte containing the compound including a repeating unit represented by Formula 1 above, the proton bonded to the nitrogen element in the sulfonimide may be detached. In addition, in this case, the anion of sulfonimide has a relatively low electron density compared to an anion of other organic groups such as carboxylate, and thus, has weak electrostatic attraction with lithium ions, so that it is possible to achieve an effect of improving the transference number of lithium ions, i.e., ion conductivity. In addition, if the compound represented by Formula A above contains an alkoxy group, oxygen of the alkoxy group together with two sulfonyl groups adjacent to an ortho position is capable of chelating to lithium ions, thereby improving the lithium affinity, and as a result, it is possible to achieve an effect of improving the lithium ion conductivity of a solid electrolyte to which the material is applied. Particularly, the above lithium salt may have an effect of improving a lithium ion transference number compared to a material hydrogenated from a typical ethylene oxide-based material or a material mixed separately with a lithium salt, e.g., a PEO mixture mixed with a lithium salt.

In addition, according to an embodiment of the present invention, the compound represented by Formula A above may be one or more compounds selected from the group consisting of Formulas A-a, A-b, and A-c below.

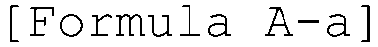

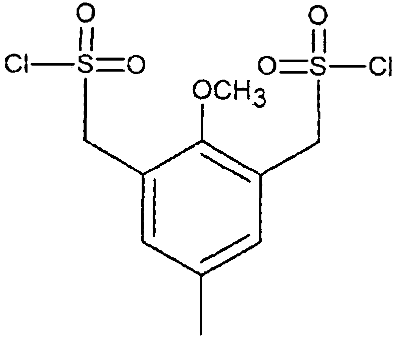

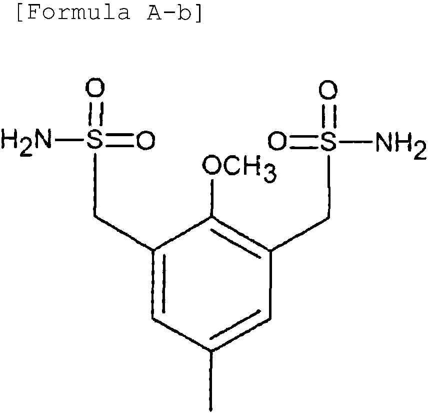

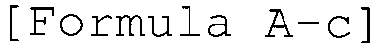

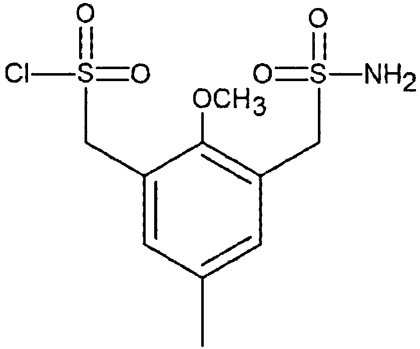

If the compound represented by Formula A above is one or more selected from the group consisting of Formulas A-a, A-b, and A-c above, the compound may have higher lithium ion conductivity than a polymer material of a solid electrolyte, which has been typically used.

### Composite (monomer)

The present invention provides a composite including the compound represented by Formula A above and a lithium compound. By further including the lithium compound, the composite of the present invention may increase the transference number of lithium ions, thereby improving the ion conductivity, and may achieve an effect of reducing diffusion resistance of the lithium ions, thereby implementing an effect of improving cycle capacity properties of a lithium secondary battery.

According to an embodiment of the present invention, the composite may be a composite represented by Formula B below. In the composite represented by Formula B above, the compound represented by Formula A above and the lithium compound are bonded by electrostatic attraction.

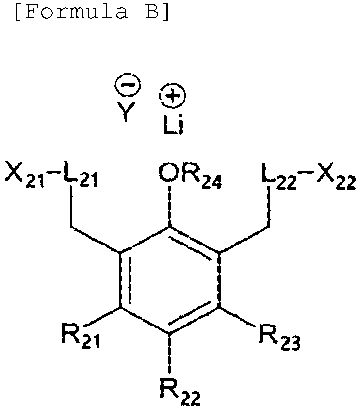

In Formula B above, the R₂₁, R₂₂. and R₂₃ are each independently a hydrogen element, a halogen element, a substituted or unsubstituted C₁-C₁₀ alkyl group, a substituted or unsubstituted C₁-C₁₀ alkoxy group, a substituted or unsubstituted C₂-C₁₀ alkenyl group, or a substituted or unsubstituted C₂-C₁₀ alkynyl group, the R₂₄ is a hydrogen element, or a substituted or unsubstituted C₁-C₁₀ alkyl group, the L₂₁ and L₂₂ are each independently a substituted or unsubstituted C₁-C₁₀ alkylene group, -P(=O)(OR₂₅)-, - (C=O) -, - (S=O)-, or -S(=O)₂-, the X₂₁ and X₂₂ are each independently a halogen element, -NH₂, phosphate (-OP(=O)(OR₂₅)₂), nitrate (-ON(=O)(OR₂₅)), tosylate(-OTs), sulfonate(-OS(=O)₂R₂₅), or carboxylate (-OC(=O)CH₃), the R₂₅ is a substituted or unsubstituted C₁-C₁₀ alkyl group, a substituted or unsubstituted C₂-C₁₀ alkenyl group, or a substituted or unsubstituted C₂-C₁₀ alkynyl group, and the Y⁻ is one or more selected from the group consisting of a halogen anion, BF₄⁻, ClO₄⁻, AlCl₄⁻, AlO₄⁻, PF₆⁻, CF₃SO₃⁻, CH₃CO₂⁻, CF₃CO₂⁻, AsF₆⁻, SbF₆⁻, CH₃SO₃⁻, bis (fluorosulfonyl) imide (FSI⁻)(N(SO₂F)₂⁻), bisperfluoroethanesulfonimide (BETI) (N(SO₂CF₂CF₃)₂⁻), and (bis)trifluoromethanesulfonimide (TFSI) (N(SO₂CF₃)₂⁻).

According to an embodiment of the present invention, in Formula B above, the R₂₂, R₂₄, and R₂₅ may be C₁-C₅ alkyl groups. In addition, in Formula B above, the L₂₁ and L₂₂ may be -S(=O)₂-, and the X₂₁ and X₂₂ may each independently be a halogen element or -NH₂. The halogen element may be any one of F, Cl, I, and Br. An effect according to the definition of the substituent above is the same as the effect of the compound represented by Formula A above.

The lithium compound may be expressed as Li⁺ Y⁻. The anion Y⁻ of the lithium compound may be one or more selected from the group consisting of a halogen anion, BF₄⁻, ClO₄⁻, AlCl₄⁻, AlO₄⁻, PF₆⁻, CF₃SO₃⁻, CH₃CO₂⁻, CF₃CO₂⁻, AsF₆⁻, SbF₆⁻, CH₃SO₃⁻, bis(fluorosulfonyl)imide (FSI⁻) (N(SO₂F)₂⁻), bisperfluoroethanesulfonimide (BETI) (N(SO₂CF₂CF₃)₂⁻), and (bis)trifluoromethanesulfonimide (TFSI) (N(SO₂CF₃)₂⁻), and specifically, the anion Y⁻ of the lithium compound may be a halogen anion.

According to an embodiment of the present invention, in the structure of Formula B above, if R₂₄ is substituted by an alkyl group, so that the above composite contains an alkoxy group, the above lithium compound may be positioned in close proximity to the alkoxy group by electrostatic attraction, and specifically the lithium compound may be positioned in the form of Li⁺ Y⁻.

In addition, the lithium compound included in the above composite may be included in an amount of 1 wt% to 75 wt% in the composite. If the lithium compound is included in a content of the above-described range, 1 mole to 4 moles of lithium cations may be present per mole of the composite, thereby increasing a lithium ion transference number, and diffusion resistance of lithium ions may be reduced, thereby implementing an effect of improving cycle capacity properties.

According to an embodiment of the present invention, the composite may be one or more composites selected from the group consisting of Formulas B-a, B-b, and B-c.

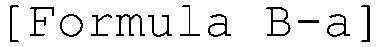

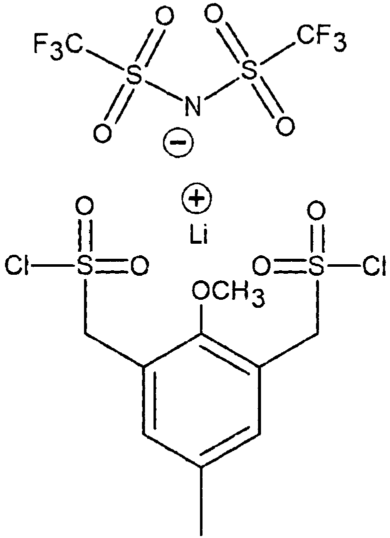

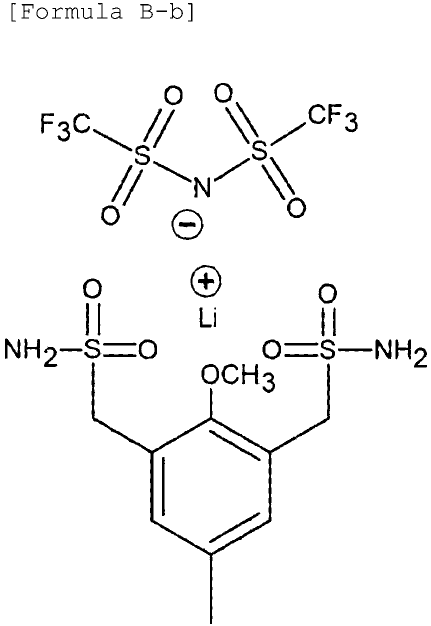

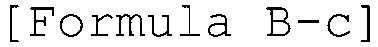

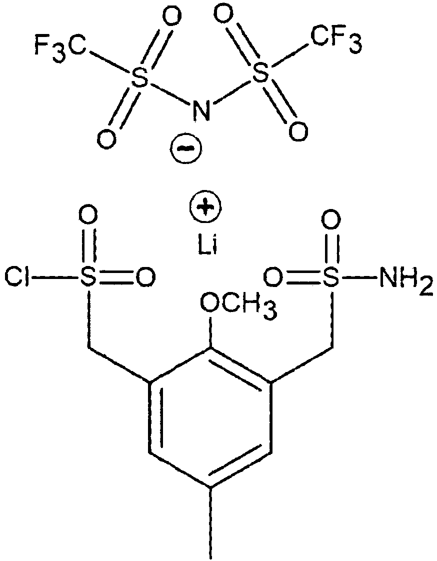

If the composite represented by Formula B above is one or more composites selected from the group consisting of Formulas B-a, B-b, and B-c above, the composite may have higher lithium ion conductivity than a polymer material of a solid electrolyte, which has been typically used.

### Polymer

The present invention provides a polymer including a repeating unit derived from the compound represented by Formula A above, a repeating unit derived from the composite represented by Formula B above, or a combination thereof.

According to an embodiment of the present invention, the compound and the composite may each be a monomer, and the monomer may be polymerized to form a polymer which is a solid electrolyte material having ion conductivity. Specifically, the compound represented by Formula A above and the composite represented by Formula B above may contain a halogen element or -NH₂ at the positions of X₂₁ and X₂₂, and for example, the halogen element may be one or more among F, Cl, I, and Br. In this case, the compound represented by Formula A above and the composite represented by Formula B above may undergo polymerization by a condensation reaction between the halogen element and the -NH₂ contained therein to form a polymer including sulfonylimide.

In addition, according to an embodiment of the present invention, the polymer may further include one or more of repeating units derived from fluorine-based and alkylene oxide monomers in addition to the repeating units derived from the compound and the composite.

According to an embodiment of the present invention, the repeating unit derived from the compound may be represented by Formula W-a or W-b below.

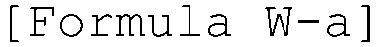

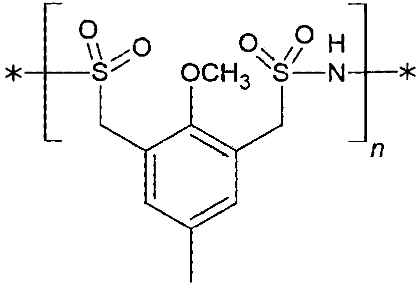

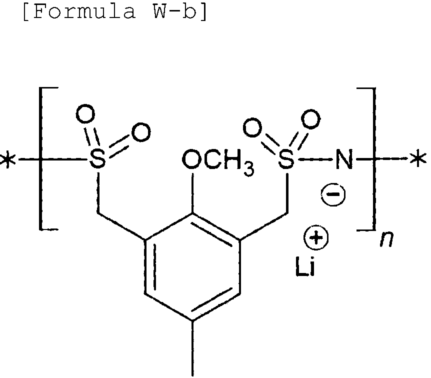

In Formulas W-a and W-b above, the n is an integer of 1 to 300, and the * is a connection site between the repeating units.

Formula W-b above is a repeating unit derived from a lithium salt of the compound represented by Formula A above, and a compound including the repeating unit may have an effect of further improving a lithium ion transference number.

In addition, according to an embodiment of the present invention, the repeating unit derived from the composite may be represented by Formula X-a or X-b below.

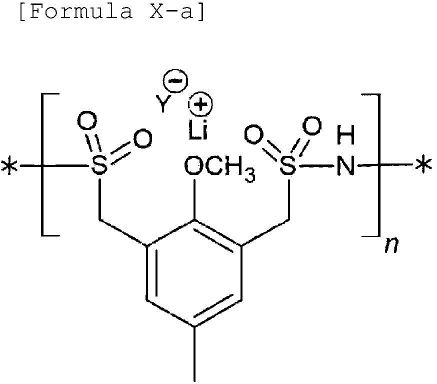

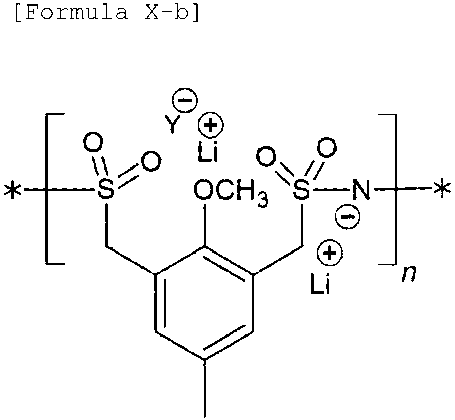

In Formulas X-a and X-b above, the Y⁻ is one or more selected from the group consisting of a halogen anion, BF₄⁻, ClO₄⁻, AlCl₄⁻, AlO₄⁻, PF₆⁻, CF₃SO₃⁻, CH₃CO₂⁻, CF₃CO₂⁻, AsF₆⁻, SbF₆⁻, CH₃SO₃⁻, bis (fluorosulfonyl) imide (FSI⁻) (N(SO₂F)₂⁻), bisperfluoroethanesulfonimide (BETI) (N(SO₂CF₂CF₃)₂⁻), and (bis) trifluoromethanesulfonimide (TFSI) (N(SO₂CF₃)₂⁻), the n is an integer of 1 to 300, and the * is a connection site between the repeating units.

Formula X-b above includes a lithiated compound, and thus, may have an effect of further improving the lithium ion transference number of a solid electrolyte.

In addition, according to an embodiment of the present invention, the combination of the repeating unit derived from the compound and the repeating unit derived from the composite may be represented by Formula Y or Z below.

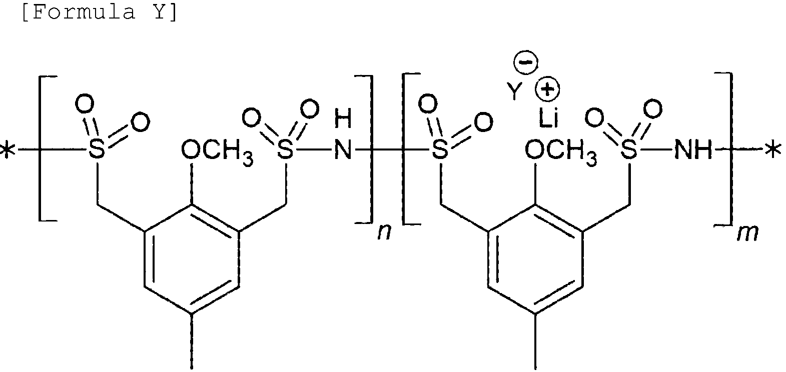

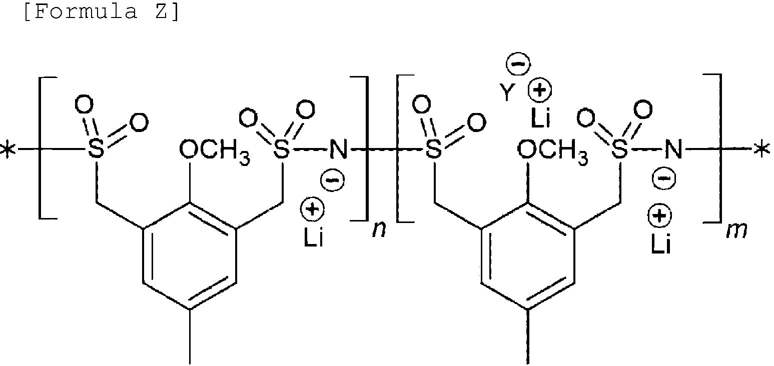

In Formulas Y and Z above, the Y⁻ is one or more selected from the group consisting of a halogen anion, BF₄⁻, ClO₄⁻, AlCl₄⁻, AlO₄⁻, PF₆⁻, CF₃SO₃⁻, CH₃CO₂⁻, CF₃CO₂⁻, AsF₆⁻, SbF₆⁻, CH₃SO₃⁻, bis (fluorosulfonyl) imide (FSI⁻) (N(SO₂F)₂⁻), bisperfluoroethanesulfonimide (BETI) (N(SO₂CF₂CF₃)₂⁻), and (bis) trifluoromethanesulfonimide (TFSI) (N(SO₂CF₃)₂⁻), the n and m are each independently an integer of 1 to 300, and the * is a connection site between the repeating units.

Formula Z above includes a lithiated compound, and thus, may have an effect of further improving the lithium ion transference number of a solid electrolyte.

### Solid electrolyte

The present invention provides a solid electrolyte including one or more selected from the group consisting of the compound and the composite. Specifically, the compound may be a compound including the repeating unit represented by Formula 1 above or the repeating unit represented by Formula 2 above, and the composite may be a composite including the repeating unit represented by Formula 3 or Formula 4. In addition, the present invention provides a solid electrolyte including the above-described polymer. Here, the polymer may include a repeating unit derived from the compound represented by Formula A above, a repeating unit derived from the composite, or a combination thereof. Since the solid electrolyte includes a polymer including a structure having affinity with lithium, the mobility of lithium ions is improved, so that there may be an effect of improving ion conductivity.

According to an embodiment of the present invention, the solid electrolyte may further include a crosslinkable compound, and the crosslinkable compound may be one or more selected from the group consisting of polyfunctional acrylate, vinyl group, isocyanate, and alcohol structures. One or more functional groups selected from the group consisting of Formulas d to g below at at least one end of both ends of the above repeating unit, and the crosslinkable compound may form a network between the repeating units through a radical polymerization or additive polymerization reaction. The contents regarding the above crosslinkable compound is the same as those described under the Table of Contents of Compounds.

Meanwhile, the present invention mixes a solvent and one or more selected from the group consisting of the compound and the composite to prepare a mixed solution, and uses the mixed solution to form an ion conductivity polymer electrolyte film through a film manufacturing process. The solvent may be one or more selected from the group consisting of methanol, ethanol, 2-propanol, butanol, 2-ethoxyethanol, isopropyl alcohol, ethyl acetate, butyl acetate, acetone, dichloroethane, acetonitrile, tetrahydrofuran, N-methyl-2-pyrrolidone, N,N-dimethylformamide, N,N-diethylformamide, N,N-dimethylacetamide, dimethylsulfoxide, hexamethylphosamide, 1,3-dimethyl-2-imidazolidinone, triethyl phosphate, and gamma-butyrolactone. The content of the above-described solvent may be determined in consideration of the viscosity and the like of the mixed solution.

In addition, the film manufacturing process may be a solution casting method (solution softening method), a melt extrusion method, a calender method, or a compression molding method, and more specifically, may be the solution cast method. The solution casting method may be performed by a process of performing coating on a separate substrate and then separating and combining the same with a positive electrode and a negative electrode. The substrate may be a glass substrate or a plastic substrate. The plastic substrate may include various plastic films such as polyethylene terephthalate, polyethylene naphthalate, polypropylene, polyethylene, 3-acetic acid cellulose, 2-acetic acid cellulose, poly(meth)acrylic acid alkyl ester, poly(meth)acrylic acid ester copolymer, polyvinyl chloride, polyvinyl alcohol, polycarbonate, polystyrene, cellophane, polyvinylidene chloride copolymer, polyamide, polyimide, vinyl chloride·vinyl acetate copolymer, polytetrafluoroethylene, and polytrifluoroethylene. The thickness of a support is preferable 5 µm to 150 µm, and more preferably, 10 µm to 50 µm. In addition, for the coating, methods such as spin coating, dip coating, solvent casting, slot die coating, spray coating, roll coating, extrusion coating, curtain coating, die coating, wire bar coating, knife coating or the like may be used.

### All-solid-state battery

The present invention provides an all-solid-state battery including the above-described solid electrolyte.

According to an embodiment of the present invention, the all-solid-state battery includes a positive electrode, a negative electrode (or anode-less), and a solid electrolyte layer including a solid electrolyte disposed between the positive electrode and the negative electrode. The all-solid-state battery according to the present invention has a small decrease in ionic conductivity, and the electronic conductivity of a solid electrolyte included therein is low, so that the initial efficiency, lifespan properties, and output properties of the all-solid-state battery may be excellent. At this time, the all-solid-state battery of the present invention may be manufactured by a method commonly known in the art. For example, the all-solid-state battery may be manufactured by stacking and pressing such that a solid electrolyte layer is present between a positive electrode and a negative electrode.

### (1) Positive electrode

The positive electrode may be manufactured by coating a positive electrode slurry, which includes a positive electrode active material, a binder, a conductive material, a solvent, etc., on a positive electrode current collector.

The positive electrode current collector is not particularly limited as long as it has conductivity without causing a chemical change in the battery, and for example, stainless steel, aluminum, nickel, titanium, fired carbon, or aluminum or stainless steel that is surface-treated with one of carbon, nickel, titanium, silver, and the like may be used. Also, microscopic irregularities may be formed on the surface of the positive electrode current collector to improve the binding force of a positive electrode active material, and the positive electrode current collector may be used in various forms, such as a film, a sheet, a foil, a net, a porous body, a foam body, and a non-woven fabric body.

The positive electrode active material is a compound capable of reversible intercalation and de-intercalation of lithium, and specifically, may include a lithium metal oxide including one or more metals, such as cobalt, manganese, nickel, and aluminum, and lithium. More specifically, the lithium metal oxide may be a lithium-manganese-based oxide (e.g., LiMnO₂, LiMn₂O₄, etc.), a lithium-cobalt-based oxide (e.g., LiCoO₂, etc.), a lithium-nickel-based oxide (e.g., LiNiO₂, etc.), a lithium-nickel-manganese-based oxide (e.g., LiNi_{1-Y}Mn_{Y}O₂ (wherein 0<Y<1), LiMn_{2-z}Ni_{z}O₄ (wherein 0<Z<2), etc.), a lithium-nickel-cobalt-based oxide (e.g., LiNi_{1-Y1}Co_{Y1}O₂ (wherein 0<Y1<1), etc.), a lithium-manganese-cobalt-based oxide (e.g., LiCo_{1-Y2}Mn_{Y2}O₂ (wherein 0<Y2<1), LiMn_{2-z1}Co_{z1}O₄ (wherein 0<Z1<2, etc.), a lithium-nickel-manganese-cobalt-based oxide (e.g., Li(NiₚCo_{q}Mnᵣ₁)O₂ (wherein 0<p<1, 0<q<1, 0<r1<1, and p+q+r1=1) or Li(Niₚ₁Co_{q1}Mnᵣ₂)O₄ (wherein 0<p1<2, 0<q1<2, 0<r2<2, and p1+q1+r2=2), etc.), or a lithium-nickel-cobalt-transition metal (M) oxide (e.g., Li(Niₚ₂Co_{q2}Mnᵣ₃M_{S2})O₂ (wherein M is selected from the group consisting of Al, Fe, V, Cr, Ti, Ta, Mg, and Mo, and p2, q2, r3, and s2 are each an atomic fraction of stand-alone elements, wherein 0<p2<1, 0<q2<1, 0<r3<1, 0<s2<1, and p2+q2+r3+s2=1), etc.) and the like, and any one thereof or a mixture of two or more thereof may be included.

Among these, due to the fact that the capacity properties and stability of a battery may be increased, the lithium metal oxide may be LiCoO₂, LiMnO₂, LiNiO₂, a lithium nickel-manganese-cobalt oxide (e.g., Li(Ni_{1/3}Mn_{1/3}Co_{1/3})O₂, Li(Ni_{0.6}Mn_{0.2}Co_{0.2})O₂, Li(Ni_{0.5}Mn_{0.3}Co_{0.2})O₂, Li(Ni_{0.7}Mn_{0.15}Co_{0.15})O₂, Li(Ni_{0.8}Mn_{0.1}Co_{0.1})O₂, etc.), or a lithium-nickel-cobalt-aluminum oxide (e.g., Li(Ni_{0.8}Co_{0.15}Al_{0.05})O₂, etc.), and when considering the effect of remarkable improvement according to the type and content ratio control of constituent elements forming a lithium metal oxide, the lithium metal oxide may be Li(Ni_{0.6}Mn_{0.2}Co_{0.2})O₂, Li(Ni_{0.5}Mn_{0.3}Co_{0.2})O₂, Li(Ni_{0.7}Mn_{0.15}Co_{0.15})O₂, Li(Ni_{0.8}Mn_{0.1}Co_{0.1})O₂, and the like, and any one thereof or a mixture of two or more thereof may be used.

The positive electrode active material may be included in an amount of 60 wt% or greater, 70 wt% or greater, 80 wt% or greater, or 99 wt% or less, or 98 wt% or less based on the total weight of solids excluding the solvent in the positive electrode slurry.

The binder is a component for assisting in coupling between a conductive material, an active material, and a current collector. Examples of the binder may include polyvinylidene fluoride, polyvinyl alcohol, carboxymethyl cellulose, starch, hydroxypropyl cellulose, regenerated cellulose, polyvinylpyrrolidone, polytetrafluoroethylene, polyethylene, polypropylene, an ethylene-propylene-diene monomer, a sulfonated ethylene-propylene-diene monomer, styrene-butadiene rubber, fluorine rubber, various copolymers thereof, and the like.

Commonly, the binder may be included in an amount of 1 wt% to 20 wt%, preferably 1 wt% to 15 wt%, and more preferably 1 wt% to 10 wt% based on the total weight of solids excluding the solvent in the positive electrode slurry.

The conductive material is a component for further improving the conductivity of a positive electrode active material.

The conductive material is not particularly limited as long as it has conductivity without causing a chemical change in the battery. For example, graphite; a carbon-based material such as carbon black, acetylene black, Ketjen black, channel black, furnace black, lamp black, and thermal black; conductive fiber such as carbon fiber and metal fiber; fluorocarbon; metal powder such as aluminum powder, and nickel powder; a conductive whisker such as zinc oxide and potassium titanate; a conductive metal oxide such as titanium oxide; and a conductive material such as a polyphenylene derivative, and the like may be used.

Commonly, the conductive material may be included in an amount of 1 wt% or greater, 20 wt% or less, 15 wt% or less, or 10 wt% or less based on the total weight of solids excluding the solvent in the positive electrode slurry.

The solvent may include an organic solvent such as N-methyl-2-pyrrolidone (NMP), and may be used in an amount such that a preferred viscosity is achieved when the positive electrode active material, and selectively a binder, a conductive material, and the like are included. For example, the solvent may be included in an amount such that the concentration of a solid including the positive electrode active material, and selectively the binder and the conductive material is 50 wt% or greater, 60 wt% or greater, 70 wt% or greater, 95 wt% or less, 90 wt% or less, or 85 wt% or less.

### (2) Negative electrode

The negative electrode may be manufactured by, for example, coating a negative electrode slurry, which includes a negative electrode active material, a binder, a conductive material, a solvent, etc., on a negative electrode current collector, or a graphite electrode made of carbon (C) or a lithium metal itself having a thickness of 50 µm or less may be used as the negative electrode, or the negative electrode may be excluded as anode-less.

For example, if the negative electrode is manufactured by coating a negative electrode slurry on the negative electrode current collector, the negative electrode current collector typically has a thickness of 3 µm to 500 µm. The negative electrode current collector is not particularly limited as long as it has high conductivity without causing a chemical change in the battery, and for example, copper, stainless steel, aluminum, nickel, titanium, fired carbon, copper or stainless steel that is surface-treated with one of carbon, nickel, titanium, silver, and the like, an aluminum-cadmium alloy, and the like may be used. Also, as in the case of the positive electrode current collector, microscopic irregularities may be formed on the surface of the negative electrode current collector to improve the binding force of a negative electrode active material, and the negative electrode current collector may be used in various forms of such as a film, a sheet, a foil, a net, a porous body, a foam body, and a non-woven fabric body.

Examples of the negative electrode active material may include one or two or more kinds of negative electrode active materials selected from the group consisting of natural graphite, artificial graphite, a carbonaceous material; a metal (Me) such as a lithium-containing titanium composite oxide (LTO), Si, SiOₓ, Sn, Li, Zn, Mg, Cd, Ce, Ni or Fe; an alloy composed of the metal (Me); an oxide (MeOₓ) of the metal (Me); and a composite of the metal (Me) and carbon. As the negative electrode active material, a silicon-based negative electrode active material including silicon (Si), a silicon oxide (SiOₓ), a silicon alloy, or the like, or a lithium metal may be used. In the case of the silicon-based negative electrode active material, a thin and stable SEI layer including a siloxane bond is formed, so that the high-temperature stability and lifespan properties of a battery may be further improved.

In addition, in the case of the lithium metal, it may be a typical lithium metal which includes a lithium metal or lithium alloy (e.g., an alloy of lithium with a metal such as aluminum, zinc, bismuth, cadmium, antimony, silicon, lead, tin, gallium or indium). The lithium metal negative electrode active material may be in the form of a foil, and by depositing the lithium metal negative electrode active material on one side of the negative electrode current collector, the lithium metal negative electrode active material may form a separate layer from the negative electrode current collector.

The negative electrode active material may be included in an amount of 60 wt% or greater, 70 wt% or greater, 80 wt% or greater, or 99 wt% or less, or 98 wt% or less based on the total weight of solids excluding the solvent in the negative electrode slurry.

The binder is a component for assisting in coupling between a conductive material, an active material, and a current collector. Examples of the binder may include polyvinylidene fluoride, polyvinyl alcohol, carboxymethyl cellulose, starch, hydroxypropyl cellulose, regenerated cellulose, polyvinylpyrrolidone, polytetrafluoroethylene, polyethylene, polypropylene, an ethylene-propylene-diene monomer, a sulfonated ethylene-propylene-diene monomer, styrene-butadiene rubber, fluorine rubber, various copolymers thereof, and the like.

Commonly, the binder may be included in an amount of 1 wt% or greater, 20 wt% or less, 15 wt% or less, or 10 wt% or less based on the total weight of solids excluding the solvent in the negative electrode slurry.

The conductive material is a component for further improving the conductivity of a negative electrode active material. The conductive material is not particularly limited as long as it has conductivity without causing a chemical change in the battery, and for example, graphite such as natural graphite or artificial graphite; carbon black such as acetylene black, Ketjen black, channel black, furnace black, lamp black, and thermal black; conductive fiber such as carbon fiber and metal fiber; fluorocarbon; metal powder such as aluminum powder, and nickel powder; a conductive whisker such as zinc oxide and potassium titanate; a conductive metal oxide such as titanium oxide; and a conductive material such as a polyphenylene derivative, or the like may be used.

The conductive material may be included in an amount of 1 wt% or greater, 20 wt% or less, 15 wt% or less, or 10 wt% or less based on the total weight of solids excluding the solvent in the negative electrode slurry.

The solvent may include water, or an organic solvent such as N-methyl-2-pyrrolidone (NMP), and may be used in an amount such that a preferred viscosity is achieved when the negative electrode active material, and selectively a binder, a conductive material, and the like are included. For example, the solvent may be included in an amount such that the concentration of a solid including the negative electrode active material, and selectively the binder and the conductive material is 50 wt% or greater, 60 wt% or greater, 70 wt% or greater, 95 wt% or less, 90 wt% or less, or 85 wt% or less.

When a metal itself is used as the negative electrode, the metal thin film itself may be used as the negative electrode, or the negative electrode may be manufactured by physically bonding, roll-pressing, or depositing a metal on the negative electrode current collector. The depositing method may be electrical vapor deposition or chemical vapor deposition.

For example, the metal thin film itself, or the metal bonded/roll-pressed/deposited on the negative electrode current collector may be one or more metals selected from the group consisting of lithium (Li), nickel (Ni), tin (Sn), copper (Cu), and indium (In), an alloy of two metals thereof, or the like.

### (3) Solid electrolyte layer

The solid electrolyte layer may further include a binder in addition to the solid electrolyte including the compound according to the present invention.

The binder is a component for assisting in binding between solid electrolyte particles. Examples of the binder may include polyvinylidene fluoride, polyvinyl alcohol, carboxymethyl cellulose, starch, hydroxypropyl cellulose, regenerated cellulose, polyvinylpyrrolidone, polytetrafluoroethylene, polyethylene, polypropylene, an ethylene-propylene-diene monomer, a sulfonated ethylene-propylene-diene monomer, styrene-butadiene rubber, fluorine rubber, various copolymers thereof, and the like.

The binder may be included in an amount of 0.1 wt% or greater, 5 wt% or less, 3 wt% or less, or 2 wt% or less based on the total weight of the solid electrolyte layer.

The present invention provides a battery module including the all-solid-state battery as a unit cell and a battery pack including the battery module. The battery module and the battery pack include the secondary battery which has high capacity, high rate properties, and cycle properties, and thus, may be used as a power source of a medium-and-large sized device selected from the group consisting of an electric car, a hybrid electric vehicle, a plug-in hybrid electric vehicle, and a power storage system.

Hereinafter, embodiments of the present invention will be described in detail so that those skilled in the art may easily carry out the present invention. However, the present invention may be implemented in various different forms, and the following examples are for illustrative purposes only to facilitate the understanding of the present invention, and do not limit the scope of the present invention. It will be apparent to those skilled in the art that various changes and modifications can be made without departing from the scope and technical idea of the present description, and it is obvious that such variations and modifications fall within the scope of the claims of the present specification.

### Synthesis Example 1.

### Preparation of compound represented by Formula a-1-1

A solution in which 1 equivalent of 4-methylanisole, 1.3 equivalents of p-formaldehyde, and 50 equivalents of HCl (6 N aqueous solution) were mixed was heated and stirred at a temperature of 80 °C for 5 hours. Thereafter, the solution was cooled to room temperature, and then a precipitate was separated, and dried under the condition of a reduced pressure to obtain a crude material. The crude material was purified by silica gel column chromatography to obtain 1,3-bis(chloromethyl)-2-methoxy-5-methylbenzene, which is a primary intermediate material.

An acetonitrile (ACN)/H₂O solution in which 2 equivalents of Na₂S₂O₄ and 4 equivalents of NaHCO₃ were dissolved was added to a 3-neck round bottom flask (RBF) and stirred. The primary intermediate material was added dropwise to the above stirred material at a temperature of 45 °C, and after confirming through thin layer chromatography (TLC) that the conversion was completed, the reaction was terminated. After the reaction was completed, the stirring was stopped, and a water layer was separated from a phase-separated mixture. From the water layer, water was removed using a rotary evaporator under the conditions of a high temperature and a reduced pressure to obtain a salt material. The salt material was completely dissolved in a small amount of water, and then dropped into MeOH to obtain a precipitate. The precipitate was dispersed in a dichloroethane (DCE) solution, and 3 equivalents of PCl₅ were added thereto. A crude solution obtained after stirring the mixture for about 12 hours under the condition of reflux was filtered to remove the residual salt. The obtained filtered DCE solution was washed using H₂O, and then dried using MgSO₄ to remove the solvent, resulting in obtaining (2-methoxy-5-methyl-1,3-phenylene)dimethanesulfonylchloride, which is a compound represented by Formula a-1-1 below.

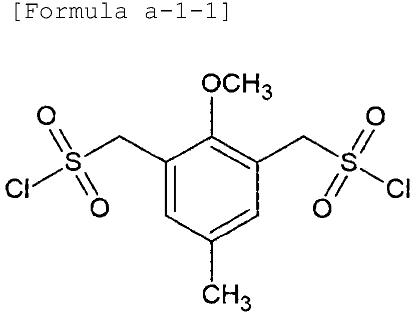

The synthesis of the compound represented by Formula a-1-1 was confirmed through ¹H-NMR spectrum (Bruker Corporation, AVANCE NEO). The ¹H-NMR data of the compound represented by Formula a-1-1 above is as follows.
¹H-NMR (400 MHZ. d6-Dimethyl sulfoxide): δ(ppm) = 6.99(s, 2H), 4.67(s, 4H), 3.78(s, 3H), 2.17(s, 3H)

### Synthesis Example 2

### Preparation of compound represented by Formula b-1

An ACN/H₂O solution in which 2 equivalents of Na₂S₂O₄ and 4 equivalents of NaHCO₃ were dissolved was added to a 3-neck round bottom flask (RBF) and stirred. 1,4-bromooctafluorobutane was added dropwise to the above stirred material at a temperature of 45 °C, and after confirming through ¹⁹F NMR that the conversion was completed, the reaction was terminated. After the reaction was completed, the stirring was stopped, and a water layer was separated from a phase-separated mixture. From the water layer, water was removed using a rotary evaporator under the conditions of a high temperature and a reduced pressure to obtain a salt material. The salt material was completely dissolved in a small amount of water, and then dropped into MeOH to obtain a precipitate. The precipitate was dispersed in a dichloroethane (DCE) solution, and 3 equivalents of PCl₅ were added thereto. A crude solution obtained after stirring the mixture for about 12 hours under the condition of reflux was filtered to remove the residual salt. The obtained filtered DCE solution was washed using H₂O, and then dried using MgSO₄ to remove the solvent, resulting in obtaining 1,1,2,2,3,3,4,4-octafluorobutane-1,4-disulfonyl dichloride, which is a compound represented by Formula b-1 below.

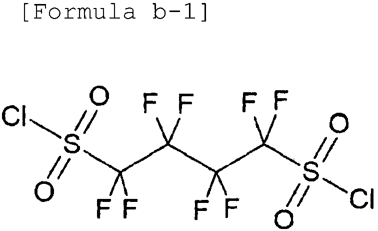

The synthesis of the compound represented by Formula b-1 was confirmed through ¹⁹F-NMR spectrum (Bruker Corporation, AVANCE NEO). The ¹⁹F-NMR data of the compound represented by Formula b-1 above is as follows.
¹⁹F-NMR (400 MHZ. d6-Dimethyl sulfoxide): δ(ppm) = - 104.2(t, 13Hz, 4F), -118.8(t, J = 12Hz, 4F)

### Synthesis Example 3

### Preparation of compound represented by Formula c-1-1

A tetrahydrofurane (THF) solution in which 1 equivalent of polyethylene glycol (number average molecular weight: 200) was dispersed was added to a 3-neck round bottom flask (RBF), and then 3 equivalent of NaH was dissolved therein and stirred. A THF solution in which 1,3-propane sultone was dissolved was added dropwise to the above stirred material at a temperature of 45 °C. The mixture was stirred under the condition of reflux heating, and after confirming through 1H NMR that the conversion was completed, the solution was cooled to room temperature. Thereafter, methanol (MeOH) was added thereto to quench the residual NaH. The solvent was removed therefrom using a rotary evaporator under the condition of a reduced pressure to obtain a crude material, the crude material was washed and purified using hexane and ether to obtain poly(ethylene glycol) bis(sodium propane-1-sulfonate, which is a primary intermediate material.

The primary intermediate material was dispersed in a dichloromethane (DCM) solvent, and then 3 equivalents of PCl₅ was added thereto. The mixture was stirred at a temperature of 40 °C for about 12 hours to obtain a crude material, and after removing the solvent therefrom using a rotary evaporator under the condition of a reduced pressure, the crude material was washed and purified using hexane and ether to obtain poly(ethylene glycol) bis(sodium propane-1-sulfonyl chloride), which is a secondary intermediate material.

Under a nitrogen atmosphere, 7 equivalents of a NH₄OH aqueous solution was added to a 3-neck RBF and then stirred at room temperature. The secondary intermediate material was dispersed in acetonitrile, and the above dispersed solution was slowly added to the above stirred material over the course of 7 hours, and then the solution was stirred at a temperature of 40 °C for about 12 hours. Thereafter, water was added thereto, and then extraction was performed several times using ethyl acetate. An organic layer was dried using Na₂SO₄, and then after removing the solvent therefrom using a rotary evaporator under the condition of a reduced pressure, the organic layer was washed and purified using ether, and then dried to obtain poly(ethylene glycol) bis(propane-1-sulfonamide), which is a compound represented by Formula c-1-1.

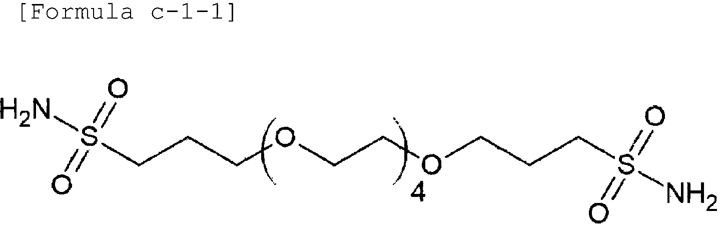

The synthesis of the compound represented by Formula c-1-1 was confirmed through ¹H-NMR spectrum (Bruker Corporation, AVANCE NEO). The ¹H-NMR data of the compound represented by Formula c-1-1 above is as follows.
¹H-NMR (500 MHZ. d6-Dimethyl sulfoxide): δ(ppm) = 6.87(broad S, 4H), 3.43(m, 23H), 3.03(m, 4H), 1.89(m, 4H)

### Synthesis Example 4

### Preparation of compound represented by Formula d-1

1 equivalent of 3-sulfopropyl methacrylate potassium salt (TCI), 0.1 equivalents of DMF and, and tetrahydrofuran (THF) were added to a 1-neck round bottom flask (RBF), and 5.5 equivalents of thionyl chloride was slowly added dropwise thereto and stirred. The reaction was completed after 3 hours, and then quenching was performed using water, and extraction was performed 3 times using dichloromethane (DCM) to obtain an organic layer. The solvent was removed therefrom using a rotary evaporator under the condition of a reduced pressure to obtain 3-(chlorosulfonyl)propyl methacrylate, which is a compound represented by Formula d-1.

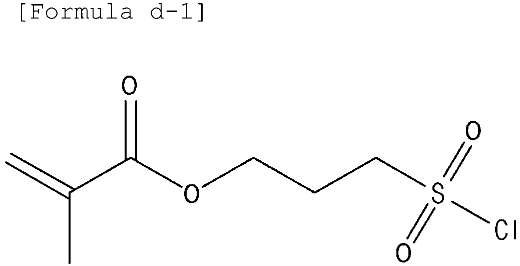

The synthesis of a compound represented by Formula d-1 was confirmed through ¹H-NMR spectrum (Bruker Corporation, AVANCE NEO). The ¹H-NMR data of the compound represented by Formula d-1 above is as follows.
¹H-NMR (500 MHZ. d6-Dimethyl sulfoxide): δ (ppm) = 6.13-5.63(s, 2H), 4.34(m, 2H), 3.79(m, 2H), 2.45(m, 2H), 1.96 (s, 3H)

### Synthesis Example 5

### Preparation of compound represented by Formula A-a

A solution in which 1 equivalent of 4-methylanisole, 1.3 equivalents of p-formaldehyde, and 50 equivalents of HCl (6 N aqueous solution) were mixed was heated and stirred at a temperature of 80 °C for 5 hours. Thereafter, the solution was cooled to room temperature, and then a precipitate was separated, and dried under the condition of a reduced pressure to obtain a crude material. The crude material was purified by silica gel column chromatography to obtain 1,3-bis(chloromethyl)-2-methoxy-5-methylbenzene, which is a primary intermediate material.

An acetonitrile (ACN)/H₂O solution in which 2 equivalents of Na₂S₂O₄ and 4 equivalents of NaHCO₃ were dissolved was added to a 3-neck round bottom flask (RBF) and stirred. The primary intermediate material was added dropwise to the above stirred material at a temperature of 45 °C, and after confirming through thin layer chromatography (TLC) that the conversion was completed, the reaction was terminated. After the reaction was completed, the stirring was stopped, and a water layer was separated from a phase-separated mixture. From the water layer, water was removed using a rotary evaporator under the conditions of a high temperature and a reduced pressure to obtain a salt material. The salt material was completely dissolved in a small amount of water, and then dropped into MeOH to obtain a precipitate. The precipitate was dispersed in a dichloroethane (DCE) solution, and 3 equivalents of PCl₅ were added thereto. A crude solution obtained after stirring the mixture for about 12 hours under the condition of reflux was filtered to remove the residual salt. The obtained filtered DCE solution was washed using H₂O, and then dried using MgSO₄ to remove the solvent, resulting in obtaining (2-methoxy-5-methyl-1,3-phenylene)dimethanesulfonylchloride, which is a compound represented by Formula A-a below (the same as the compound represented by Formula a-1-1 prepared in Synthesis Example 1 above) .

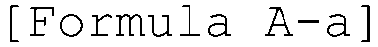

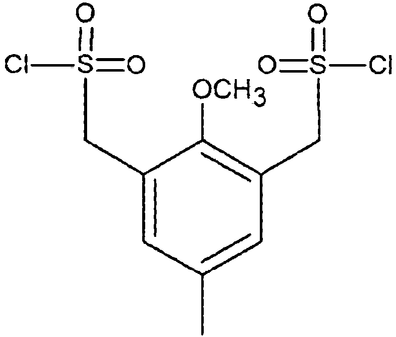

The synthesis of the compound represented by Formula A-a was confirmed through ¹H-NMR spectrum (Bruker Corporation, AVANCE NEO). The ¹H-NMR data of the compound represented by A-a above is as follows.
¹H-NMR (500 MHZ. d6-Dimethyl sulfoxide): δ(ppm) = 6.99(s, 2H), 4.67(s, 4H), 3.78(s, 3H), 2.17(s, 3H)

### Synthesis Example 6

### Preparation of compound represented by Formula A-b

Under a nitrogen atmosphere, 5 equivalents of NH₃ (7 M in MeOH) was added to a 3-neck RBF and then stirred at room temperature. 1 equivalent of the compound represented by Formula A-a prepared in Synthesis Example 1 above was dispersed in acetonitrile, and the dispersed solution was slowly added to the above stirred material over the course of 7 hours, and then the solution was stirred at a temperature of 40 °C for about 12 hours. Thereafter, water was added thereto, and then extraction was performed several times using ethyl acetate. An organic layer was dried using Na₂SO₄, and then after removing the solvent therefrom using a rotary evaporator under the condition of a reduced pressure, the organic layer was washed and purified using ether, and then dried to obtain (2-methoxy-5-methyl-1,3-phenylen)dimethanesulfonamide, which is a compound represented by Formula A-b below.

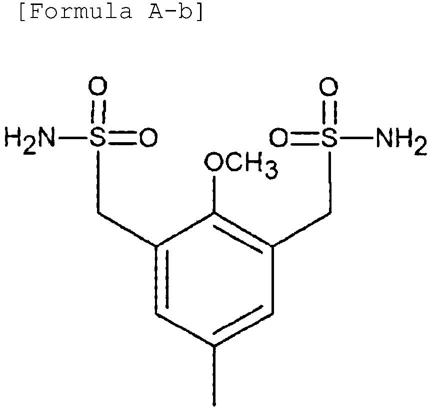

The synthesis of the compound represented by Formula A-b was confirmed through ¹H-NMR spectrum (Bruker Corporation, AVANCE NEO). The ¹H-NMR data of the compound represented by A-b above is as follows.
¹H-NMR (500 MHZ. d6-Dimethyl sulfoxide): δ(ppm) = 7.12(broad s, 4H), 6.98(s, 2H), 4.35(s, 4H), 3.78(s, 3H), 2.17(s, 3H)

### Synthesis Example 7

### Preparation of compound represented by Formula A-c

Under a nitrogen atmosphere, 1 equivalents of NH₃ (7 M in MeOH) was added to a 3-neck RBF and then stirred at room temperature. 1 equivalent of the compound represented by Formula A-b prepared in Synthesis Example 2 above was dispersed in acetonitrile, and the dispersed solution was slowly added to the above stirred material over the course of 1 hour, and then the solution was stirred at room temperature for about 24 hours. Thereafter, water was added thereto, and then extraction was performed several times using ethyl acetate. An organic layer was dried using Na₂SO₄, and then after removing the solvent therefrom using a rotary evaporator under the condition of a reduced pressure, the organic layer was washed and purified using ether, and then dried to obtain (2-methoxy-5-methyl-3-sulfamoylmethyl)phenyl)methanesulfonylchloride, which is a compound represented by Formula A-c below.

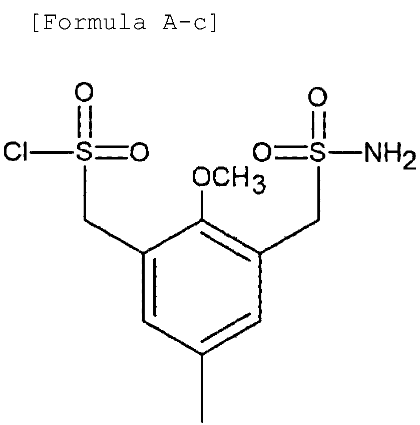

The synthesis of the compound represented by Formula A-c was confirmed through ¹H-NMR spectrum (Bruker Corporation, AVANCE NEO). The ¹H-NMR data of the compound represented by A-c above is as follows.
¹H-NMR (500 MHZ. d6-Dimethyl sulfoxide): δ(ppm) = 7.12(broad s, 2H), 6.97 (s, 2H), 4.67(s, 2H), 4.35(s, 2H), 3.78(s, 3H), 2.16(s, 3H)

### Synthesis Example 8

### Preparation of compound represented by Formula B-a

Under a nitrogen atmosphere, an acetonitrile solution in which 1 equivalent of the compound represented by Formula A-a prepared in Synthesis Example 1 above was dispersed was stirred. An aqueous solution in which 5 equivalents of lithium bis(trifluoromethyl)sulfonylimide was dissolved was slowly added to the corresponding mixture over the course of 1 hour, and then the solution was stirred at room temperature for about 24 hours. Thereafter, water was added thereto, and then extraction was performed several times using ethyl acetate. An organic layer was dried using Na₂SO₄, and then after removing the solvent therefrom using a rotary evaporator under the condition of a reduced pressure, the organic layer was washed and purified using ether, and then dried to obtain (2-methoxy-5-methyl-1,3-phenylene)dimethanesulfonyl chloride, lithium bis(trifluoromethyl)sulfonylimide, which is a compound represented by Formula B-a below.

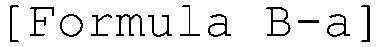

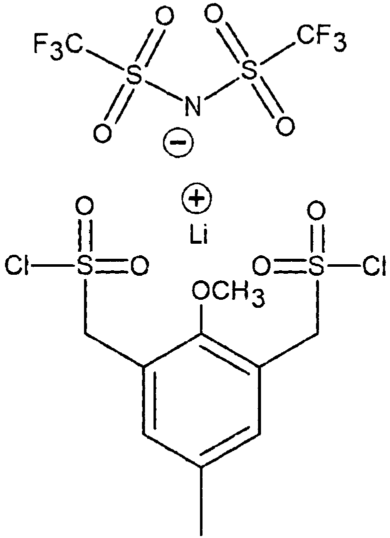

The synthesis of the compound represented by Formula B-a was confirmed through ¹H-NMR spectrum (Bruker Corporation, AVANCE NEO). The ¹H-NMR data of the compound represented by B-a above is as follows.
¹H-NMR (500 MHZ. d6-Dimethyl sulfoxide): δ(ppm) = 7.08(s, 2H), 4.67(s, 4H), 3.90(s, 3H), 2.17(s, 3H)

### Preparation Example 1

### Preparation of compound represented by Formula 1-a

3 equivalents of triethylamine, 1 equivalent of the compound represented by Formula c-1-1 above, 0.2 equivalents of the compound represented by Formula a-1-1 above, and 0.8 equivalents of the compound represented by Formula b-1 above were dispersed in a dimethylformamide (DMF) solution, and then stirred at a temperature of 120 °C for 24 hours. Thereafter, the above solution was cooled to room temperature, and water and dichloromethane were added thereto to obtain a mixture, from which a precipitate was filtered and separated. Thereafter, the obtained solid material was washed using methanol and then vacuum-dried. The vacuum-dried solid material and 3 equivalents of an ammonia solution (7 M in MeOH, Sigma Aldrich Corporation) were dispersed in a dimethylformamide (DMF) solution, and then stirred at a temperature of 50 °C for 24 hours. Thereafter, the above stirred solution was cooled to room temperature, and dichloromethane was added thereto to obtain a mixture, from which a precipitate was filtered and separated to obtain a compound represented by Formula 1-a below.

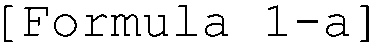

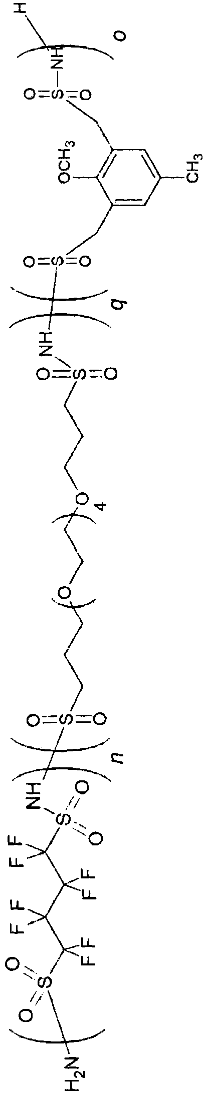

In Formula 1-a above, the n is an integer of 1 to 20, the q is an integer of 1 to 20, and the o is an integer of 1 to 20.

The synthesis of the compound represented by Formula 1-a above was confirmed through ¹H-NMR spectrum (Bruker Corporation, AVANCE NEO) and GPC (Waters Corporation, e2695 GPC). It was analyzed that the number-average molecular weight (Mn) was 11200 g/mol, and PDI=1.8. The ¹H-NMR data of the compound represented by 1-a above is as follows.
¹H-NMR (500 MHZ. d6-Dimethyl sulfoxide): δ(ppm) = 6.99(s, 0.4H), 4.29(s, 0.8H), 3.78(s, 0.6H), 3.43(m, 23H), 3.02(m, 4H), 2.17(s, 0.6H), 1.89(m, 4H)
¹⁹F-NMR (470 MHZ. d6-Dimethyl sulfoxide): δ(ppm) = - 113.2(t, 4F), -119.1(t, 4F)

### Preparation Example 2

### Preparation of compound represented by Formula 1-b

3 equivalents of triethylamine, 1 equivalent of the compound represented by Formula c-1-1 above, and 1 equivalent of the compound represented by Formula a-1-1 above were dispersed in a dimethylformamide (DMF) solution, and then stirred at a temperature of 120 °C for 24 hours. Thereafter, the above solution was cooled to room temperature, and water and dichloromethane were added thereto to obtain a mixture, from which a precipitate was filtered and separated. Thereafter, the obtained solid material was washed using methanol and then vacuum-dried. The vacuum-dried solid material and 3 equivalents of an ammonia solution (7 M in MeOH, Sigma Aldrich Corporation) were dispersed in a dimethylformamide (DMF) solution, and then stirred at a temperature of 50 °C for 24 hours. Thereafter, the above stirred solution was cooled to room temperature, and dichloromethane was added thereto to obtain a mixture, from which a precipitate was filtered and separated to obtain a compound represented by Formula 1-b below.

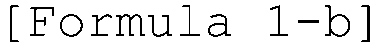

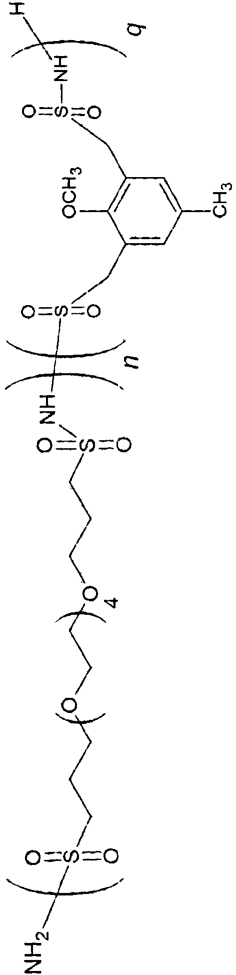

In Formula 1-b above, the n is an integer of 1 to 20, and the q is an integer of 1 to 20.

The synthesis of the compound represented by Formula 1-b above was confirmed through ¹H-NMR spectrum (Bruker Corporation, AVANCE NEO) and GPC (Waters Corporation, e2695 GPC). It was analyzed that the number-average molecular weight (Mn) was 7100 g/mol, and PDI=1.6. The ¹H-NMR data of the compound represented by 1-b above is as follows.
¹H-NMR (500 MHZ. d6-Dimethyl sulfoxide): δ(ppm) = 6.99(s, 2H), 4.29(s, 4H), 3.78(s, 3H), 3.43(m, 23H), 2.43(m, 4H), 2.17(s, 3H), 1.89(m, 4H)

### Preparation Example 3

### Preparation of compound represented by Formula 2-a

3 equivalents of triethylamine, 1 equivalent of the compound represented by Formula c-1-1 above, 0.2 equivalents of the compound represented by Formula a-1-1 above, and 0.8 equivalents of the compound represented by Formula b-1 above were dispersed in a dimethylformamide (DMF) solution, and then stirred at a temperature of 120 °C for 24 hours. Thereafter, the above solution was cooled to room temperature, and water and dichloromethane were added thereto to obtain a mixture, from which a precipitate was filtered and separated. Thereafter, the obtained solid material was washed using methanol and then vacuum-dried. The vacuum-dried solid material was dispersed in Anhydrous DMF, and then 5 equivalents of lithium hydride was slowly added thereto and stirred at room temperature for 24 hours. Thereafter, unreacted lithium hydride was filtered to obtain a filtrate, from which a precipitate was filtered and separated using an excess amount of tetrahydrofuran (THF).

The precipitate obtained by filtration and separation and 3 equivalents of an ammonia solution (7 M in MeOH, Sigma Aldrich Corporation) were dispersed in a dimethylformamide (DMF) solution, and then stirred at a temperature of 50 °C for 24 hours. Thereafter, the above stirred solution was cooled to room temperature, and dichloromethane was added thereto to obtain a mixture, from which a precipitate was filtered and separated to obtain a compound represented by Formula 2-a below.

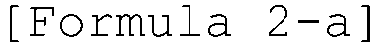

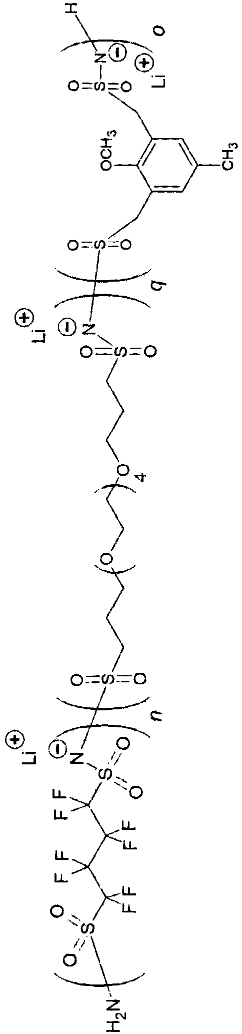

In Formula 2-a above, the n is an integer of 1 to 20, the q is an integer of 1 to 20, and the o is an integer of 1 to 20.

The synthesis of the compound represented by Formula 2-a above was confirmed through ¹H-NMR spectrum (Bruker Corporation, AVANCE NEO) and GPC (Waters Corporation, e2695 GPC). It was analyzed that the number-average molecular weight (Mn) was 12400 g/mol, and PDI=1.9. The ¹H-NMR data of the compound represented by 2-a above is as follows.
¹H-NMR (500 MHZ. d6-Dimethyl sulfoxide): δ(ppm) = 6.99(s, 0.4H), 4.64(s, 0.4H), 3.78(s, 0.6H), 3.43(m, 23H), 3.32(m, 4H), 2.17(s, 0.6H), 1.89(m, 4H)
¹⁹F-NMR (470 MHZ. d6-Dimethyl sulfoxide): δ(ppm) = - 114.1(t, 4F), -119.1(t, 4F)

### Preparation Example 4

### Preparation of compound represented by Formula 2-b

3 equivalents of triethylamine, 1 equivalent of the compound represented by Formula c-1-1 above, and 1 equivalent of the compound represented by Formula a-1-1 above were dispersed in a dimethylformamide (DMF) solution, and then stirred at a temperature of 120 °C for 24 hours. Thereafter, the above solution was cooled to room temperature, and water and dichloromethane were added thereto to obtain a mixture, from which a precipitate was filtered and separated. Thereafter, the obtained solid material was washed using methanol and then vacuum-dried. The vacuum-dried solid material was dispersed in Anhydrous DMF, and then 5 equivalents of lithium hydride was slowly added thereto and stirred at room temperature for 24 hours. Thereafter, unreacted lithium hydride was filtered to obtain a filtrate, from which a precipitate was filtered and separated using an excess amount of tetrahydrofuran (THF).

The precipitate obtained by filtration and separation and 3 equivalents of an ammonia solution (7 M in MeOH, Sigma Aldrich Corporation) were dispersed in a dimethylformamide (DMF) solution, and then stirred at a temperature of 50 °C for 24 hours. Thereafter, the above stirred solution was cooled to room temperature, and dichloromethane was added thereto to obtain a mixture, from which a precipitate was filtered and separated to obtain a compound represented by Formula 2-b below.

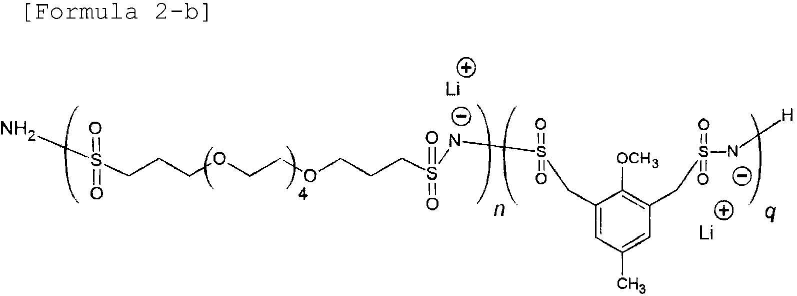

In Formula 2-b above, the n is an integer of 1 to 20, and the q is an integer of 1 to 20.

The synthesis of the compound represented by Formula 2-b above was confirmed through ¹H-NMR spectrum (Bruker Corporation, AVANCE NEO) and GPC (Waters Corporation, e2695 GPC). It was analyzed that the number-average molecular weight (Mn) was 7300 g/mol, and PDI=1.6. The ¹H-NMR data of the compound represented by 2-b above is as follows.
¹H-NMR (500 MHZ. d6-Dimethyl sulfoxide): δ(ppm) = 6.99(s, 2H), 4.29(s, 4H), 3.78(s, 3H), 3.43(m, 23H), 3.33(m, 4H), 2.17(s, 3H), 1.89(m, 4H)

### Preparation Example 5

### Preparation of compound represented by Formula 1-c

After the compound represented by Formula 1-a was prepared in Preparation Example 1 above, 3 equivalents of TEA, 1 equivalent of the compound represented by Formula 1-a, and 2.5 equivalents of the compound represented by Formula d-1 were dispersed in a DMF solution and stirred at room temperature for 24 hours. Thereafter, dichloromethane was added thereto to obtain a mixture, from which a precipitate was filtered and separated to obtain a compound represented by Formula 1-c below.

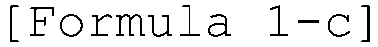

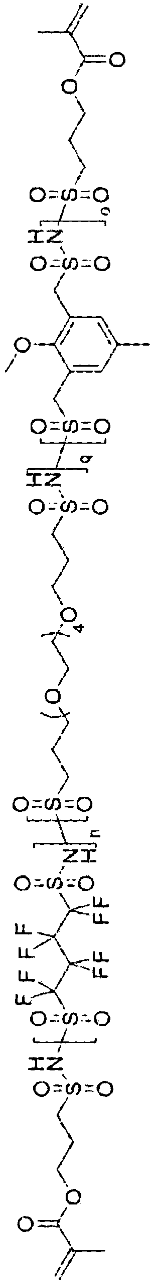

In Formula 1-c above, the n is an integer of 1 to 20, the q is an integer of 1 to 20, and the o is an integer of 1 to 20.

The synthesis of the compound represented by Formula 1-c above was confirmed through ¹H-NMR spectrum (Bruker Corporation, AVANCE NEO) and GPC (Waters Corporation, e2695 GPC). It was analyzed that the number-average molecular weight (Mn) was 15000 g/mol, and PDI=1.9. The ¹H-NMR data of the compound represented by 1-c above is as follows.
¹H-NMR (500 MHZ. d6-Dimethyl sulfoxide): δ(ppm) = 6.99(s, 0.4H), 6.0(s, 2H), 5.6(s, 2H), 4.29(s, 0.8H), 3.78(s, 0.6H), 3.43(m, 31H), 3.02(m, 8H), 2.1(s, 6.6H), 1.89(m, 4H)
¹⁹F-NMR (470 MHZ. d6-Dimethyl sulfoxide): δ(ppm) = - 113.2(t, 4F), -119.1(t, 4F)

### Preparation Example 6

### Preparation of compound represented by Formula 2-c

After the compound represented by Formula 2-a was prepared in Preparation Example 3 above, 3 equivalents of TEA, 1 equivalent of the compound represented by Formula 2-a, and 2.5 equivalents of the compound represented by Formula d-1 were dispersed in a DMF solution and stirred at room temperature for 24 hours. Thereafter, dichloromethane was added thereto to obtain a mixture, from which a precipitate was filtered and separated to obtain a compound represented by Formula 2-c below.

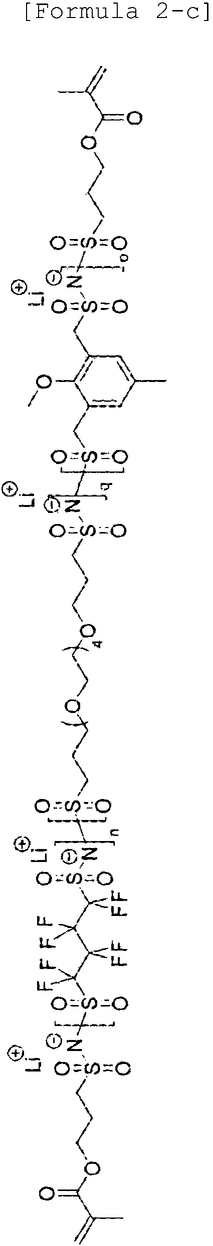

The synthesis of the compound represented by Formula 2-c above was confirmed through ¹H-NMR spectrum (Bruker Corporation, AVANCE NEO) and GPC (Waters Corporation, e2695 GPC). It was analyzed that the number-average molecular weight (Mn) was 15200 g/mol, and PDI=1.9. The ¹H-NMR data of the compound represented by 2-c above is as follows.
¹H-NMR (500 MHZ. d6-Dimethyl sulfoxide): δ(ppm) = 6.99(s, 0.4H), 6.0(s, 2H), 5.6(s, 2H), 4.64(s, 0.4H), 3.78(s, 0.6H), 3.43(m, 27H), 3.32 (m, 8H), 2.4{(m, 4H), 2.17 (s, 6.6H), 1.89 (m, 4H)
¹⁹F-NMR (470 MHZ. d6-Dimethyl sulfoxide): δ(ppm) = - 114.1(t, 4F), -119.1(t, 4F)

### Preparation Example 7

### Preparation of compound represented by Formula W-1

3 equivalents of triethylamine, 1 equivalent of the compound represented by Formula A-a above, and 1 equivalent of the compound represented by Formula A-b above were dispersed in a dimethylformamide (DMF) solution, and then stirred at a temperature of 120 °C for 24 hours. Thereafter, the above solution was cooled to room temperature, and water and dichloromethane were added thereto to obtain a mixture, from which a precipitate was filtered and separated. Thereafter, the obtained solid material was washed using methanol and then vacuum-dried. The vacuum-dried solid material and 3 equivalents of an ammonia solution (7 M in MeOH, Sigma Aldrich Corporation) were dispersed in a dimethylformamide (DMF) solution, and then stirred at a temperature of 50 °C for 24 hours. Thereafter, the above stirred solution was cooled to room temperature, and dichloromethane was added thereto to obtain a mixture, from which a precipitate was filtered and separated to obtain a compound represented by Formula W-1 below.

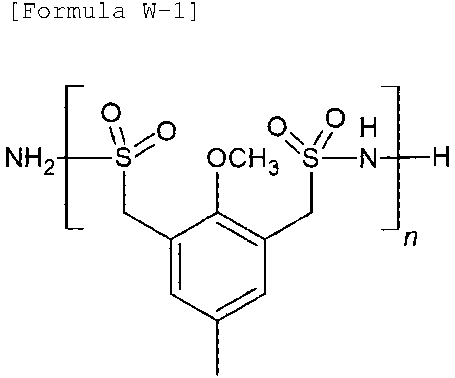

In Formula W-1 above, the n is an integer of 1 to 300.

The synthesis of the compound represented by Formula W-1 above was confirmed through GPC (Waters Corporation, e2695 GPC). It was analyzed that the number-average molecular weight (Mn) was 9600 g/mol, and PDI=1.9.

### Preparation Example 8

### Preparation of compound represented by Formula X-1

Under a nitrogen atmosphere, an acetonitrile solution in which 1 equivalent of the compound represented by Formula W-1 prepared in Preparation Example 1 above was dispersed was stirred. An aqueous solution in which 5 equivalents of lithium bis(trifluoromethyl)sulfonylimide was dissolved was slowly added to the corresponding mixture over the course of 1 hour, and then the solution was stirred at room temperature for about 24 hours. Thereafter, water was added thereto, and then extraction was performed several times using ethyl acetate. An organic layer was dried using Na₂SO₄, and then after removing the solvent therefrom using a rotary evaporator under the condition of a reduced pressure, the organic layer was washed and purified using ether, and then dried to obtain a compound represented by Formula X-1 below.

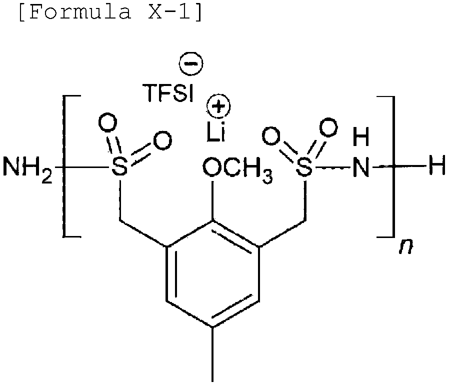

In Formula X-1 above, the n is an integer of 1 to 300.

The synthesis of the compound represented by Formula X-1 above was confirmed through GPC (Waters Corporation, e2695 GPC). It was analyzed that the number-average molecular weight (Mn) was 9900 g/mol, and PDI=1.9.

### Preparation Example 9

### Preparation of compound represented by Formula Z-1

Under a nitrogen atmosphere, the above material represented by Formula X-1 prepared in Preparation Example 1 above was dispersed in Anhydrous DMF, and then 5 equivalents of lithium hydride was slowly added thereto, and then the solution was stirred at room temperature for about 24 hours. Thereafter, unreacted lithium hydride was filtered to obtain a filtrate, from which a precipitate was filtered and separated using an excess amount of tetrahydrofuran (THF) to obtain a compound represented by Formula Z-1 below.

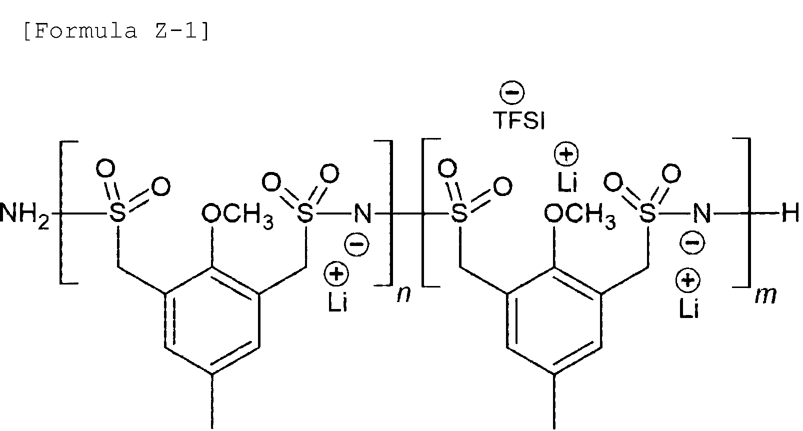

In Formula Z-1 above, the n and m are each independently an integer of 1 to 300.

The synthesis of the compound represented by Formula Z-1 above was confirmed through GPC (Waters Corporation, e2695 GPC). It was analyzed that the number-average molecular weight (Mn) was 10200 g/mol, and PDI=1.9.

### Preparation Example 10

### Preparation of compound represented by Formula Q

3 equivalents of triethylamine, 1 equivalent of (2,5-dimethyl-1,3-phenylene)dimethanesulfonamide, and 1 equivalent of (2,5-dimethyl-1,3-phenylene)dimethanesulfonyl chloride were dispersed in a dimethylformamide (DMF) solution, and then stirred at a temperature of 120 °C for 24 hours. Thereafter, the above solution was cooled to room temperature, and water and dichloromethane were added thereto to obtain a mixture, from which a precipitate was filtered and separated. Thereafter, the obtained solid material was washed using methanol and then vacuum-dried to obtain a compound represented by Formula Q below.

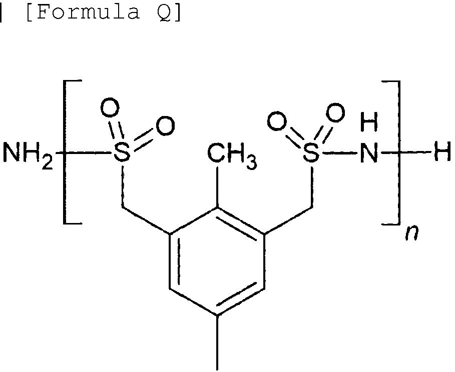

In Formula Q above, the n is an integer of 1 to 300.

The synthesis of the compound represented by Formula Q above was confirmed through GPC (Waters Corporation, e2695 GPC). It was analyzed that the number-average molecular weight (Mn) was 6100 g/mol, and PDI=1.7.

### Example 1

3 g of the compound represented by Formula 1-a prepared from Preparation Example 1 was dispersed in 30 mL of a N-methyl-2-pyrrolidone solvent to obtain a transparent solution, and thereafter, the transparent solution was subjected to solution casting uniformly on a flat glass plate. Thereafter, the above solution was dried at a temperature of 40 °C for about 12 hours, and then dried in a vacuum oven at a temperature of 70 °C for 24 hours. Through the above-described process, an ion conductive polymer electrolyte film having a thickness of 20 µm to 40 µm was prepared.

### Example 2

An ion conductive polymer electrolyte film was prepared in the same manner as in Example 1 above, except that 3 g of the compound represented by Formula 1-b prepared from Preparation Example 2 was used.

### Example 3

An ion conductive polymer electrolyte film was prepared in the same manner as in Example 1 above, except that 3 g of the compound represented by Formula 2-a prepared from the Preparation Example 3 was used.

### Example 4

An ion conductive polymer electrolyte film was prepared in the same manner as in Example 1 above, except that 3 g of the compound represented by Formula 2-b prepared from Preparation Example 4 was used.

### Example 5

Lithium bis(trifluoromethyl)sulfonylimide (EO:Li⁺ molar ratio = 20:1) was dissolved in N-methyl-2-pyrrolidone, and then 3 g (20 wt% vs N-methyl-2-pyrrolidone) of the compound represented by Formula 1-a prepared from Preparation Example 1 was added thereto. The mixture was stirred using an overhead-stirrer under the condition of room temperature for 2 hours. Thereafter, the stirred mixture was subjected to ultrasonication treatment (exposed to sonication conditions) for 20 minutes in a bath-sonicator to remove air bubbles, and a solution in which a compound represented by Formula 3-a below was dissolved was obtained.

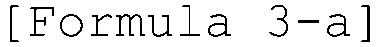

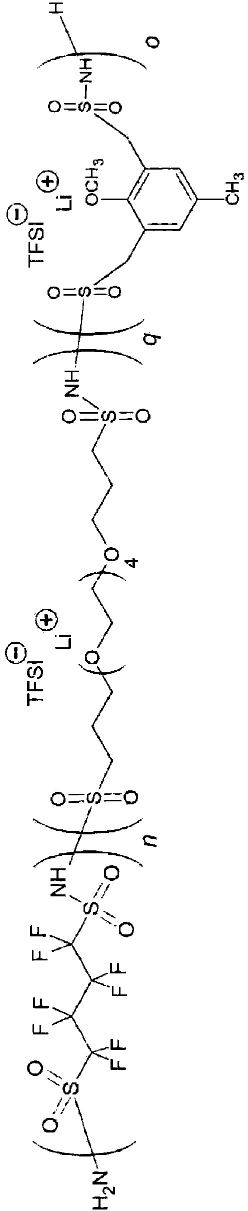

In Formula 3-a above, the n is an integer of 1 to 20, the q is an integer of 1 to 20, and the o is an integer of 1 to 20.

Thereafter, the solution was subjected to solution casting uniformly on a flat glass plate. Thereafter, the above solution was dried at a temperature of 40 °C for about 12 hours, and then, dried again in a vacuum oven at a temperature of 70 °C for 24 hours to prepare an ion conductive polymer electrolyte film having a thickness of 20 µm to 40 µm.

### Example 6

An ion conductive polymer electrolyte film containing a compound represented by Formula 4-a was prepared in the same manner as in Example 5 above, except that 3 g of the compound represented by Formula 2-a prepared from Preparation Example 3 was used instead of the compound represented by Formula 1-a.

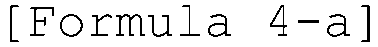

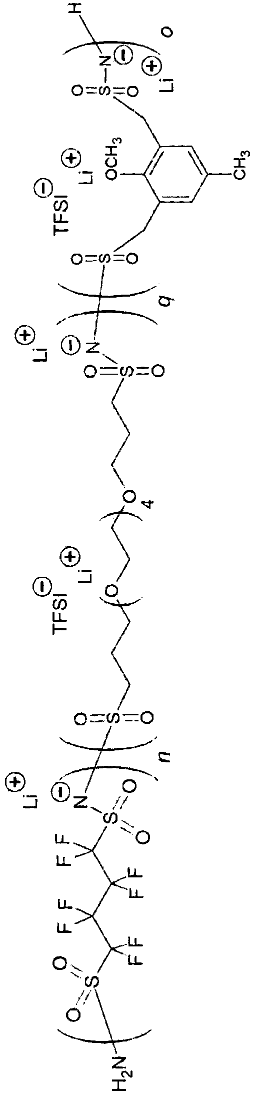

In Formula 4-a above, the n is an integer of 1 to 20, the q is an integer of 1 to 20, and the o is an integer of 1 to 20.

### Example 7

3 g of the compound represented by Formula 1-c prepared from Preparation Example 5 was dispersed in 30 mL of a N-methyl-2-pyrrolidone solvent to obtain a transparent solution, and 0.1 g of AINB was introduced thereto. Thereafter, the above solution was subjected to solution casting uniformly on a flat glass plate. Thereafter, the above solution was dried at a temperature of 40 °C for about 12 hours, and then dried in a vacuum oven at a temperature of 70 °C for 24 hours. Through the above-described process, an ion conductive polymer electrolyte film having a thickness of 20 µm to 40 µm was prepared.

### Example 8

An ion conductive polymer electrolyte film was prepared in the same manner as in Example 7 above, except that 0.1 g of AINB and 0.5 g of PETA were introduced instead of introducing 0.1 g of AINB in Example 7 above.

### Example 9

An ion conductive polymer electrolyte film was prepared in the same manner as in Example 7 above, except that 3 g of the compound represented by Formula 2-c prepared from the Preparation Example 6 was used.

### Example 10

An ion conductive polymer electrolyte film was prepared in the same manner as in Example 8 above, except that 3 g of the compound represented by Formula 2-c prepared from the Preparation Example 6 was used.

### Example 11

3 g of the compound represented by Formula W-1 prepared from Preparation Example 7 was dispersed in 30 mL of a N-methyl-2-pyrrolidone solvent to obtain a transparent solution, and thereafter, the transparent solution was subjected to solution casting uniformly on a flat glass plate. Thereafter, the above solution was dried at a temperature of 40 °C for about 12 hours, and then dried in a vacuum oven at a temperature of 70 °C for 24 hours. Through the above-described process, an ion conductive polymer electrolyte film having a thickness of 20 µm to 40 µm was prepared.

### Example 12

An ion conductive polymer electrolyte film was prepared in the same manner as in Example 11 above, except that 3 g of the compound represented by Formula X-1 prepared from Preparation Example 8 was used.

### Example 13

An ion conductive polymer electrolyte film was prepared in the same manner as in Example 11 above, except that 3 g of the compound represented by Formula Z-1 prepared from Preparation Example 9 was used.

### Comparative Example 1

Lithium bis(trifluoromethyl)sulfonylimide (EO:Li⁺ molar ratio = 20:1) was dissolved in acetonitrile, and then 3 g (20 wt% vs N-methyl-2-pyrrolidone) of PEO was added thereto. At this time, the PEO has a composition in which PEO (Sigma-Aldrich Corporation) having a Mw = 100,000 and PEO (Sigma-Aldrich Corporation) having a MW = 600,000 were mixed at a weight ratio of 9:1. The mixture was stirred using an overhead-stirrer under the condition of room temperature for 2 hours. Thereafter, the stirred mixture was subjected to ultrasonication treatment (exposed to sonication conditions) for 20 minutes in a bath-sonicator to remove air bubbles, and a PEO-LiTFSI solution was obtained. Thereafter, using a film applicator, the PEO-LiTFSI solution was added dropwise on a MRF-38 release film. First, the above solution was dried under the condition of room temperature for about 12 hours, and then vacuum-dried in a vacuum oven at a temperature of 40 °C for 8 hours. Through the above-described process, a PEO-LiTFSI polymer electrolyte film having a thickness of 10 µm to 50 µm was prepared.

### Comparative Example 2

An ion conductive polymer electrolyte film was prepared in the same manner as in Example 11 above, except that 3 g of the compound represented by Formula Q prepared from Preparation Example 10 was used.

### Experimental Example 1 - Measurement of ion conductivity

In a glove box, the polymer electrolyte film prepared in each of Examples and Comparative Examples was soaked in a solvent of EC/PC (1:1 weight ratio), and the polymer electrolyte film was interposed between two stainless steel electrodes (used as a negative electrode and a positive electrode) in a battery case to manufacture a coin-cell including an electrode assembly.

By using a potentiostat (Biologics Corporation SP-200), the coin cell manufactured above was measured at room temperature (25 °C) for impedance with an amplitude of 10 mV and a measurement frequency range of 0.1 Hz to 7 Mhz, and the measured impedance was substituted into an equation of L=I/RS (L= ion conductivity, I= electrode thickness, R= measured impedance value, S= electrode area) to measure ion conductivity. The measured ion conductivity is shown in Table 1 or 2 below.

### Experimental Example 2 - Measurement of lithium ion transference number

In a glove box, the polymer electrolyte film prepared in each of Examples and Comparative Examples was soaked in a solvent of EC/PC (1:1 weight ratio), and the polymer electrolyte film was interposed between lithium foil electrodes (used as a negative electrode and a positive electrode) having a thickness of 200 µm in a battery case to manufacture a coin-cell including an electrode assembly stored at a temperature of 60 °C for about 12 hours.

By using a potentiostat (Biologics Corporation SP-200), the coin cell manufactured above was measured for resistance of a passivation layer before and after a chronoamperometry experiment. The measured resistance was substituted into an equation of t_{Li+} = Iₛ(ΔV - IₒRₒ)/Iₒ (△V - I_{S}R_{S}) (△V = voltage applied to a cell = 3 mV, Iₒ = initial current, I_{S} = steady-state current, Rₒ = initial resistance of passivation layer on lithium electrode surface, R_{S} = steady-state resistance of passivation layer on lithium electrode surface) to measure a lithium ion transference number t_{Li+}. The measured lithium ion transference numbers are shown in Table 1 or 2 below.

### Experimental Example 3 - Tensile strength and tensile strain

The film prepared by each of Examples 7 to 10 and Comparative Example 1 was measured for tensile strength and tensile strain by using a static material testing machine (measuring device) of ZwickiLine Corporation, which are shown in Table 2 below. Specifically, a specimen cut to 10 mm in width and 30 mm in length was fixed to the above measuring device, and while tensioning the specimen at a rate of 5 mm per minute, the force at the time at which the specimen was broken and the change in the length of the specimen were measured. The tensile strength refers to a value obtained by dividing the force at the time of deformation or fracture by the cross-sectional area of the specimen before the deformation, and the tensile strain refers to a change in the length of the specimen with respect to the applied force.

**[Table 1]**

| Classifica tion | Solid electrolyte | Ion conductivity (mS/cm) | Lithium ion transference number |
|---|---|---|---|
| Example 1 | Solid electrolyte including Formula 1-a | 5.6 × 10⁻⁴ | 0.33 |
| Example 2 | Solid electrolyte including Formula 1-b | 4.8 × 10⁻⁴ | 0.29 |
| Example 3 | Solid electrolyte including Formula 2-a | 7.9 × 10⁻³ | 0.94 |
| Example 4 | Solid electrolyte including Formula 2-b | 4.1 × 10⁻³ | 0.91 |
| Example 5 | Solid electrolyte including Formula 3-a | 8.3 × 10⁻⁴ | 0.83 |
| Example 6 | Solid electrolyte including Formula 4-a | 1.1 × 10⁻² | 0.74 |
| Example 11 | Solid electrolyte including Formula W-1 | 4.2 × 10⁻⁴ | 0.33 |
| Example 12 | Solid electrolyte including Formula X-1 | 6.3 × 10⁻⁴ | 0.31 |
| Example 13 | Solid electrolyte including Formula Z-1 | 1.9 × 10⁻³ | 0.92 |
| Comparative Example 1 | Solid electrolyte including PEO-LiTFSI | 2.6 × 10⁻⁴ | 0.28 |
| Comparative Example 2 | Solid electrolyte including Formula Q | 7.1 × 10⁻⁵ | 0.30 |

Referring to Table 1, it can be confirmed that Examples 3 to 6 have a higher level of lithium ion conductivity and ion transference number than Comparative Example 1. In addition, it can be confirmed that Examples 1 and 2 also have an equivalent or higher level of lithium ion conductivity and ion transference number than Comparative Example 1. The Examples include an ion conductive polymer in which a sulfonimide anion with an affinity with lithium ions and with a low electron density is fixed in a main chain, wherein the ion conductive polymer may move dissociated lithium ions in an electrolyte with higher efficiency, so that the Examples may have higher ion conductivity and a higher ion transference number properties than Comparative Examples including a typical alkylene oxide-based polymer.

In addition, it can be confirmed that Example 1 using the compound represented by Formula 1-a has higher ion conductivity and a higher ion transference number than Example 2 using the compound represented by Formula 1-b, and that Example 3 using the compound represented by Formula 2-a has higher ion conductivity and a higher ion transference number than Example 4 using the compound represented by Formula 2-b. This can be due to the fact that fluorine, which has high electronegativity, delocalizes an anion of neighboring sulfonimide, thereby lowering the electron density of the sulfonimide, and as a result, the electrostatic attraction between lithium ions and a solid electrolyte is alleviated, which results in moving dissociated lithium ions in an electrolyte with higher efficiency.

In addition, referring to Table 1, it can be confirmed that Examples 11 to 13 are superior in ion conductivity and lithium ion transference number to Comparative Examples 1 and 2. Particularly, it can be confirmed that Example 13 is significantly superior to Comparative Examples 1 and 2. The compound included in the solid electrolyte of Examples 11 to 13 above includes an aromatic ring structure in which an alkoxy group is substituted at one carbon position of a benzene ring, and an alkyl group substituted by a sulfonyl group is positioned on both sides of an ortho position, and thus, has a structure having affinity with lithium, so that the ion conductivity and ion transference number are excellent, and particularly, the compound included in the solid electrolyte of Example 13 has an ion conductive structure in which a sulfonimide anion is fixed to a main chain, and thus, may move dissociated lithium ions with higher efficiency. As a result, Examples 11 to 13 may have higher ion conductivity and a higher ion transference number than Comparative Example 2 above not including an alkoxy group and Comparative Example 1 above including a typical alkylene oxide-based polymer.

**[Table 2]**

| Classification | Solid electrolyte | Crosslinkable compound | Ion conductivity (mS/cm) | Lithiumion transference number | Tensile strength (MPa) | Tensile strain (%) |
|---|---|---|---|---|---|---|
| Example 7 | Solid electrolyte including Formula 1-c | X | 6.0 × 10⁻⁴ | 0.40 | 0.82 | 31 |
| Example 8 | Solid electrolyte including Formula 1-c | PETA | 7.2 × 10⁻⁴ | 0.32 | 2.1 | 42 |
| Example 9 | Solid electrolyte including Formula 2-c | X | 8.0 × 10⁻³ | 0.934 | 0.89 | 29 |
| Example 10 | Solid electrolyte including Formula 2-c | PETA | 9.1 × 10⁻³ | 0.91 | 2.4 | 37 |
| Comparative Example 1 | Solid electrolyte including PEO-LiTFSI | - | 2.6 × 10⁻⁴ | 0.28 | 0.1 | 10 |

Referring to Table 2, it can be confirmed that Examples 9 and 10 have a higher level of lithium ion conductivity and ion transference number than Comparative Example 1. In addition, it can be confirmed that Examples 7 and 8 also have a higher level of lithium ion transference number than Comparative Example 1. The Examples include an ion conductive polymer in which a sulfonimide anion with an affinity with lithium ions and with a low electron density is fixed in a main chain, wherein the ion conductive polymer may move dissociated lithium ions in an electrolyte with higher efficiency, so that the Examples may have higher ion conductivity and a higher ion transference number properties than Comparative Examples including a typical alkylene oxide-based polymer.

In addition, it can be confirmed that Example 9 using the compound represented by Formula 2-c has higher ion conductivity and a higher ion transference number than Example 7 using the compound represented by Formula 1-c, and that Example 10 to which PETA, a crosslinkable compound, was added has a slightly lower lithium ion transference number than Example 9 to which PETA was not added. This is due to the fact that a network was formed by a radical reaction between the double bonds, and is the result of forming a solid electrolyte structure firmly, thereby maintaining structural uniformity while increasing the durability of physical strength such as tensile strength and tensile strain not to significantly reduce the ion conductivity and transference number of lithium ions.

### Experimental Example 4 - Evaluation of lifespan properties of battery

In a glove box, the polymer electrolyte film prepared in each of Examples and Comparative Examples was soaked in a solvent of EC/PC (1:1 weight ratio), and the polymer electrolyte film was interposed between lithium foil electrodes (used as a negative electrode and a positive electrode) having a thickness of 200 µm in a battery case to manufacture a coin-cell including an electrode assembly stored under the condition of 60 °C for about 12 hours.

The coin cell was charged at a current density of 0.2 mA/cm² at 25 °C using a charger/discharger up to an 1 hour cutoff, and discharged for 1 hour, which was set to 1 cycle, and 3 cycles of the charge/discharge process were performed. Subsequently, the coin cell was charged at a current density of 1.0 mA/cm² using a charger/discharger up to an 1 hour cutoff, and discharged for 1 hour, which was set to 1 cycle, and 400 cycles of the charge/discharge process were performed. At this time, a voltage generated in the first cycle when the current density was 1.0 mA/cm² was measured, and also, the cycle number at which a voltage, which is 1.5 times the voltage generated in the first cycle when the current density was 1.0 mA/cm² is generated, is shown in Table 3 below.

**[Table 3]**

| Classification | Cycle at which a voltage that is 1.5 times the voltage generated in first cycle is generated |
|---|---|
| Example 1 | 236 |
| Example 2 | 227 |
| Example 3 | 389 |
| Example 4 | 342 |
| Example 5 | >400 |
| Example 6 | >400 |
| Example 7 | 260 |
| Example 8 | 300 |
| Example 9 | 380 |
| Example 10 | >400 |
| Example 11 | 178 |
| Example 12 | 221 |
| Example 13 | >400 |
| Comparative Example 1 | 118 |
| Comparative Example 2 | 144 |

Referring to Table 3, it can be confirmed that Examples 1 to 13 suppress an increase in the voltage compared to Comparative Examples 1 and 2, that is, the lifespan of a battery to which a lithium metal negative electrode is applied is improved. Particularly, in the case of Examples 3 to 6, 9, 10, and 13 to which an ion conductive polymer lithiated or including a lithium compound is applied, it can be confirmed that the lifespan improvement effect is significantly excellent. This can be attributed to the improvement of ion conductivity and ion transference number, as well as an effect caused by the compound including the ion conductive polymer according to the present invention, which suppresses an increase in electrode surface resistance and prevents the formation of dendritic crystals on the surface of a lithium metal electrode.

## Claims

1. A compound comprising a repeating unit represented by Formula 1 below, or a compound being a lithium salt thereof: 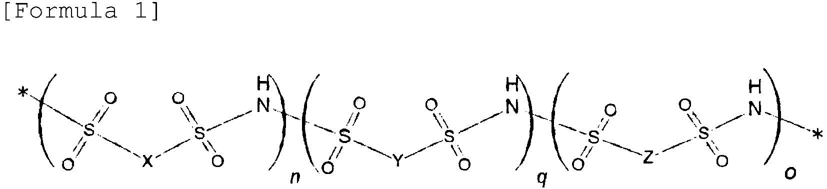 wherein in Formula 1 above,
the X, Y, and Z are each independently one or more selected from the group consisting of Formulas a-1, b, and c-1 below,
the n is an integer of 1 to 100,
the q is an integer of 1 to 100,
the o is an integer of 0 to 100, and
the * is a connection site between the repeating units, or an end site, and 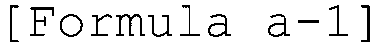 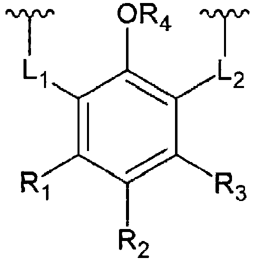 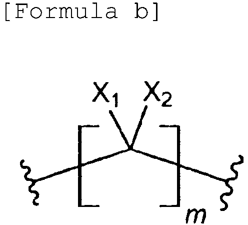 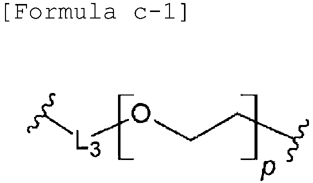
wherein in Formulas a-1, b, and c-1 above,
the R₁, R₂, and R₃ are each independently a hydrogen element, a halogen element, -CN, -NO₂, a substituted or unsubstituted C₁-C₁₀ alkyl group, a substituted or unsubstituted C₁-C₁₀ alkoxy group, a substituted or unsubstituted C₂-C₁₀ alkenyl group, a substituted or unsubstituted C₂-C₁₀ alkynyl group, -C(=O)R₅, -P(=O)(OR₅)₂, - P(OR₅)(OR₆), -OP(=O)(OR₅)(OR₆), -S(=O)R₅, or -S(=O)₂R₅,
the R₄ is a hydrogen element, or a substituted or unsubstituted C₁-C₁₀ alkyl group,
the R₅ and R₆ are each independently a hydrogen element, a substituted or unsubstituted C₁-C₁₀ alkyl group, or a substituted or unsubstituted C₂-C₁₀ alkenyl group,
the L₁, L₂, and L₃ are each independently a direct linkage, or a substituted or unsubstituted C₁-C₁₀ alkylene group,
the X₁ and X₂ are each independently a hydrogen element, a halogen element, -CN, -NO₂, a substituted or unsubstituted C₁-C₁₀ alkyl group, or a substituted or unsubstituted C₆-C₁₂ aryl group,
the m is an integer of 1 to 40, and
the p is an integer of 1 to 200.

2. The compound of claim 1, wherein at least one of the X, Y, and Z comprises Formula a-1 above.

3. The compound of claim 1, wherein the R₂ and R₄ are each independently a C₁-C₅ alkyl group.

4. The compound of claim 1, wherein the L₁, L₂, and L₃ are each independently a C₁-C₅ alkylene group.

5. The compound of claim 1, wherein the X₁ and X₂ are each independently a hydrogen element, or a halogen element.

6. The compound of claim 1, wherein:
the n is an integer of 1 to 20;
the q is an integer of 1 to 20;
the o is an integer of 1 to 20;
the m is an integer of 1 to 10; and
the p is an integer of 1 to 50.

7. The compound of claim 1, wherein the compound including a repeating unit represented by Formula 1 above is represented by Formula 1-a or Formula 1-b below: 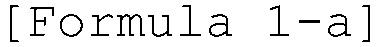 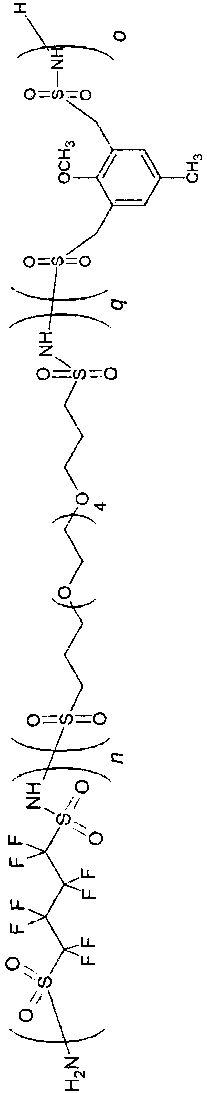 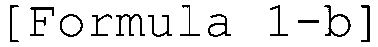 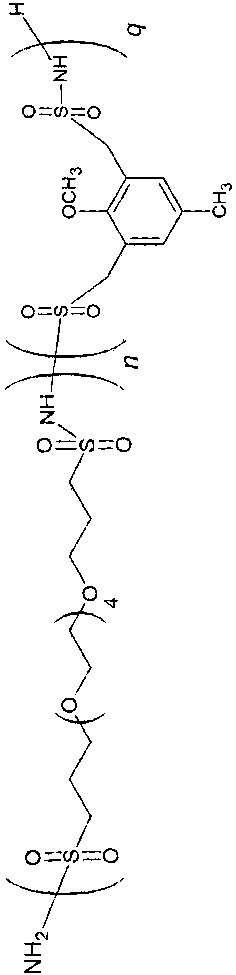 wherein in Formulas 1-a and 1-b above, the n is an integer of 1 to 20, the q is an integer of 1 to 20, and the o is an integer of 1 to 20.

8. The compound of claim 1, wherein the compound comprises one or more functional groups selected from the group consisting of Formulas d to g below at at least one end site among the end sites: 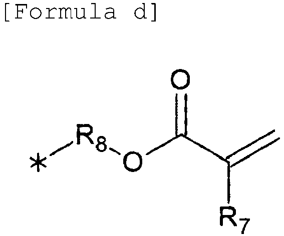 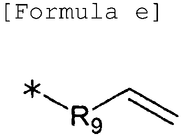 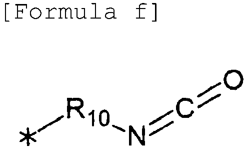 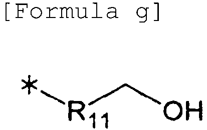 wherein in Formulas d to g above,
the R₇ is a hydrogen element, or a substituted or unsubstituted C₁-C₁₀ alkyl group,
the R₈ to R₁₁ are each independently a direct linkage, a substituted or unsubstituted C₁-C₁₀ alkylene group, or -R₁₂-OR₁₃-,
the R₁₂ and R₁₃ are each independently a direct linkage, or a substituted or unsubstituted C₁-C₁₀ alkylene group, and
the * is a bonding position.

9. The compound of claim 8, wherein:
the R₇ is a C₁-C₆ alkyl group;
the R₈ to R₁₁ are each independently a direct linkage, a C₁-C₆ alkylene group substituted or unsubstituted with a fluorine element, or -R₁₂-O-R₁₃-; and
the R₁₂ and R₁₃ are each independently a direct linkage, or a C₁-C₆ alkylene group substituted or unsubstituted with a fluorine element.

10. The compound of claim 8, wherein the compound is represented by Formula 1-c below: 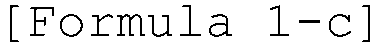 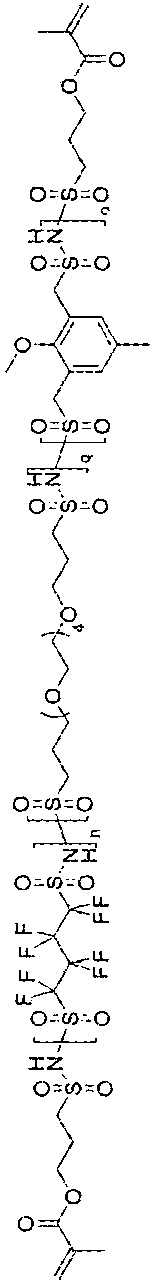 wherein in Formula 1-c above, the n is an integer of 1 to 20, the q is an integer of 1 to 20, and the o is an integer of 1 to 20.

11. The compound of claim 1, wherein the lithium salt comprises a repeating unit represented by Formula 2 below: 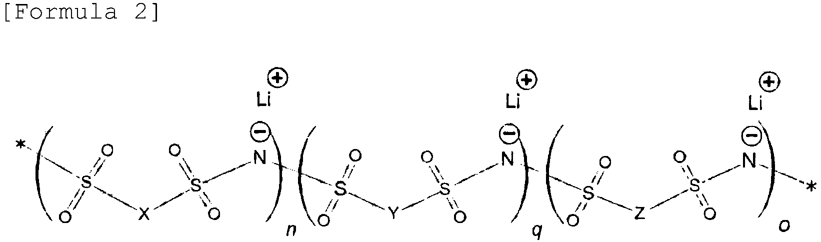 wherein in Formula 2 above,
the X, Y, and Z are each independently one or more selected from the group consisting of Formulas a-1, b, and c-1 below,
the n is an integer of 1 to 100,
the q is an integer of 1 to 100,
the o is an integer of 0 to 100, and
the * is a connection site between the repeating units, or an end site, and 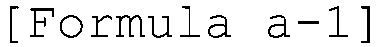 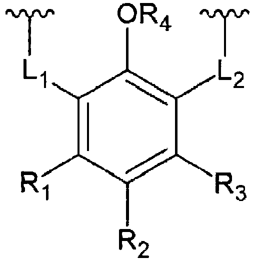 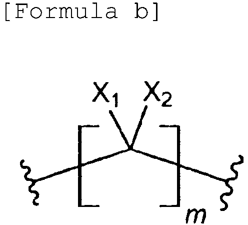 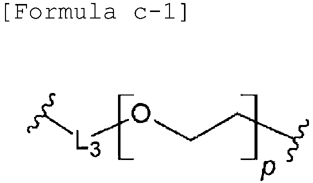
wherein in Formulas a-1, b, and c-1 above,
the R₁, R₂, and R₃ are each independently a hydrogen element, a halogen element, -CN, -NO₂, a substituted or unsubstituted C₁-C₁₀ alkyl group, a substituted or unsubstituted C₁-C₁₀ alkoxy group, a substituted or unsubstituted C₂-C₁₀ alkenyl group, a substituted or unsubstituted C₂-C₁₀ alkynyl group, -C(=O)R₅, -P(=O)(OR₅)₂, - P(OR₅)(OR₆), -OP(=O)(OR₅)(OR₆), -S(=O)R₅, or -S(=O)₂R₅,
the R₄ is a hydrogen element, or a substituted or unsubstituted C₁-C₁₀ alkyl group,
the R₅ and R₆ are each independently a hydrogen element, a substituted or unsubstituted C₁-C₁₀ alkyl group, or a substituted or unsubstituted C₂-C₁₀ alkenyl group,
the L₁, L₂, and L₃ are each independently a direct linkage, or a substituted or unsubstituted C₁-C₁₀ alkylene group,
the X₁ and X₂ are each independently a hydrogen element, a halogen element, -CN, -NO₂, a substituted or unsubstituted C₁-C₁₀ alkyl group, or a substituted or unsubstituted C₆-C₁₂ aryl group,
the m is an integer of 1 to 40, and
the p is an integer of 1 to 200.

12. The compound of claim 11, wherein the compound including a repeating unit represented by Formula 2 above is represented by Formula 2-a or Formula 2-b below: 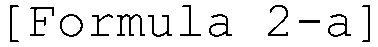 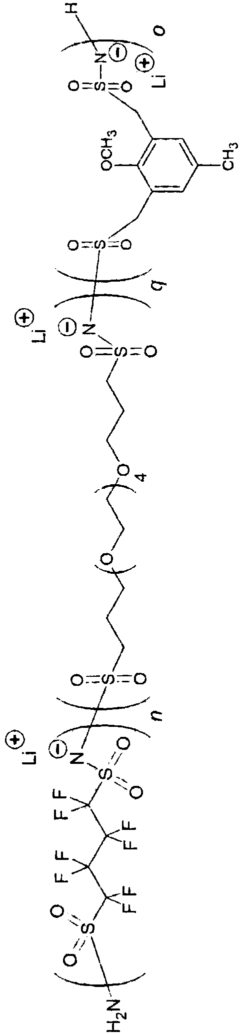 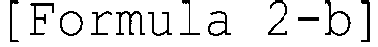 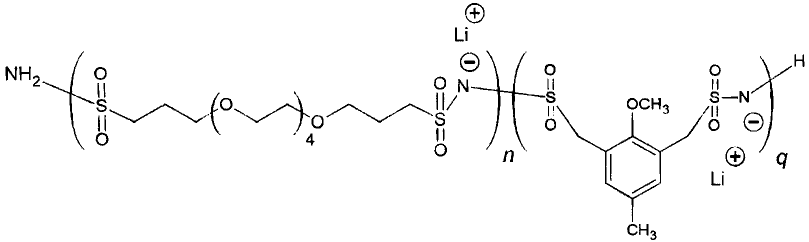 wherein in Formulas 2-a and 2-b above, the n is an integer of 1 to 20, the q is an integer of 1 to 20, and the o is an integer of 1 to 20.

13. The compound of claim 11, wherein the compound comprises one or more functional groups selected from the group consisting of Formulas d to g below at at least one end site among the end sites: 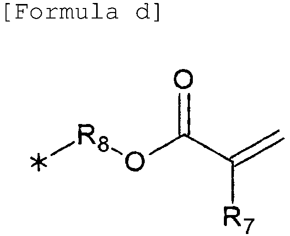 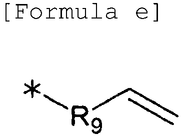 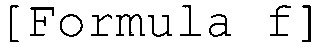 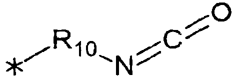 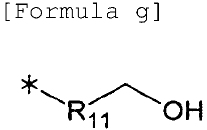 wherein in Formulas d to g above,
the R₇ is a hydrogen element, or a substituted or unsubstituted C₁-C₁₀ alkyl group,
the R₈ to R₁₁ are each independently a direct linkage, a substituted or unsubstituted C₁-C₁₀ alkylene group, or -R₁₂-OR₁₃-,
the R₁₂ and R₁₃ are each independently a direct linkage, or a substituted or unsubstituted C₁-C₁₀ alkylene group, and
the * is a bonding position.

14. The compound of claim 13, wherein the compound including a repeating unit represented by Formula 2 above is represented by Formula 2-c below: 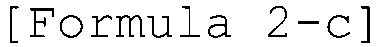 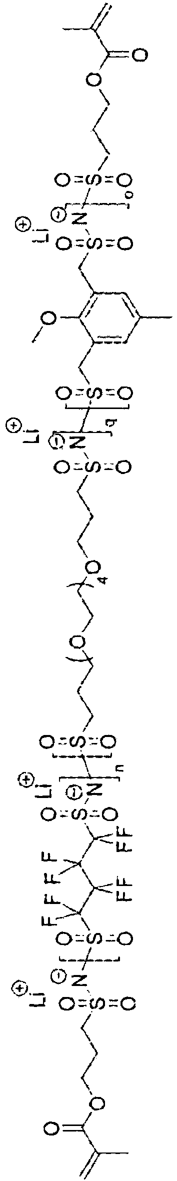 wherein in Formula 2-c above, the n is an integer of 1 to 20, the q is an integer of 1 to 20, and the o is an integer of 1 to 20.

15. A composite comprising the compound according to claim 1 and a lithium compound.

16. The composite of claim 15, wherein the lithium compound is one or more selected from the group consisting of LiCl, LiBr, LiI, LiBF₄, LiClO₄, LiB₁₀Cl₁₀, LiAlCl₄, LiAlO₄, LiPF₆, LiCF₃SO₃, LiCH₃CO₂, LiCF₃CO₂, LiAsF₆, LiSbF₆, LiCH₃SO₃, lithium bis(fluorosulfonyl) imide (LiFSI) (LiN(SO₂F)₂) lithium bisperfluoroethanesulfonimide (LiBETI), (LiN(SO₂CF₂CF₃)₂), and lithium (bis)trifluoromethanesulfonimide (LiTFSI) (LiN(SO₂CF₃)₂).

17. The composite of claim 15, wherein the composite is represented by one or more selected from the group consisting of Formulas 3-a, 4-a, and 4-b below: 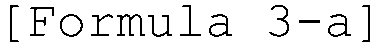 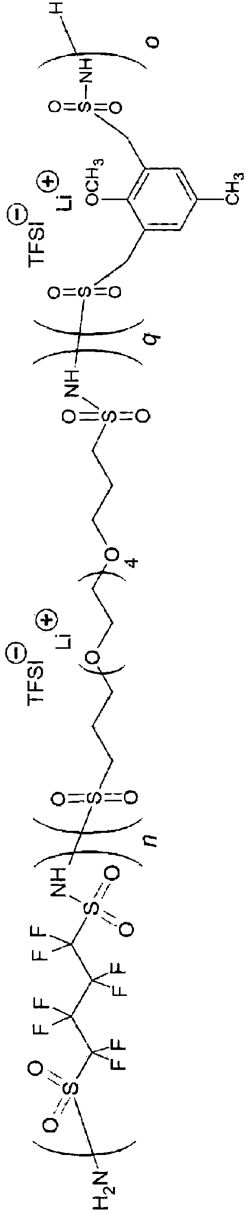 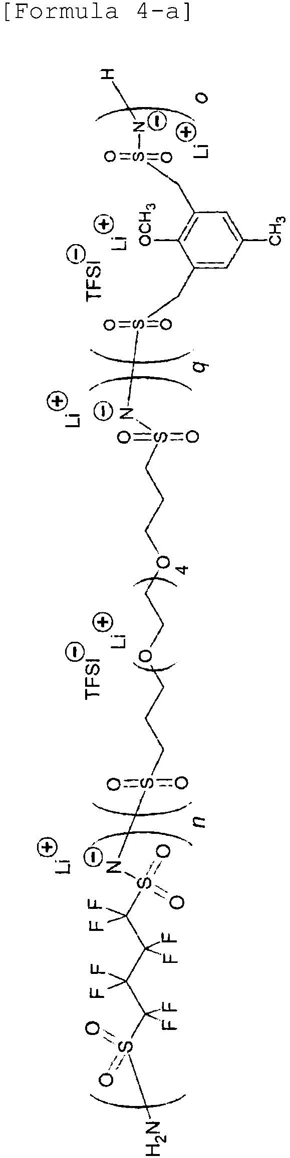 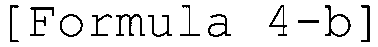 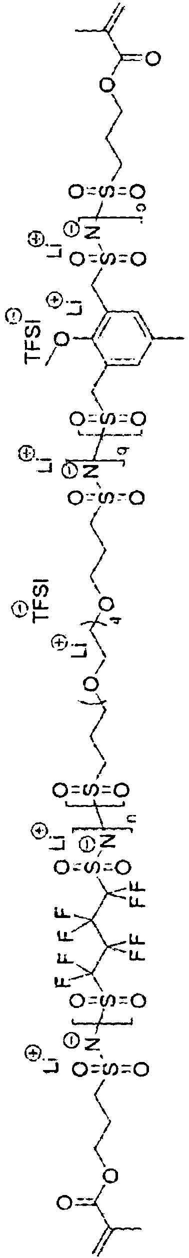 wherein in Formulas 3-a, 4-a, and 4-b above, the n is an integer of 1 to 20, the q is an integer of 1 to 20, and the o is an integer of 1 to 20.

18. A solid electrolyte comprising one or more selected from the group consisting of the compound of claim 1 and the composite of claim 15.

19. The solid electrolyte of claim 18, further comprising a crosslinkable compound.

20. An all-solid-state battery comprising the solid electrolyte according to claim 19.

21. A compound represented by Formula A below: 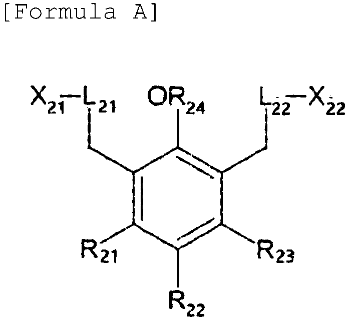 wherein in Formula A above,
the R₂₁, R₂₂, and R₂₃ are each independently a hydrogen element, a halogen element, a substituted or unsubstituted C₁-C₁₀ alkyl group, a substituted or unsubstituted C₁-C₁₀ alkoxy group, a substituted or unsubstituted C₂-C₁₀ alkenyl group, or a substituted or unsubstituted C₂-C₁₀ alkynyl group,
the R₂₄ is a hydrogen element, or a substituted or unsubstituted C₁-C₁₀ alkyl group,
the L₂₁ and L₂₂ are each independently a substituted or unsubstituted C₁-C₁₀ alkylene group, -P(=O) (OR₂₅)-, -(C=O)-, - (S=0)-, or -S(=O)₂-,
the X₂₁ and X₂₂ are each independently a halogen element, -NH₂, phosphate (-OP(=O)(OR₂₅)₂), nitrate (-ON(=O)(OR₂₅)), tosylate (-OTs), sulfonate (-OS(=O)₂R₂₅), or carboxylate (-OC(=O)CH₃), and
the R₂₅ is a substituted or unsubstituted C₁-C₁₀ alkyl group, a substituted or unsubstituted C₂-C₁₀ alkenyl group, or a substituted or unsubstituted C₂-C₁₀ alkynyl group.

22. The compound of claim 21, wherein the R₂₂, R₂₄, and R₂₅ are each independently a C₁-C₅ alkyl group.

23. The compound of claim 21, wherein the L₂₁ and L₂₂ are -S(=O)₂-.

24. The compound of claim 21, wherein the X₂₁ and X₂₂ are each independently a halogen element or -NH₂.

25. The compound of claim 21, wherein the compound represented by Formula A above is one or more selected from the group consisting of Formulas A-a, A-b, and A-c below:

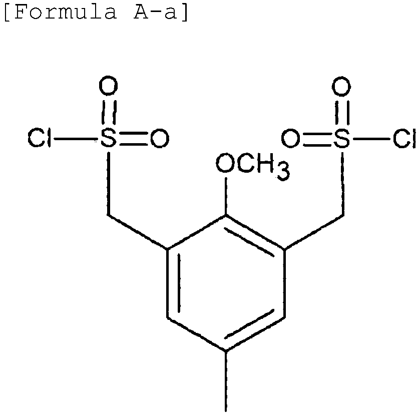

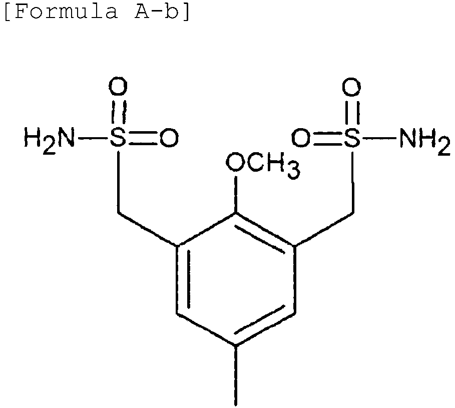

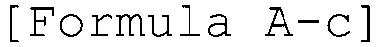

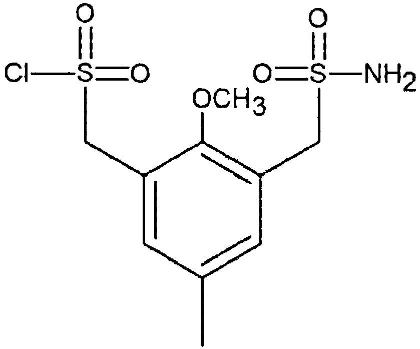

26. A composite comprising the compound according to claim 21 and a lithium compound.

27. The composite of claim 26, wherein the composite is represented by Formula B below: 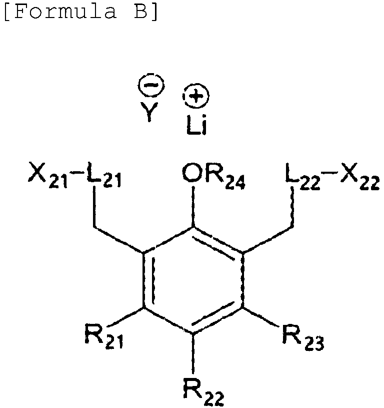 wherein in Formula B above,
the R₂₁, R₂₂, and R₂₃ are each independently a hydrogen element, a halogen element, a substituted or unsubstituted C₁-C₁₀ alkyl group, a substituted or unsubstituted C₁-C₁₀ alkoxy group, a substituted or unsubstituted C₂-C₁₀ alkenyl group, or a substituted or unsubstituted C₂-C₁₀ alkynyl group,
the R₂₄ is a hydrogen element, or a substituted or unsubstituted C₁-C₁₀ alkyl group,
the L₂₁ and L₂₂ are each independently a substituted or unsubstituted C₁-C₁₀ alkylene group, -P(=O) (OR₂₅)-, -(C=O)-, - (S=0)-, or -S(=O)₂-,
the X₂₁ and X₂₂ are each independently a halogen element, -NH₂, phosphate (-OP(=O)(OR₂₅)₂), nitrate (-ON(=O)(OR₂₅)), tosylate (-OTs), sulfonate (-OS(=O)₂R₂₅), or carboxylate (-OC(=O)CH₃),
the R₂₅ is a substituted or unsubstituted C₁-C₁₀ alkyl group, a substituted or unsubstituted C₂-C₁₀ alkenyl group, or a substituted or unsubstituted C₂-C₁₀ alkynyl group, and
the Y⁻ is one or more selected from the group consisting of a halogen anion, BF₄⁻, ClO₄⁻, AlCl₄⁻, AlO₄⁻, PF₆⁻, CF₃SO₃⁻, CH₃CO₂⁻, CF₃CO₂⁻, AsF₆⁻, SbF₆⁻, CH₃SO₃⁻, bis(fluorosulfonyl) imide (FSI⁻) (N(SO₂F)₂⁻), bisperfluoroethanesulfonimide (BETI) (N(SO₂CF₂CF₃)₂⁻), and (bis) trifluoromethanesulfonimide (TFSI) (N(SO₂CF₃)₂⁻).

28. The composite of claim 26, wherein the composite is one or more selected from the group consisting of Formulas B-a, B-b, and B-c below:

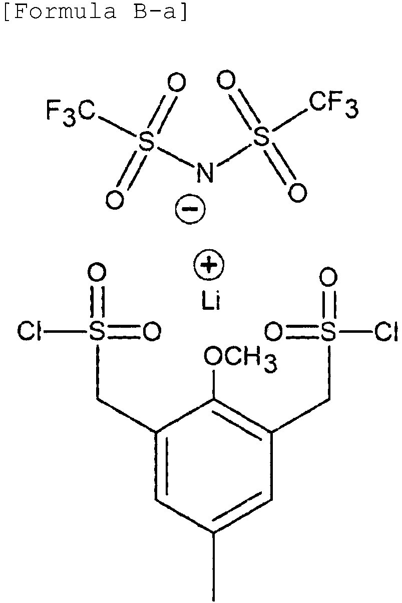

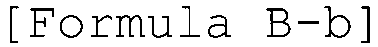

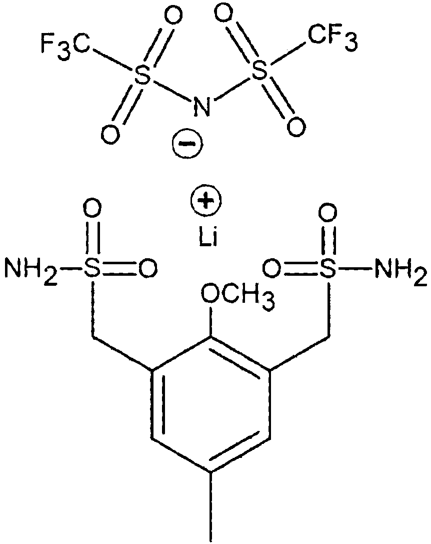

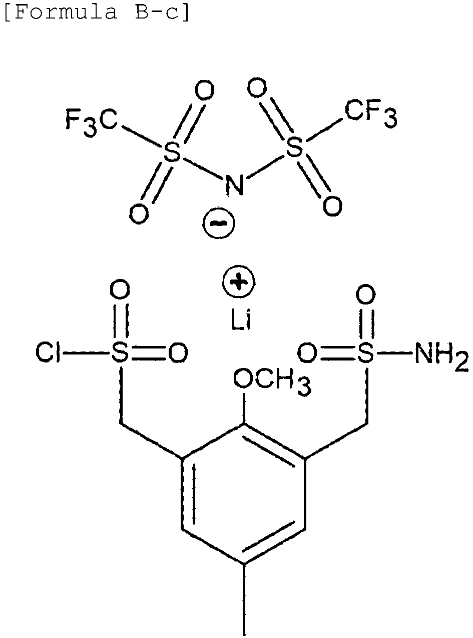

29. A polymer comprising a repeating unit derived from the compound according to claim 21, a repeating unit derived from the composite according to claim 26, or a combination thereof.

30. The polymer of claim 29, wherein the repeating unit derived from the compound according to claim 21 is represented by Formula W-a or W-b below: 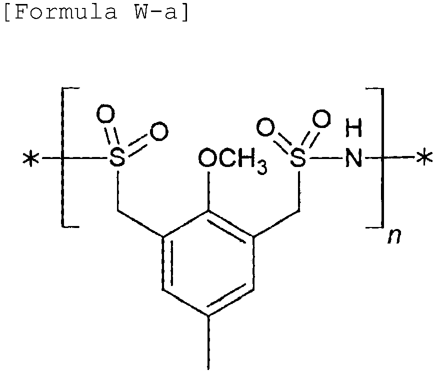 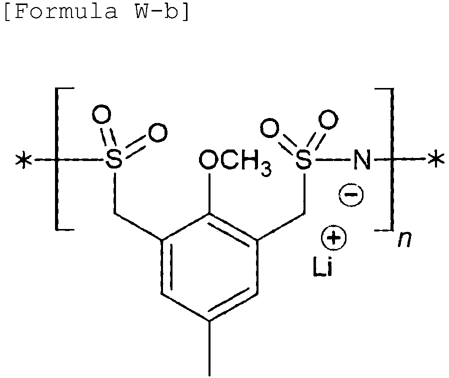 wherein in Formulas W-a and W-b above,
the n is an integer of 1 to 300, and
the * is a connection site between the repeating units.

31. The polymer of claim 29, wherein the repeating unit derived from the composite according to claim 26 is represented by Formula X-a or X-b below: 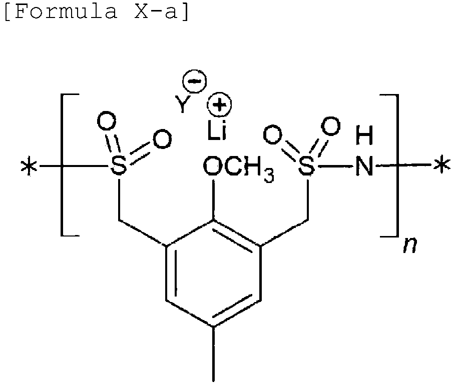 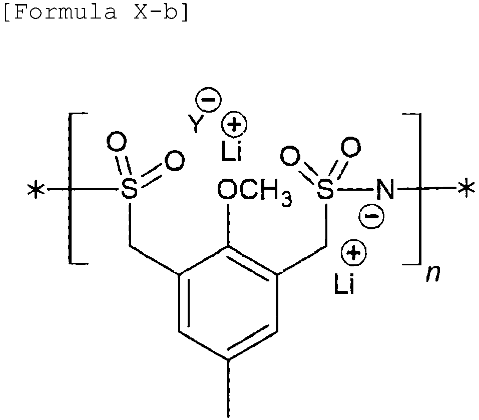
wherein in Formulas X-a and X-b above,
the Y⁻ is one or more selected from the group consisting of a halogen anion, BF₄⁻, ClO₄⁻, AlCl₄⁻, AlO₄⁻, PF₆⁻, CF₃SO₃⁻, CH₃CO₂⁻, CF₃CO₂⁻, AsF₆⁻, SbF₆⁻, CH₃SO₃⁻, bis(fluorosulfonyl) imide (FSI⁻) (N (SO₂F)₂⁻), bisperfluoroethanesulfonimide (BETI) (N(SO₂CF₂CF₃)₂⁻), and (bis)trifluoromethanesulfonimide (TFSI) (N (SO₂CF₃)₂⁻),
the n is an integer of 1 to 300, and
the * is a connection site between the repeating units.

32. The polymer of claim 29, wherein the combination of the repeating unit derived from the compound according to claim 21 and the repeating unit derived from the composite according to claim 26 is represented by Formula Y or Z below: 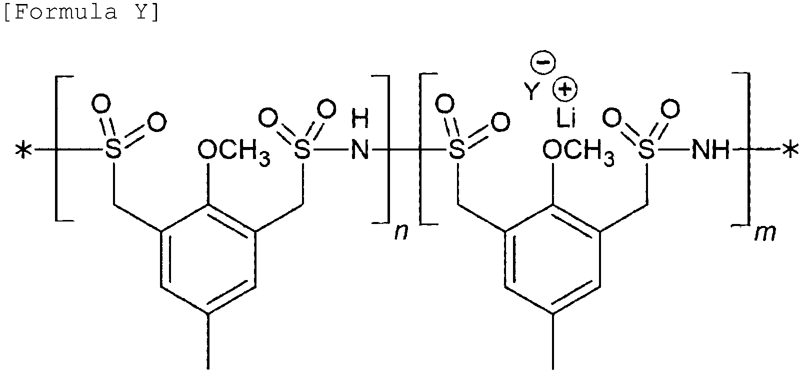 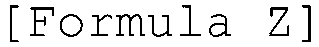 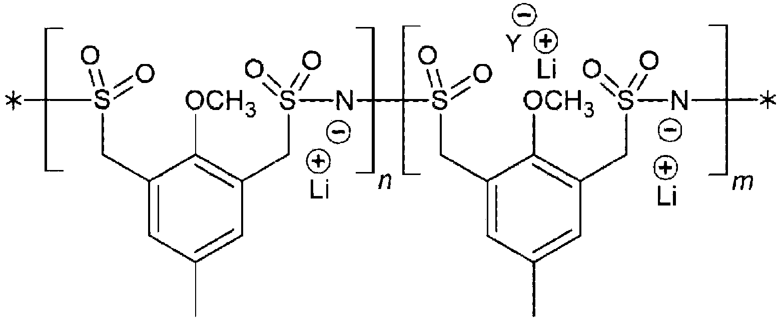 wherein in Formulas Y and Z above,
the Y⁻ is one or more selected from the group consisting of a halogen anion, BF₄⁻, ClO₄⁻, AlCl₄⁻, AlO₄⁻, PF₆⁻, CF₃SO₃⁻, CH₃CO₂⁻, CF₃CO₂⁻, AsF₆⁻, SbF₆⁻, CH₃SO₃⁻, bis(fluorosulfonyl) imide (FSI⁻) (N (SO₂F)₂⁻), bisperfluoroethanesulfonimide (BETI) (N(SO₂CF₂CF₃)₂⁻), and (bis)trifluoromethanesulfonimide (TFSI) (N (SO₂CF₃)₂⁻),
the n and m are each independently an integer of 1 to 300, and
the * is a connection site between the repeating units.

33. A solid electrolyte comprising the polymer according to claim 29.
